# EUROPEAN PATENT SPECIFICATION

(11) **EP 2 117 562 B1**
(45) Date of publication and mention of the grant of the patent: **26.10.2011**
(21) Application number: 08708026.3
(22) Date of filing: 21.01.2008
(51) Int. Cl.: A61K 31/704, A61K 31/4015, A61K 31/401, A61P 25/08

(54) **PHARMACEUTICAL COMPOSITIONS COMPRISING NK1 RECEPTOR ANTAGONISTS AND SODIUM CHANNEL BLOCKERS**
PHARMAZEUTISCHE ZUBEREITUNGEN MIT NK1-REZEPTOR-ANTAGONISTEN UND NATRIUMKANALBLOCKERN
COMPOSITIONS PHARMACEUTIQUES CONTENANT DES ANTAGONISTES DU RÉCEPTEUR DE NK1 ET DES BLOQUEURS DE CANAL SODIQUE

(30) Priority: 24.01.2007 GB 0701366
(43) Date of publication of application: 18.11.2009
(73) Proprietor: Glaxo Group Limited, Greenford, Middlesex UB6 0NN (GB)
(72) Inventor: ALVARO, Giuseppe, I-37135 Verona (IT); LARGE, Charles, I-37135 Verona (IT)
(74) Representative: Mauro, Marina Eliana
(86) International application number: PCT/EP2008/050622
(87) International publication number: WO 2008/090115

(56) References cited:
- WO-A-2007/042240
- WO-A-2007/042250

## Description

The present invention relates to pharmaceutical compositions comprising an NK1 receptor antagonist and an α-aminocarboxyamide derivatives Sodium Channel blocker, for example (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, as a combined preparation for simultaneous or sequential administration. The invention further relates to the use of such compositions in the treatment of certain disorders, including epilepsy and mood disorders.

Substance P is a short-chain polypeptide that functions as a neurotransmitter and a neuromodulator. It belongs to the tachykinin neuropeptide family. In the central nervous system Substance P has been associated with the regulation of mood disorders, anxiety, stress, reinforcement, neurogenesis, respiratory rhythm, neurotoxicity, nausea/emesis and pain. The endogenous receptor for Substance P is the neurokinin 1 receptor (NK1 receptor). A large number of NK1 receptor antagonists are known, including aprepitant (Emend^{™}), which is marketed for use in the prevention of acute and delayed chemotherapy-induced nausea and vomiting and in the prevention of post operative nausea and vomiting. Other potential uses of NK1 receptor antagonists include treatment of anxiety and depression, pain, inflammatory dieases, over-active bladder, sleep disorders, allergic disorders, CNS disorders, skin disorders, cough and gastrointestinal disorders.

Voltage-gated sodium channels are responsible for the initial phase of the action potential, which is a wave of electrical depolarisation usually initiated at the soma of the neuron and propagated along the nerve axon to the terminals. At the terminals, the action potential triggers the influx of calcium and the release of neurotransmitter. Some sodium channel blockers, such as lamotrigine and carbamazepine are used to treat epilepsy. In this case, partial inhibition of voltage-gated sodium channels reduces neuronal excitability and reduces seizure propagation. A key feature of these drugs is their use-dependent mechanism of action. The drugs are thought to stabilise an inactivated configuration of the channel that is adopted rapidly after the channel opens. This inactivated state provides a refractory period before the channel returns to its resting (closed) state ready to be reactivated. As a result, use-dependent sodium channel blockers retard the firing of neurons at high frequency, for example in response to painful stimuli, and will help to prevent repetitive firing during periods of prolonged neuronal depolarisation that might occur, for example, during a seizure. Action potentials triggered at low frequencies, for example in the heart, will not be significantly affected by these drugs, although the safety margin differs in each case, since at high enough concentrations each of these drugs is capable of blocking the resting or open states of the channels.

Drugs that block voltage-gated sodium channels in a use-dependent manner are also used in the treatment of bipolar disorder, either to reduce symptoms of mania or depression, or as mood stabilisers to prevent the emergence of mood episodes. Clinical and preclinical evidence also suggests that use-dependent sodium channel blockers may help to reduce the symptoms of schizophrenia. It is hypothesised that efficacy in these psychiatric disorders may result in part from a reduction of excessive glutamate release. The reduction in glutamate release is thought to be a consequence of use-dependent sodium channel inhibition in key brain areas, such as the frontal cortex. However, interaction with voltage-gated calcium channels may also contribute to the efficacy of these drugs.

Lamotrigine is an effective anticonvulsant that is also indicated in the US for the prevention of mood episodes in patients with bipolar I disorder. However, the efficacy of the drug in an acute setting is limited by the need for a 4-6 week dose-titration to avoid rash. In addition, lamotrigine and other sodium channel blockers are limited in the range of doses that can be explored to achieve efficacy due to the appearance of CNS side-effects.

WO2007/042240 and WO2007/042250, both published prior to the international filing date but later than the priority date claimed (P-documents) disclose compounds useful in the treatment of diseases and conditions mediated by modulation of use-dependent voltage gated sodium channels. WO2007/042250 discloses prolinamide derivatives and WO2007/042240 discloses quaternary alpha-aminocarboxamide derivatives.

The aim of the present invention is to identify new pharmaceutical compositions that allow an improved clinical efficacy with respect to the individual components when administered alone.
A further aim of the present invention is to identify new pharmaceutical compositions that allow the use of a decreased dose of the active ingredient or ingredients, in order to achieve an improved tolerability profile, i.e. reduce side effects.

A solution provided by the present invention is a pharmaceutical composition comprising an NK1 receptor antagonist and a Sodium Channel blocker which is a compound of formula (I), 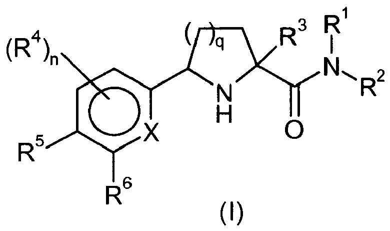 wherein
- R¹ and R²: are independently hydrogen, C₁₋₆alkyl or C₃₋₆cydoalkylC₁₋₆alkyl; or R¹ and R², together with the nitrogen to which they are attached, may form an unsubstitued 3-, 4-, 5- or 6-membered saturated ring;
- R³: is C₁₋₆alkyl, C₁₋₆haloalkyl, C₁₋₃alkoxyC₁₋₃alkyl or C₁₋₃haloalkoxyC₁₋₃alkyl; or R¹ and R³, together with the interconnecting atoms, form a saturated or unsaturated 4- to 6-membered ring;
- X: is carbon or nitrogen;
- n: is 0, 1 or 2, wherein when present each R⁴ is independently selected from the list consisting of C₁₋₃alkyl, halogen, cyano, haloC₁₋₃akyl, hydroxy, C₁₋₃alkoxy and C₁₋₃haloalkoxy;
- q: is 1 or 2;
- either R⁵ or R⁶: is -O-R⁷ or -OCH₂R⁷, wherein the other R⁵ or R⁶ is hydrogen or R⁴; and wherein R⁷ is either a phenyl ring or a 5- or 6-membered aromatic heterocyclic ring (independently containing one or more nitrogen, sulphur or oxygen atoms) wherein either the phenyl ring or the heterocyclic ring is optionally substituted by one or more groups independently selected from the list consisting of C₁₋₃alkyl, halogen, cyano, haloC₁₋₃alkyl, hydroxy, C₁₋₃alkoxy and C₁-₃haloalkoxy;
or a pharmaceutically acceptable salt or solvate thereof,
as a combined preparation for simultaneous or sequential administration.

In one embodiment, a solution provided by the present invention is a pharmaceutical composition comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, as a combined preparation for simultaneous or sequential administration.

A further solution provided by the present invention is a pharmaceutical composition comprising an NK1 receptor antagonist and a Sodium Channel blocker compound of formula (I) as above defined, or a pharmaceutically acceptable salt or solvate thereof, wherein at least one of them is at sub therapeutic dose, as a combined preparation for simultaneous or sequential administration.

In another embodiment, a further solution provided by the present invention is a pharmaceutical composition comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, wherein at least one of them is at sub therapeutic dose, as a combined preparation for simultaneous or sequential administration.

Thus, in a first aspect the invention provides a pharmaceutical composition comprising an NK1 receptor antagonist and a Sodium Channel blocker compound of formula (I) as above defined, or a pharmaceutically acceptable salt or solvate thereof, as a combined preparation for simultaneous or sequential administration.

In one embodiment, the invention provides a pharmaceutical composition comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, as a combined preparation for simultaneous or sequential administration.

In another embodiment, the invention provides a pharmaceutical composition comprising an NK1 receptor antagonist and a Sodium Channel blocker compound of formula (I) as above defined, or a pharmaceutically acceptable salt or solvate thereof, wherein at least one of them is at sub therapeutic dose, as a combined preparation for simultaneous or sequential administration.

In a further embodiment, the invention provides a pharmaceutical composition comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, wherein at least one of them is at sub therapeutic dose, as a combined preparation for simultaneous or sequential administration.

Pharmaceutical compositions according to the present invention comprise an NK1 receptor antagonist and a Sodium Channel blocker compound of formula (I) as above defined, or a pharmaceutically acceptable salt or solvate thereof, wherein one of them may be used at sub therapeutic dose, together with one or more pharmaceutically acceptable carriers or excipients and optionally other therapeutic agents. The carrier(s) must be acceptable in the sense of being compatible with the other ingredients of the formula and not deleterious to the recipient thereof. When the individual components of the composition are administered separately, they are generally each presented as a pharmaceutical compositions. The references hereinafter to composition refer, unless otherwise stated, to compositions comprising either both the NK1 receptor antagonist and a compound of formula (I) as above defined, or a pharmaceutically acceptable salt or solvate thereof, or only one component thereof.

In one embodiment, the Sodium Channel blocker compound of formula (I) used in pharmaceutical compositions of the invention is (2*R*,5*R*)-2-(4-{[(2-fluorophenyl)methyl]oxy}phenyl)-7-methyl-1,7-diazaspiro[4.4]nonan-6-one,or a pharmaceutically accaptable salt or solvate thereof.

A subtherapeutic dose is intended to mean a dose of a drug below that required to produce significant clinical benefit for the patient when administered alone.

In a further aspect the invention provides a pharmaceutical composition comprising an NK1 receptor antagonist and a Sodium Channel blocker compound of formula (I), or a pharmaceutically acceptable salt or solvate thereof, for use in therapy.

In a further embodiment, the invention provides a pharmaceutical composition comprising an NK1 receptor antagonist and a Sodium Channel blocker compound of formula (I), or a pharmaceutically acceptable salt or solvate thereof, wherein at least one of them is at sub therapeutic dose, for use in therapy.

In another embodiment, the invention provides a pharmaceutical composition comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, for use in therapy.

In a still further embodiment, the invention provides a pharmaceutical composition comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, wherein at least one of them is at sub therapeutic dose, for use in therapy.

In one aspect, the invention provides a pharmaceutical composition comprising an NK1 receptor antagonist and a Sodium Channel blocker compound of formula (I) as above defined, or a pharmaceutically acceptable salt or solvate thereof, for use in the treatment of epilepsy.

In one embodiment, the invention provides a pharmaceutical composition comprising an NK1 receptor antagonist and a Sodium Channel blocker compound of formula (I) as above defined, or a pharmaceutically acceptable salt or solvate thereof, wherein at least one of them is at sub therapeutic dose, for use in the treatment of epilepsy.

In another aspect, the invention provides a pharmaceutical composition comprising an NK1 receptor antagonist and a Sodium Channel blocker compound of formula (I), or a pharmaceutically acceptable salt or solvate thereof, for use in the treatment of mood disorders or pain.

In further embodiment, the invention provides a pharmaceutical composition comprising an NK1 receptor antagonist and a Sodium Channel blocker compound of formula (I), or a pharmaceutically acceptable salt or solvate thereof, wherein at least one of them is at sub therapeutic dose, for use in the treatment of mood disorders or pain.

In one embodiment the invention provides a pharmaceutical composition comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, for use in the treatment of epilepsy.

In another embodiment the invention provides a pharmaceutical composition comprising an NK1 receptor antagonist and ((2*R*,5*R*)2-(4-{[(2-fluorophenyl)methyl]oxy}phenyl)-7-methyl-1,7-diazaspiro[4.4]nonan-6-one, or a pharmaceutically acceptable salt or solvate thereof, wherein at least one of them is at sub therapeutic dose, for use in the treatment of epilepsy.

In a further embodiment, the invention provides a pharmaceutical composition comprising an NK1 receptor antagonist and (2*R*,5*R*)-2-(4-([(2-fluorophenyl)methyl]oxy)phenyl)-7-methyl-1,7-diazaspiro[4.4]nonan-6-one, or a pharmaceutically acceptable salt or solvate thereof, for use in the treatment of mood disorders or pain.

In a still another embodiment, the invention provides a pharmaceutical composition comprising an NK1 receptor antagonist and ((2*R*,5*R*)-2-(4-{[(2-fluorophenyl)methyl]oxy}phenyl)-7-methyl-1,7-diazaspiro[4.4]nonan-6-one,or a pharmaceutically acceptable salt or solvate thereof, wherein at least one of them is at sub therapeutic dose, for use in the treatment of mood disorders or pain.

In a still further aspect, the invention provides the use of a pharmaceutical composition, comprising an NK1 receptor antagonist and a Sodium Channel blocker compound of formula (I) as above defined, or a pharmaceutically acceptable salt or solvate thereof, in the manufacture of a medicament for the treatment of epilepsy.

In one embodiment, the invention provides the use of a pharmaceutical composition, comprising an NK1 receptor antagonist and a Sodium Channel blocker compound of formula (I) as above defined, or a pharmaceutically acceptable salt or solvate thereof, wherein at least one of them is at sub therapeutic dose, in the manufacture of a medicament for the treatment of epilepsy.

In another embodiment, the invention provides the use of a pharmaceutical composition, comprising an NK1 receptor antagonist and a Sodium Channel blocker compound of formula (I) as above defined, or a pharmaceutically acceptable salt or solvate thereof, in the manufacture of a medicament for the treatment of mood disorders.

In a still further embodiment, the invention provides the use of a pharmaceutical composition, comprising an NK1 receptor antagonist and a Sodium Channel blocker compound of formula (I) as above defined, or a pharmaceutically acceptable salt or solvate thereof, wherein at least one of them is at sub therapeutic dose, in the manufacture of a medicament for the treatment of mood disorders.

In an embodiment, the invention provides the use of a pharmaceutical composition, comprising an NK1 receptor antagonist and (2*R*,5*R*)-2-(4-{[(2-fluorophenyl)methyl]oxy}phenyl)-7-methyl-1,7-diazaspiro[4.4]nonan-6-one,or a pharmaceutically acceptable salt or solvate thereof, in the manufacture of a medicament for the treatment of epilepsy.

In one embodiment, the invention provides the use of a pharmaceutical composition, comprising an NK1 receptor antagonist and (2*R*,5*R*)-2-(4-{[(2-fluorophenyl)methyl]oxy}phenyl)-7-methyl-1,7-diazaspiro[4.4]nonan-6-one,or a pharmaceutically acceptable salt or solvate thereof, wherein at least one of them is at sub therapeutic dose, in the manufacture of a medicament for the treatment of epilepsy.

In another embodiment, the invention provides the use of a pharmaceutical composition, comprising an NK1 receptor antagonist and (2*R*,5*R*)-2-(4-{[(2-fluorophenyl)methyl]oxy}phenyl)-7-methyl-1,7-diazaspiro[4.4]nonan-6-one,or a pharmaceutically acceptable salt or solvate thereof, in the manufacture of a medicament for the treatment of mood disorders.

In a still further embodiment, the invention provides the use of a pharmaceutical composition, comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, wherein at least one of them is at sub therapeutic dose, in the manufacture of a medicament for the treatment of mood disorders.

In an additional aspect, the invention provides the use of a pharmaceutical composition, comprising an NK1 receptor antagonist and a Sodium Channel blocker compound of formula (I) as above defined, or a pharmaceutically acceptable salt or solvate thereof, for the treatment of epilepsy.

In an additional embodiment, the invention provides the use of a pharmaceutical composition, comprising an NK1 receptor antagonist and a Sodium Channel blocker compound of formula (I) as above defined, or a pharmaceutically acceptable salt or solvate thereof, wherein at least one of them is at sub therapeutic dose, for the treatment of epilepsy.

In another aspect, the invention provides the use of a pharmaceutical composition, comprising an NK1 receptor antagonist and a Sodium Channel blocker compound of formula (I) as above defined, or a pharmaceutically acceptable salt or solvate thereof, for the treatment of mood disorders or pain.

In one embodiment, the invention provides the use of a pharmaceutical composition, comprising an NK1 receptor antagonist and a Sodium Channel blocker compound of formula (I) as above defined, or a pharmaceutically acceptable salt or solvate thereof, wherein at least one of them is at sub therapeutic dose, for the treatment of mood disorders or pain.

In an additional embodiment, the invention provides the use of a pharmaceutical composition, comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, for the treatment of epilepsy.

In an additional embodiment, the invention provides the use of a pharmaceutical composition, comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, wherein at least one of them is at sub therapeutic dose, for the treatment of epilepsy.

In an additional embodiment, the invention provides the use of a pharmaceutical composition, comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, for the treatment of mood disorders or pain.

In another embodiment, the invention provides the use of a pharmaceutical composition, comprising an NK1 receptor antagonist and (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, wherein at least one of them is at sub therapeutic dose, for the treatment of mood disorders or pain.

Within the context of the present invention, the term epilepsy is intented to include Seizure disorders and epilepsy syndromes. The various types of the Epilepsy and seizures mentioned hereinbelow are contemplated as part of the present invention: partial onset seizures (replacing temporal lobe epilepsy, neocortical epilepsy and Rasumssen's), generalized onset seizures, the seizures of the Lennox Gastaut syndrome (tonic, atonic, mycolonic, atypical absence and generalized tonic-clonic), absence seizure syndromes and juvenile myoclonic epilepsy.

Pharmaceutical compositions of the invention may also be useful in the treatment and/or prevention of disorders treatable and/or preventable with anti-convulsive agents, such as epilepsy including post-traumatic epilepsy, obsessive compulsive disorders (OCD), sleep disorders (including circadian rhythm disorders, insomnia & narcolepsy), tics (e.g. Giles de la Tourette's syndrome), ataxias, muscular rigidity (spasticity), and temporomandibular joint dysfunction.

Further diseases or conditions that may be treated by administration of the pharmaceutical compositions of the invention are selected from the list consisting of: psychotic disorders, mood disorders and pain.

Within the context of the present invention, the terms describing the Psychiatric indications used herein are classified in the Diagnostic and Statistical Manual of Mental Disorders, 4th Edition, published by the American Psychiatric Association (DSM-IV) and/or the International Classification of Diseases, 10th Edition (ICD-10). The various subtypes of the disorders mentioned herein are contemplated as part of the present invention. Numbers in brackets after the listed diseases below refer to the classification code in DSM-IV.

Within the context of the present invention, the term "psychotic disorder" includes:
i) Schizophrenia including the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and With Hallucinations; Substance-Induced Psychotic Disorder including the subtypes With Delusions (293.81) and With Hallucinations (293.82); and Psychotic Disorder Not Otherwise Specified (298.9).

Within the context of the present invention, the term "mood disorders" includes:
i) Depression and mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90).
   The term "mood disorders" also includes mood disorders in Epilepsy patients.

Within the context of the present invention, the term "pain" includes : chronic inflammatory pain (e.g. pain associated with rheumatoid arthritis, osteoarthritis, rheumatoid spondylitis, gouty arthritis and juvenile arthritis); musculoskeletal pain; lower back and neck pain; sprains and strains; neuropathic pain; sympathetically maintained pain; myositis; pain associated with cancer and fibromyalgia; pain associated with migraine; pain associated with cluster and chronic daily headache; pain associated with influenza or other viral infections, such as the common cold; rheumatic fever; pain associated with functional bowel disorders such as non-ulcer dyspepsia, non-cardiac chest pain and irritable bowel syndrome; pain associated with myocardial ischemia; post operative pain; headache; toothache; dysmenorrhea; neuralgia; fibromyalgia syndrome; complex regional pain syndrome (CRPS types I and II); neuropathic pain syndromes (including diabetic neuropathy; chemoterapeutically induced neuropathic pain; sciatica; non-specific lower back pain; multiple sclerosis pain;; HIV-related neuropathy; post-herpetic neuralgia; trigeminal neuralgia); and pain resulting from physical trauma, amputation, cancer, toxins or chronic inflammatory conditions.

In one embodiment, the "mood disorder" which may be treated by administration of the pharmaceutical compositions of the invention is a bipolar disorder.

It will be appreciated that references herein to "treatment" extend to prophylaxis, prevention of recurrence and suppression or amelioration of symptoms (whether mild, moderate or severe) as well as the treatment of established conditions.

In one embodiment, the pharmaceutical composition of the invention as herein above defined comprises a compound of formula (I) as above defined and generically and specifically disclosed in WO2007/042240.

In one embodiment, the pharmaceutical composition of the invention as herein above defined comprises one of the NK1 receptor antagonists generically and specifically disclosed in the following patent specifications whose disclosures are here incorporated by reference:
US Patent Specification Nos. 4839465, 5338845, 5594022, 6169097, 6197772, 6222038, 6204265, 6329392, 6316445, 2001039286, 2001034343, 2001029297, 2002193402, 2002147212, 2002147207, 2002143003 and 2002022624; and in European Patent Specification Nos. 284942, 327009, 333174, 336230, 360390, 394989, 428434, 429366, 436334, 443132, 446706, 482539, 484719, 499313, 512901, 512902, 514273, 514275, 517589, 520555, 522808, 525360, 528495, 532456, 533280, 577394, 591040, 615751, 684257, 1176144, 1110958, 1176144, 1172106, 1103545, and 1256578; and in International Patent Publication Nos. 90/05525, 90/05729, 91/02745, 91/12266, 91/18016, 91/18899, 92/01688, 92/06079, 92/15585, 92/17449, 92/20676, 92/21677, 92/22569, 93/00331, 93/01159, 93/01160, 93/01165, 93/01169, 93/01170, 94/01402, 94/26735, 95/06645, 95/08549, 95/14017, 95/16679, 95/18124, 95/23798, 95/28389, 95/33744, 96/05181, 96/18643, 96/21661, 96/29326, 96/32386, 96/34857, 96/37489, 97/02824, 97/05110, 97/08166, 97/13514, 97/14671, 97/16440, 97/17362, 97/19074, 97/19084, 97/19942, 97/21702, 97/22597, 97/22604, 97/23455, 97/24324, 97/24350, 97/25322, 97/25988, 97/27185, 97/30989, 97/30990, 97/30991, 97/32865, 97/38692, 97/44035, 97/49393, 97/49710, 98/02158, 98/04561, 98/07694, 98/07722, 98/08826, 98/13369, 98/17276, 98/18761, 98/18785, 98/18788, 98/20010, 98/24438, 98/24439, 98/24440, 98/24441, 98/24442, 98/24442, 98/24443, 98/24444, 98/24445, 98/24446, 98/24447, 98/28297, 98/43639, 98/45262, 98/49170, 98/54187, 98/57954, 98/57972, 99/00388, 99/01444, 99/01451, 99/07677, 99/07681, 99/09987, 99/21823, 99/24423, 99/25364, 99/26924, 99/27938, 99/36424, 99/52903, 99/59583, 99/59972, 99/62893, 99/62900, 99/64000, 00/02859, 00/06544, 00/06571, 00/06572, 00/06578, 00/06580, 00/15621, 00/20003, 00/21512, 00/21564, 00/23061, 00/23062, 00/23066, 00/23072, 00/20389, 00/25745, 00/26214, 00/26215, 00/34243, 00/34274, 00/39114, 00/47562, 01/77069, 01/25233, 01/30348, 01/87866, 01/94346, 01/90083, 01/87838, 01/85732, 01/77100, 01/77089, 01/77069, 01/46176, 01/46167, 01/44200, 01/32625, 01/29027, 01/25219, 02/32865, 02/00631, 02/81461, 02/92604, 02/38575, 02/57250, 02/22574, 02/74771, 02/26710, 02/28853, 02/102372, 02/85458, 02/81457, 02/74771, 02/62784, 02/60898, 02/60875, 02/51848, 02/51807, 02/42280, 02/34699, 02/32867, 02/32866, 02/26724, 02/24673, 02/24629, 02/18346, 02/16344, 02/16343, 02/16324, 02/12168, 02/08232 and 02/06236; and in British Patent Specification Nos. 2216529, 2266529, 2268931, 2269170, 2269590, 2271774, 2292144, 2293168, 2293169 and 2302689; and in Japanese Patent Specification No 6040995.

In a further embodiment, the pharmaceutical composition of the invention as hereinabove defined comprises an NK1 receptor antagonist selected from the group consisting of aprepitant (Emend^{™}), netupitant (R-1124), fosaprepitant (MK-0517), SSR-240600, cizolirtine, AV 608, TA-5538, E 6039 and nolpitantium besilate (SR 140333).

In a further embodiment, the pharmaceutical composition of the invention as hereinabove defined comprises an NK1 antagonist of formula (II) 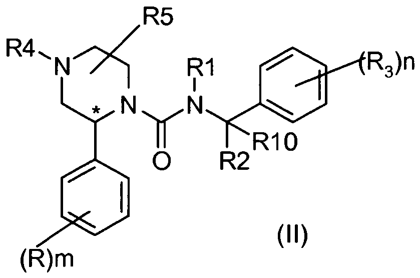 wherein
R represents a halogen atom or a C₁₋₄ alkyl group;
R1 represents hydrogen or a C₁₋₄alkyl group;
R2 represents hydrogen, a C₁₋₄ alkyl, C₂₋₆ alkenyl or a C₃₋₇ cycloalkyl group; or R1 and R2 together with nitrogen and carbon atom to which they are attached respectively represent a 5-6 membered heterocyclic group;
R3 represents a trifluoromethyl, a C₁₋₄ alkyl, a C₁₋₄ alkoxy, a trifluoromethoxy or a halogen group;
R4 represents hydrogen, a (CH₂)_{q}R7 or a (CH₂)ᵣCO(CH₂)ₚR7 group;
R5 represents hydrogen, a C₁₋₄ alkyl or a COR6 group;
R6 represents hydrogen, hydroxy, amino, methylamino, dimethylamino a 5 membered heteroaryl group containing 1 to 3 heteroatoms selected from oxygen, sulphur and nitrogen or a 6 membered heteroaryl group containing 1 to 3 nitrogen atoms;
R7 represents hydrogen, hydroxy or NR8R9 wherein R8 and R9 represent independently hydrogen or C₁₋₄alky) optionally substituted by hydroxy, or by amino;
R10 represents hydrogen, a C₁₋₄ alkyl group or
R10 together with R2 represents a a C₃₋₇ cycloalkyl group;
m is zero or an integer from 1 to 3; n is zero or an integer from 1 to 3; both p and r are independently zero or an integer from 1 to 4; q is an integer from 1 to 4; provided that , when R1 and R2 together with nitrogen and carbon atom to which they are attached respectively represent a 5 to 6 membered heterocyclic group, i) m is 1 or 2; ii) when m is 1, R is not fluorine and iii) when m is 2, the two substituents R are not both fluorine,
or a pharmaceutically acceptable salt or solvate thereof.

Compounds of formula (II) and pharmaceutically acceptable salts and solvates thereof are described in PCT publication No. WO01/25219, published 12 April 2001. Compounds of formula (II) and pharmaceutically acceptable salts and solvates thereof may be prepared by any method described in WO01/25219.

In a further embodiment, the pharmaceutical composition of the invention as herein above defined comprises an NK1 antagonist of formula (II) from the group consisting of:
2-(4-fluoro-2-methyl-phenyl)-piperazine-1-carboxylic acid (3,5-bis-trifluoromethyl-benzyl)-methyl-amide;
2-(2-Isopropyl-phenyl)-piperazine-1-carboxylic acid (3,5-bis-trifluoromethyl-benzyl)-methyl-amide;
2-(4-Fluoro-3-methyl-phenyl)-piperazine-1-carboxylic acid (3,5-bis-trifluoromethyl-benzyl)-methyl-amide;
2-(2,4-Difluoro-phenyl)-piperazine-1-carboxylic acid (3,5-bis-trifluoromethyl-benzyl)-methyl-amide;
2-(4-fluoro-2-methyl-phenyl)-piperazine-1-carboxylic acid [1-(3,5-bis-trifluoromethylphenyl)ethyl]-methyl-amide;
2-(4-Fluoro-phenyl)- piperazine-1-carboxylic acid (3,4-bis-trifluoromethyl-benzyl)-methylamide;
2-Phenyl-piperazine-1-carboxylic acid (3,5-bis-trifluoromethyl-benzyl)-methyl-amide; 2-(2,4-dichloro-phenyl)-piperazine-1-carboxylicacid (3,5-bistrifluoro methyl-benzyl)-methyl-amide;
2-(3,4-dichloro-phenyl)-piperazine-1-carboxylic acid (3,5-bistrifluoro methyl-benzyl)-methyl-amide;
2-(4-Fluoro-2-methyl-phenyl)-3-methyl-piperazine-1-carboxylic acid (3,5-bis-trifluoromethyl-benzyl)-methyl-amide;
2-(2-Methyl-4-Fluoro-phenyl)-6-Methyl-piperazine-1-carboxylic acid (3,5-bis-trifluoromethyl-benzyl)-methyl-amide;
2-(4-fluoro-2-methyl-phenyl)-piperazine-1-carboxylic acid [1-(3,5-bis-trifluoromethylphenyl)ethyl]-methyl-amide;
4-(2-Amino-acetyl)-2-(S)-(4-fluoro-2-methyl-phenyl)-piperazine-1-carboxylic acid (3,5-bis - trifluoromethyl-benzyl)-methyl-amide ;
2-(S)-(4-Fluoro-2-methyl-phenyl)-4-(piperidine-4-carbonyl)-piperazine-1-carboxylic acid (3,5-bis-trifluoromethyl-benzyl)-methyl-amide;
4-(2-Amino-ethyl)-2-(S)-(4-fluoro-2-methyl-phenyl)-piperazine-1-carboxylic acid (3,5-bis-trifluoromethyl-benzyl)-methyl-amide;
2-(S)-(4-Fluoro-2-methyl-phenyl)-piperazine-1-carboxylic acid [(1-3,5-bis-trifluoromethylphenyl)-cyclopropyl]-methyl-amide;
[2-(3,5-Bis-trifluoromethyl-phenyl)-pyrrolidin-1-yl]-[2-(S)-(4-fluoro-2-methyl-phenyl)-piperazin-1-yl]-methanone;
[2-(3,5-Bis-trifluoromethyl-phenyl)-3,6-dihydro-2*H*-pyridyn-1-yl]-[2-(S)-(4-fluoro-2-methylphenyl)-piperazin-1-yl]-methanone;
2-(3,5-Bis-trifluoromethyl-phenyl)-piperidin-1-yl]-[2-(S)-(4-fluoro-2-methyl-phenyl)-piperazin-1-yl]-methanone;
2-(4-Fluoro-2-methyl-phenyl)-piperazine-1-carboxylic acid [1-(3,5-bis-trifluoromethylphenyl)-but-3-enyl]-methyl-amide;
2-(4-Fluoro-2-methyl-phenyl)-piperazine-1-carboxylic acid [1-(3,5-bis-trifluoromethylphenyl)-2-methyl-propyl]-methyl-amide;
2-(4-Fluoro-2-methyl-phenyl)-piperazine-1-carboxylic acid [(3,5-bis-trifluoromethylphenyl)-cyclopropyl-methyl]-methyl-amide;
and enantiomers or pharmaceutically acceptable salts or solvates thereof.

In another embodiment, the pharmaceutical composition of the invention as herein above defined comprises an NK1 receptor antagonist of formula (II) which is : 2-(*S*)-(4-Fluoro-2-methyl-phenyl)-piperazine-1-carboxylic acid [1-(*R*)-(3,5-bis-trifluoromethyl-phenyl)-ethyl]-methyl-amide, or a pharmaceutically acceptable salt or solvate thereof.

In a still further embodiment, the pharmaceutical composition of the invention as herein above defined comprises an NK1 antagonist of formula (11) which is : 2-(*S*)-(4-Fluoro-2-methyl-phenyl)-piperazine-1-carboxylic acid [1-(*R*)-(3,5-bis-trifluoromethyl-phenyl)-ethyl]-methyl-amide methansulphonate.

In another embodiment, the pharmaceutical composition of the invention as herein above defined comprises an NK1 antagonist of formula (III) 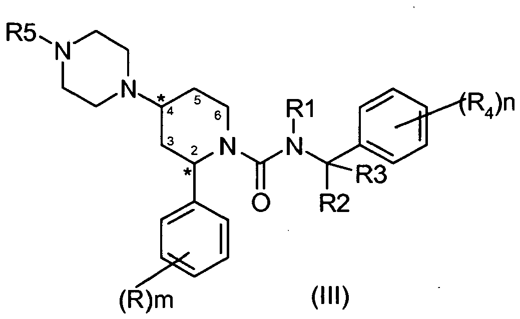 wherein
R represents a halogen atom or a C₁₋₄ alkyl group;
R₁ represents a C₁₋₄ alkyl group;
R₂ represents hydrogen or a C₁₋₄ alkyl group;
R₃ represents hydrogen or C₁₋₄ alkyl group;
R₄ represents a trifluoromethyl group;
R₅ represents hydrogen, a C₁₋₄ alkyl group or C(O)R₆;
R₆ represents C₁₋₄ alkyl, C₃₋₇ cycloalkyl, NH(C₁₋₄ alkyl) or N(C₁₋₄alkyl)₂;
m is zero or an integer from 1 to 3;
n is an integer from 1 to 3;
or a pharmaceutically acceptable salt or solvate thereof.

Compounds of formula (III) and pharmaceutically acceptable salts and solvates thereof are described in PCT publication No. WO02/32867, published 25 April 2002. Compounds of formula (III) and pharmaceutically acceptable salts and solvates thereof may be prepared by any method described in WO02/32867.

In a further embodiment, the pharmaceutical composition of the invention as herein above defined comprises an NK1 antagonist of formula (III) selected from the group consisting of:
4-(R)(4-Acetyl-piperazin-1-yl)-2-(R)-(4-fluoro-2-methyl-pheny)-piperidine-1-carboxylic acid, [1-(R)-(3,5-bis-trifluoromethyl-phenyl)-ethyl]-methylamide;
4-(S)-(4-Acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide;
4-(S)-(4-Acetyl-piperazin-1-yl)-2-(R)-(4-fluoro-2-methyl-phenyl)-piperidine-1-carboxylic acid, (3,5-bis-trifluoromethyl-benzyl)-methylamide;
4-(R)-(4-Acetyl-piperazin-1 -yl)-2-(R)-(4-fluoro-2-methyl-phenyl)-piperidine-1-carboxylic acid, (3,5-bis-trifluoromethyl-benzyl)-methylamide;
2-(R)-(4-Fluoro-2-methyl-phenyl)-4-(R,S)-(4-methyl-piperazin-1-yl)-piperidine-1-carboxylic acid, [1-(R)-(3,5-bis-trifluoromethyl-phenyl)-ethyl]-ethylamide;
2-(R)-(4-Fluoro-2-methyl-phenyl)4-(S)-piperazin-1-yl-piperidine-1-carboxylic acid, [1-(R)-(3,5-bis-trifluoromethyl-phenyl)ethyl]-methylamide;
2-(R)-(4-Fluoro-2-methyl-phenyl)4-(R,S)-(4-methyl-piperazin-1-yl)-piperidine-1-carboxylic acid, (3,5-bis-trifluoromethyl-benzyl)-methylamide ;
4-(S)-(4-Cyclopropanoyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide
4-(R)-(4-Cyclopropanoyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide;
4-(S)-[4-(2-Methyl-propanoyl)-piperazin-1-yl]-2-(R)-(4-fluoro-2-methyl-phenyl)-piperidine-1-carboxylic acid, [1-(R)-(3,5-bis-trifluoromethyl-phenyl)-ethyl]-methylamide;
4-(R)-[4-(2-Methyl-propanoyl)-piperazin-1-yl]-2-(R)-(4-fluoro-2-methyl-phenyl)-piperidine-1-carboxylic acid, [1-(R)-(3,5-bis-trifluoromethyl-phenyl)-ethyl]-methylamide;
4-(S)-[1-[(3,5-Bis-trifluoromethyl-benzyl)-methyl-carbamoyl]-2-(R)-(4-fluoro-2-methylphenyl)-piperidin-4-yl]-piperazine-1-carboxylic acid, dimethylamide;
4-(S)[1-[(3,5-Bis-trifluoromethyl-benzyl)-methyl-carbamoyl]-2-(R)-(4-fluoro-2-methylphenyl)-piperidin-4-yl]-1-carboxylic acid, methylamide;
4-(S)[1-[(3,5-Bis-trifluoromethyl-benzyl)-methyl-carbamoyl]-2-(R)(4-fluoro-2-methylphenyl)-piperidin-4-yl]-piperazine;
4-(S)-(4-Acetyl-piperazin-1-yl)-2-(R)-(4-fluoro-phenyl)-piperidine-1-carboxylic acid, [1-(R)-(3,5-bis-trifluoromethyl-phenyl)-ethyl]-methylamide;
4-(R)-(4-Acetyl-piperazin-1-yl)-2-(R)-(4-fluoro-phenyl)-piperidine-1-carboxylic acid, [1-(R)-(3,5-bis-trifluoromethyl-phenyl)-ethyl]-methylamide; and pharmaceutically acceptable salts and solvates thereof.

In another embodiment, the pharmaceutical composition of the invention as herein above defined comprises an NK1 receptor antagonist of formula (III) which is 4-(S)-(4-acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide, or a pharmaceutically acceptable salt or solvate thereof.

In a still further embodiment, the pharmaceutical composition of the invention as herein above defined comprises an NK1 antagonist of formula (III) which is 4-(S)-(4-acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide methanesulphonate.

In a still another embodiment, the pharmaceutical composition of the present invention as herein above defined comprises an NK1 antagonist of formula (IV) 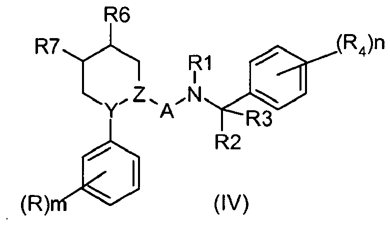 R represents halogen or C₁₋₄ alkyl ;
R₁ represents C₁₋₄ alkyl;
R₂ or R₃ independently represent hydrogen or C₁₋₄ alkyl;
R₄ represents trifluoromethyl, C₁₋₄ alkyl, C₁₋₄ alkoxy, trifluoromethoxy or halogen;
R₅ represents hydrogen , C₁₋₄ alkyl or C₃₋₇ cycloalkyl;
R₆ is hydrogen and R₇ is a radical of formula (W): 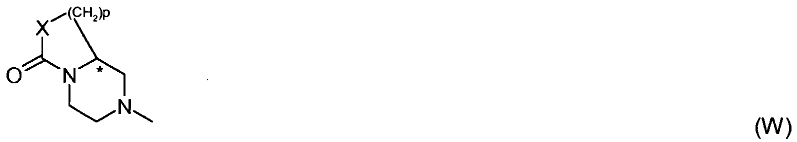 or R₆ is a radical of formula (W) and R₇ is hydrogen;
X represents CH₂, NR₅ or O;
Y represents Nitrogen and Z is CH or Y represents CH and Z is Nitrogen;
A represents C(O) or S(O)q, provided that when Y is nitrogen and Z is CH, A is not S(O)q;
m is zero or an integer from 1 to 3;
n is an integer from 1 to 3;
p and q are independently an integer from 1 to 2;
or a pharmaceutically acceptable salt or solvate thereof.

Compounds of formula (IV) and pharmaceutically acceptable salts and solvates thereof are described in PCT publication No. WO 03/066635, published 14 August 2003. Compounds of formula (IV) and pharmaceutically acceptable salts and solvates thereof may be prepared by any method described in WO 03/066635.

In a further embodiment, the pharmaceutical composition of the invention as herein above defined comprises an NK1 antagonist of formula (IV) selected from the group consisting of:
2-(R)-(4-Fluoro-2-methyl-phenyl)-4-(S)-(6-oxo-hexahydro-pyrrolo[1,2-*a*]-pyrazin-2-yl)-piperidine-1-carboxylic acid (3,5-bis-trifluoromethyl-benzyl)-methylamide;
2-(R)-(4-Fluoro-2-methyl-phenyl)-4-(S)-(6-oxo-hexahydro-pyrrolo[1,2-*a*]-pyrazin-2-yl)-piperidine-1-carboxylic acid[1-(R)-(3,5-bis-trifluoromethyl-phenyl)-ethyl]-methylamide;
1-(4-Fluoro-2-methyl-phenyl)-4-(6-oxo-hexahydro-pyrrolo[1,2-*a*]pyrazin-2-yl)-piperidine-2-carboxylic acid (3,5-bis-trifluoromethyl-benzyl)-methyl-amide;
and enantiomers, diastereoisomers pharmaceutically acceptable salts (e.g. hydrochloride, methanesulphonate or maleate) and solvates thereof.

In a still further embodiment, the pharmaceutical composition of the invention as herein above defined comprises an NK1 receptor antagonist of formula (IV) which is 2-(R)-(4-Fluoro-2-methyl-phenyl)-4-(S)-((8aS)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide; or amorphous and crystalline forms thereof and pharmaceutically acceptable salts (e.g. hydrochloride or maleate) and solvates thereof.

In a still further embodiment, the pharmaceutical composition of the invention as herein above defined comprises an NK1 receptor antagonist of formula (IV) which is 2-(R)-(4-Fluoro-2-methyl-phenyl)-4-(S)-((8aR)-6-oxo-hexahydro-pyrrolo[1,2-*a*]-pyrazin-2-yl)-piperidine-1-carboxylic acid [1-(R)-(3,5-bis-trifluoromethyl-phenyl)-ethyl]-methylamide; or amorphous and crystalline forms thereof and pharmaceutically acceptable salts (e.g. hydrochloride or maleate) and solvates thereof.

In a still another embodiment, the pharmaceutical composition of the present invention as herein above defined comprises an NK1 antagonist of formula (V) 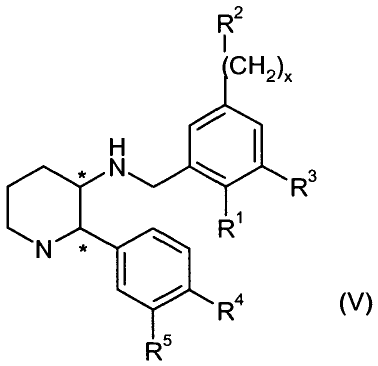 wherein R¹ is a C₁₋₄alkoxy group;
R² is 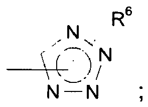 R³ is a hydrogen or halogen atom;
R⁴ and R⁵ may each independently represent a hydrogen or halogen atom, or a C₁₋₄alkyl, C₁₋₄alkoxy or trifluoromethyl group;
R⁶ is a hydrogen atom, a C₁-₄alkyl, (CH₂)ₘcyclopropyl, -S(O)ₙC₁₋₄alkyl, phenyl, NR⁷R⁸, CH₂C(O)CF₃ or trifluoromethyl group;
R⁷ and R⁸ may each independently represent a hydrogen atom, or a C₁₋₄alkyl or acyl group;
x represents zero or 1;
n represents zero, 1 or 2;
m represents zero or 1;
or a pharmaceutically acceptable salt or solvate thereof.

Compounds of formula (V) and pharmaceutically acceptable salts and solvates thereof are described in PCT publication No. WO95/08549, published 30 March 1995. Compounds of formula (V) and pharmaceutically acceptable salts and solvates thereof may be prepared by any method described in WO95/08549.

In a further embodiment, the pharmaceutical composition of the invention as herein above defined comprises an NK1 antagonist of formula (V) selected from the group consisting of:
[2-Methoxy-5-(5-phenyl-tetrazol-1-yl)-benzyl]-(2S-phenyl-piperidin-3S-yl)-amine;
[2-Methoxy-5-(5-methylimino-4,5-dihydro-tetrazol-1-yl)-benzyl]-(2S-phenyl-piperidin-3S-yl)-amine;
N-(1-{4-Methoxy-3-[(2S-phenyl-piperidin-3S-ylamino)-methyl]-phenyl}-1H-tetrazol-5-yl)-acetamide;
[5-(5-Dimethylamino-tetrazol-1-yl)-2-methoxy-benzyl]-(2S-phenyl-piperidin-3S-yl)-amine; [5-(5-Diethylamino-tetrazol-1-yl)-2-methoxy-benzyl]-(2S-phenyl-piperidin-3S-yl)-amine;
1,1,1-Trifluoro-3-(1-{4-methoxy-3-[(2S-phenyl-piperidin-3S-ylamino)-methyl]-phenyl}-1H-tetrazol-5-yl)-propan-2-one;
[5-(5-Methanesulfonyl-tetrazol-1-yl)-2-methoxy-benzyl]-(2S-phenyl-piperidin-3S-yl)-amine;
[3-Chloro-2-methoxy-5-(5-methyl-tetrazol-1-yl)-benzyl]-(2S-phenyl-piperidin-3S-yl)-amine; [2S-(4-Fluoro-phenyl)-piperidin-3S-yl]-[2-methoxy-5-(5-trifluoromethyl-tetrazol-1-yl)-benzyl]-amine;
(2S,3S)-[2-(4-Fluorophenyl)-piperidin-3-yl]-(2-methoxy-5-tetrazol-1-yl-benzyl)-amine;
(5-(5-Amino-tetrazol-1-yl)-2-methoxy-benzyl]-(2S-phenyl-piperidin-3S-yl)-amine;
(2-Ethoxy-5-tetrazol-1-yl-benzyl)-([2S,3S]-2-phenyl piperidin-3-yl) amine;
(2-Isopropoxy-5-tetrazol-1-ylbenzyl)-[(2S,3S]-2-phenyl piperidin-3-yl) amine;
and pharmaceutically acceptable salts and solvates thereof.

In a further embodiment, the pharmaceutical composition of the invention as herein above defined comprises an NK1 antagonist of formula (V) selected from the group consisting of:
(2-Methoxy-5-tetrazol-1-yl-benzyl)-(2S-phenyl-piperidin-3S-yl)-amine;
[2-Methoxy-5-(5-trifuoromethyl-tetrazol-1-yl)-benzyl]-(2S-phenyl-piperidin-3S-yl)-amine;
and pharmaceutically acceptable salts, including the dihydrochloride salts, and solvates thereof.

In a further embodiment, the pharmaceutical composition of the invention as herein above defined comprises an NK1 antagonist which is [2-Methoxy-5-(5-trifuoromethyl-tetrazol-1-yl)-benzyl]-(2S-phenyl-piperidin-3S-yl)-amine;
or pharmaceutically acceptable salts, including the dihydrochloride salts, and solvates thereof.

In a still another embodiment, the pharmaceutical composition of the present invention as herein above defined comprises an NK1 receptor antagonist of formula (VI): 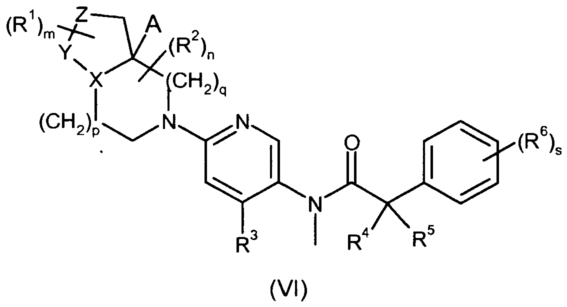 wherein:
X represents a nitrogen atom;
Y represents -C(H₂)-, (-C(H₂)-)₂, -S(O₂)- or -C(=O)-;
Z represents -C(H₂)-, -S(O₂)-, -N(R^{z})-, or an oxygen or sulphur atom;
A represents hydrogen or -CH₂OH;
R^{z} represents hydrogen, C₁₋₆ alkyl, C₁₋₆ alkoxy, -COR⁷ or -SO₂R⁷;
R¹ represents halogen, C₁₋₆alkyl, C₁₋₆ alkoxy, =O, haloC₁₋₆ alkyl, haloC₁₋₆ alkoxy, hydroxyl or -CH₂OH;
m represents an integer from 0 to 3;
R² represents halogen, =O, C₁₋₆ alkyl (optionally substituted by one or more hydroxyl groups), -COOR⁷, -CONR⁷R⁸, C₁₋₆ alkoxy, haloC₁₋₆ alkyl, haloC₁₋₆ alkoxy or C₁₋₆alkylOC₁₋₆alkyl;
n represents an integer from 0 to 3;
p and q independently represent an integer from 0 to 2;
R³ represents an -aryl, -heteroaryl, -heterocyclyl, -aryl-aryl, -aryl-heteroaryl, -aryl-heterocyclyl, -heteroaryl-aryl, -heteroaryl-heteroaryl, -heteroaryl-heterocyclyl, - heterocyclyl-aryl, -heterocyclyl-heteroaryl or -heterocyclyl-heterocyclyl group, all of which may be optionally substituted by one or more (e.g. 1, 2 or 3) halogen, C₁₋₆ alkyl (optionally substituted by one or more hydroxyl groups), C₃₋₈ cycloalkyl, C₁₋₆ alkoxy, hydroxyl, haloC₁₋₆ alkyl, haloC₁₋₆ alkoxy, cyano, -S-C₁₋₆ alkyl, -SO-C₁₋₆ alkyl, -SO₂-C₁₋₆ alkyl, -COR⁷, - CONR⁷R⁸, -NR⁷R⁸, -NR⁷COC₁₋₆ alkyl, -NR⁷SO₂C₁₋₆ alkyl, C₁₋₆ alkyl-NR7⁷R⁸, -OCONR⁷R⁸,-NR⁷CO₂R⁸ or -SO₂NR⁷R⁸ groups;
R⁴ and R⁵ independently represent C₁₋₆ alkyl, or R⁴ and R⁵ together with the carbon atom to which they are attached may together form a C₃₋₈ cycloalkyl group;
R⁶ represents halogen, C₁-₆ alkyl, C₃-₈ cycloalkyl, C₁₋₆ alkoxy, haloC₁₋₆ alkyl or haloC₁₋₆ alkoxy;
s represents an integer from 0 to 4;
R⁷ and R⁸ independently represent hydrogen, C₁₋₆ alkyl or C₃₋₈ cycloalkyl;
or a pharmaceutically acceptable salt or solvate thereof.

Compounds of formula (VI) and pharmaceutically acceptable salts and solvates thereof are described in PCT publication No. WO2007/028654, published 15 March 2007. Compounds of formula (VI) and pharmaceutically acceptable salts and solvates thereof may be prepared by any method described in WO2007/028654.

In a further embodiment, the pharmaceutical composition of the invention as herein above defined comprises an NK1 antagonist of formula (VI) selected from the group consisting of:
2-[3,5-bis(trifluoromethyl)phenyl]-*N*-{4-(4-fluoro-2-methylphenyl)-6-[(7*S*,9a*S*)-7-(hydroxymethyl)hexahydropyrazino[2,1-*c*][1,4]oxazin-8(1*H*)-yl]-3-pyridinyl}-*N*,2-dimethylpropanamide,
2-[3,5-bis(trifluoromethyl)phenyl]-*N*-{4-(4-fluoro-2-methylphenyl)-6-[(7*S*,9a*R*)-7-(hydroxymethyl)hexahydropyrazino[2,1-*c*][1,4]oxazin-8(1*H*)-yl]-3-pyridinyl}-*N*,2-dimethylpropanamide,
2-[3,5-bis(trifluoromethyl)phenyl]-*N*-{4-(4-fluoro-2-methylphenyl)-6-[(7*S*)-7-(hydroxymethyl)-2,2-dioxidohexahydropyrazino[2,1-*c*][1,4]thiazin-8(1*H*)yl]-3-pyridinyl}-*N*,2-dimethylpropanamide,
*N*-[6-[(3*S*)-8-acetyl-3-(hydroxymethyl)octahydro-2*H*-pyrazino[1,2-*a*]pyrazin-2-yl]-4-(4-fluoro-2-methylphenyl)-3-pyridinyl]-2-[3,5-bis(trifluoromethyl)phenyl]-*N*,2-dimethylpropanamide,
and pharmaceutically acceptable salts and solvates thereof.

In a further embodiment, the pharmaceutical composition of the invention as herein above defined comprises an NK1 antagonist which is 2-[3,5-Bis(trifluoromethyl)phenyl]-*N*-{4-(4-fluoro-2-methylphenyl)-6-[(7*S*,9a*S*)-7-(hydroxymethyl)hexahydropyrazino[2,1-*c*][1,4]oxazin-8(1*H*)-yl]-3-pyridinyl}-*N*,2-dimethylpropanamide or pharmaceutically acceptable salts, including the hydrochloride salt, and solvates thereof.

In one embodiment, the pharmaceutical composition of the invention as herein above defined comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and one of the NK1 receptor antagonists selected from the group consisting of: aprepitant (Emend^{™}); netupitant (R-1124); fosaprepitant (MK-0517); SSR-240600; cizolirtine; AV 608; TA-5538; E 6039; nolpitantium besilate (SR 140333); 2-(*S*)-(4-Fluoro-2-methylphenyl)-piperazine-1-carboxylic acid [1-(*R*)-(3,5-bis-trifluoromethyl-phenyl)-ethyl]-methylamide, or a pharmaceutically acceptable salt or solvate thereof; 4-(S)-(4-acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide, or a pharmaceutically acceptable salt or solvate thereof; 2-(R)-(4-Fluoro-2-methyl-phenyl)-4-(S)-((8aS)-6-oxo-hexahydro-pyrrolo[1,2-*a*]-pyrazin-2-yl)-piperidine-1-carboxylic acid [1-(R)-(3,5-bis-trifluoromethylphenyl)-ethyl]-methylamide, or a pharmaceutically acceptable salt or solvate thereof; [2-Methoxy-5-(5-trifuoromethyl-tetrazol-1-yl)-benzyl]-(2S-phenyl-piperidin-3S-)-amine, or a pharmaceutically acceptable salt or solvate thereof; 2-(R)-(4-Fluoro-2-methyl-phenyl)-4-(S)-((8aR)-6-oxo-hexahydro-pyrrolo[1,2-*a*]-pyrazin-2-yl)-piperidine-1-carboxylicacid [1-(R)-(3,5-bis-trifluoromethyl-phenyl)-ethyl]-methylamide, or a pharmaceutically acceptable salt or solvate thereof and 2-[3,5-Bis(trifluoromethyl)phenyl]-*N*-{4-(4-fluoro-2-methylphenyl)-6-[(7*S*,9a*S*)-7-(hydroxymethyl)hexahydropyrazino[2,1-*c*][1,4]oxazin-8(1*H*)-yl]-3-pyridinyl}-*N*,2-dimethylpropanamide, or a pharmaceutically acceptable salt or solvate thereof.

In one embodiment, the pharmaceutical composition of the invention as herein above defined comprises (2*R*,5*R*)-2-(4-{[(2-fluorophenyl)methyl]oxy}phenyl)-7-methyl-1,7-diazaspiro[4.4]nonan-6-one,or a pharmaceutically acceptable salt or solvate thereof, at subtherapeutic dose and one of the NK1 receptor antagonists selected from the group consisting of: aprepitant (Emend^{™}); netupitant (R-1124); fosaprepitant (MK-0517); SSR-240600; cizolirtine; AV 608; TA-5538; E 6039; nolpitantium besilate (SR 140333); 2-(*S*)-(4-Fluoro-2-methyl-phenyl)-piperazine-1-carboxylic acid [1-(*R*)-(3,5-bis-trifluoromethylphenyl)-ethyl]-methyl-amide, or a pharmaceutically acceptable salt or solvate thereof; 4-(S)-(4-acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide, or a pharmaceutically acceptable salt or solvate thereof; 2-(R)-(4-Fluoro-2-methyl-phenyl)-4-(S)-((8aS)-6-oxo-hexahydro-pyrrolo[1,2-*a*]-pyrazin-2-yl)-piperidine-1-carboxylic acid [1-(R)-(3,5-bis-trifluoromethyl-phenyl)-ethyl]-methylamide, or a pharmaceutically acceptable salt or solvate thereof; [2-Methoxy-5-(5-trifuoromethyl-tetrazol-1-yl)-benzyl]-(2S-phenyl-piperidin-3S-yl)-amine, or a pharmaceutically acceptable salt or solvate thereof; 2-(R)-(4-Fluoro-2-methyl-phenyl)-4-(S)-((8aR)-6-oxo-hexahydro-pyrrolo[1,2-*a*]-pyrazin-2-yl)-piperidine-1-carboxylic acid [1-(R)(3,5-bis-trifluoromethyl-phenyl)-ethyl]-methylamide,or a pharmaceutically acceptable salt or solvate thereof and 2-[3,5-Bis(trifluoromethyl)phenyl]-*N*-{4-(4-fluoro-2-methylphenyl)-6-[(7*S*,9a*S*)-7-(hydroxymethyl)hexahydropyrazino[2,1-*c*][1,4]oxazin-8(1*H*)-yl]-3-pyridinyl}-*N*,2-dimethylpropanamide, or a pharmaceutically acceptable salt or solvate thereof.

In one embodiment, the pharmaceutical composition of the invention as herein above defined comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and one of the NK1 receptor antagonists selected from the group consisting of: aprepitant (Emend^{™}); netupitant (R-1124); fosaprepitant (MK-0517); SSR-240600; cizolirtine; AV 608; TA-5538; E 6039; nolpitantium besilate (SR 140333); 2-(*S*)-(4-Fluoro-2-methylphenyl)-piperazine-1-carboxylic acid [1-(*R*)-(3,5-bis-trifluoromethyl-phenyl)-ethyl]-methylamide, or a pharmaceutically acceptable salt or solvate thereof; 4-(S)-(4-acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide, or a pharmaceutically acceptable salt or solvate thereof; 2-(R)-(4-Fluoro-2-methyl-phenyl)-4-(S)-((8aS)-6-oxo-hexahydro-pyrrolo[1,2-*a*]-pyrazin-2-yl)-piperidine-1-carboxylic acid [1-(R)-(3,5-bis-trifluoromethylphenyl)-ethyl]-methylamide, or a pharmaceutically acceptable salt or solvate thereof; [2-Methoxy-5-(5-trifuoromethyl-tetrazol-1-yl)-benzyl]-(2S-phenyl-piperidin-3S-yl)-amine, or a pharmaceutically acceptable salt or solvate thereof; 2-(R)-(4-Fluoro-2-methyl-phenyl)-4-(S)-((8aR)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide, or a pharmaceutically acceptable salt or solvate thereof and 2-[3,5-Bis(trifluoromethyl)phenyl]-*N*-{4-(4-fluoro-2-methylphenyl)-6-[(7S,9aS)-7-(hydroxymethyl)hexahydropyrazino[2,1-*c*][1,4]oxazin-8(1*H*)-yl]-3-pyridinyl}-*N*,2-dimethylpropanamide, or a pharmaceutically acceptable salt or solvate thereof, such NK1 receptor antagonist being administered at sub therapeutic dose.

In one embodiment, the pharmaceutical composition of the invention as herein above defined comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and one of the NK1 receptor antagonists selected from the group consisting of: aprepitant (Emend^{™}); netupitant (R-1124); fosaprepitant (MK-0517); SSR-240600; cizolirtine; AV 608; TA-5538; E 6039; nolpitantium besilate (SR 140333); 2-(S)-(4-Fluoro-2-methylphenyl)-piperazine-1-carboxylic acid [1-(*R*)-(3,5-bis-trifluoromethyl-phenyl)-ethyl]-methylamide, or a pharmaceutically acceptable salt or solvate thereof; 4-(S)-(4-acetyl-piperazin-1-yl)2-(R)(4-fluoro-2-methyl-phenyl)-piperidine-1-carboxylic acid, [1-(R)-(3,5-bis-trifluoromethyl-phenyl)-ethyl]-methylamide, or a pharmaceutically acceptable salt or solvate thereof; 2-(R)-(4-Fluoro-2-methyl-phenyl)-4-(S)-((8aS)-6-oxo-hexahydro-pyrrolo[1,2-a]-pyrazin-2-yl)-piperidine-1-carboxylicacid [1-(R)-(3,5-bis-trifluoromethylphenyl)-ethyl]-methylamide, or a pharmaceutically acceptable salt or solvate thereof; [2-Methoxy-5-(5-trifuoromethyl-tetrazol-1-yl)-benzyl]-(2S-phenyl-piperidin-35-yl)-amine, or a pharmaceutically acceptable salt or solvate thereof; 2-(R)-(4-Fluoro-2-methyl-phenyl)-4-(S)-((8aR)-6-oxo-hexahydro-pyrrolo[1,2-*a*]-pyrazin-2-yl)piperidine-1-carboxylic acid [1-(R)-(3,5-bis-trifiuoromethyl-phenyl)-ethyl]-methylamide, or a pharmaceutically acceptable salt or solvate thereof and 2-[3,5-Bis(trifluoromethyl)phenyl]-*N*-{4-(4-fluoro-2-methylphenyl)-6-[(7S,9aS)-7-(hydroxymethyl)hexahydropyrazino[2,1-*c*][1,4]oxazin-8(1*H*)-yl]-3-pyridinyl}-*N*,2-dimethylpropanamide, or a pharmaceutically acceptable salt or solvate thereof, such NK1 receptor antagonist and Sodium Channel blocker compounds being both administered at sub therapeutic dose.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof and 2-(*S*)-(4-Fluoro-2-methyl-phenyl)-piperazine-1-carboxylic acid [1-(*R*)-(3,5-bis-trifluoromethyl-phenyl)-ethyl]-methyl-amide, or a pharmaceutically acceptable salt or solvate thereof.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, at sub therapeutic dose and 2-(*S*)-(4-Fluoro-2-methyl-phenyl)-piperazine-1-carboxylic acid [1-(*R*)-(3,5-bis-trifluoromethyl-phenyl)-ethyl]-methyl-amide, or a pharmaceutically acceptable salt or solvate thereof.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and 2-(*S*)-(4-Fluoro-2-methyl-phenyl)-piperazine-1-carboxylic acid [1-(*R*)-(3,5-bis-trifluoromethyl-phenyl)-ethyl]-methyl-amide, or a pharmaceutically acceptable salt or solvate thereof, at sub therapeutic dose.

In an additional embodiment the pharmaceutical composition of the invention comprises comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and 4-(S)-(4-acetyl-piperazin-1-yl)-2-(R)-(4-fluoro-2-methyl-phenyl)-piperidine-1-carboxylic acid, [1-(R)(3,5-bis-trifluoromethyl-phenyl)-ethyl]-methylamide, or a pharmaceutically acceptable salt or solvate thereof.

In an additional embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, at subtherapeutic dose and 4-(S)-(4-acetyl-piperazin-1-yl)-2-(R)-(4-fluoro-2-methyl-phenyl)-piperidine-1-carboxylic acid, [1-(R)(3,5-bis-trifluoromethyl-phenyl)-ethyl]-methylamide,or a pharmaceutically acceptable salt or solvate thereof.

In an additional embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and 4-(S)-(4-acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide, or a pharmaceutically acceptable salt or solvate thereof, at subtherapeutic dose.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and 2-(R)-(4-Fluoro-2-methyl-phenyl)-4-(S)-((8aS)-6-oxo-hexahydro-pyrrolo[1,2-*a*]-pyrazin-2-yl)-piperidine-1-carboxylic acid [1-(R)-(3,5-bis-trifluoromethyl-phenyl)-ethyl]-methylamide,or a pharmaceutically acceptable salt or solvate thereof.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, at subtherapeutic dose and 2-(R)-(4-Fluoro-2-methyl-phenyl)-4-(S)-((8aS)-6-oxo-hexahydro-pyrrolo[1,2-a]-pyrazin-2-yl)-piperidine-1-carboxylic acid [1-(R)-(3,5-bis-trifluormethyl-phenyl)-ethyl]-methylamid, or a pharmaceutically acceptable salt or solvate thereof.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and 2-(R)-(4-Fluoro-2-methyl-phenyl)-4-(S)-((8aS)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide, or a pharmaceutically acceptable salt or solvate thereof, at subtherapeutic dose.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and 2-(R)-(4-Fluoro-2-methyl-phenyl)-4-(S)-((8aR)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide,or a pharmaceutically acceptable salt or solvate thereof.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, at subtherapeutic dose and 2-(R)-(4-Fluoro-2-methyl-phenyl)-4-(S)((8aR)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide,or a pharmaceutically acceptable salt or solvate thereof.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and 2-(R)-(4-Fluoro-2-methyl-phenyl)-4-(S)-((8aR)-6-oxo-hexahydro-pyrrolo[1,2-*a*]-pyrazin-2-yl)-piperidine-1-carboxylicacid [1-(R)-(3,5-bis-trifluoromethyl-phenyl)-ethyl]-methylamide, or a pharmaceutically acceptable salt or solvate thereof, at subtherapeutic dose.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and [2-Methoxy-5-(5-trifuoromethyl-tetrazol-1-yl)-benzyl]-(2S-phenyl-piperidin-3S-yl)-amine, or a pharmaceutically acceptable salt or solvate thereof.

In a further embodiment the pharmaceutical composition of the invention comprises (2*R*,5*R*)-2-(4-{[(2-fluorophenyl)methyl]oxy}pheny)-7-methyl-1,7-diazaspiro[4.4]nonan-6-one, or a pharmaceutically acceptable salt or solvate thereof, at subtherapeutic dose and [2-Methoxy-5-(5-trifuoromethyl-tetrazol-1-yl)-benzyl]-(2S-phenyl-piperidin-3S-yl)-amine, or a pharmaceutically acceptable salt or solvate thereof.

In a further embodiment the pharmaceutical composition of the invention comprises (2*R*,5*R*)-2-(4-{[(2-fluorophenyl)methyl]oxy}pheny)-7-methyl-1,7-diazaspiro[4.4]nonan-6-one, or a pharmaceutically acceptable salt or solvate thereof, and [2-Methoxy-5-(5-trifuoromethyl-tetrazol-1-yl)-benzyl]-(2S-phenyl-piperidin-3S-yl)-amine, or a pharmaceutically acceptable salt or solvate thereof, at subtherapeutic dose.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and 2-[3,5-Bis(trifluoromethyl)phenyl]-*N*-{4-(4-fluoro-2-methylphenyl)-6-[(7*S*,9a*S*)-7-(hydroxymethyl)hexahydropyrazino[2,1-*c*][1,4]oxazin-8(1*H*)-yl]-3-pyridinyl}-*N*,2-dimethylpropanamide, or a pharmaceutically acceptable salt or solvate thereof.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, at subtherapeutic dose and 2-[3,5-Bis(trifluoromethyl)phenyl]-*N*-{4-(4-fluoro-2-methylphenyl)-6-[(7*S*,9a*S*)-7-(hydroxymethyl)hexahydropyrazino[2,1-*c*][1,4]oxazin-8(1*H*)-yl]-3-pyridinyl}-*N*,2-dimethylpropanamide, or a pharmaceutically acceptable salt or solvate thereof.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, and 2-[3,5-Bis(trifluoromethyl)phenyl]-*N*-{4-(4-fluoro-2-methylphenyl)-6-[(7*S*,9a*S*)-7-(hydroxymethyl)hexahydropyrazino[2,1-*c*][1,4]oxazin-8(1*H*)-yl]-3-pyridinyl}-*N*,2-dimethylpropanamide, or a pharmaceutically acceptable salt or solvate thereof, at subtherapeutic dose.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate and 2-(*S*)-(4-Fluoro-2-methyl-phenyl)-piperazine-1-carboxylic acid [1-(*R*)-(3,5-bis-trifluoromethyl-phenyl)-ethyl]-methyl-amide methansulphonate.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate at sub therapeutic dose and 2-(*S*)-(4-Fluoro-2-methylphenyl)-piperazine-1-carboxylic acid [1-(*R*)-(3,5-bis-trifluoromethyl-phenyl)-ethyl]-methylamide methansulphonate.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate and 2-(*S*)-(4-Fluoro-2-methyl-phenyl)-piperazine-1-carboxylic acid [1-(*R*)-(3,5-bis-trifluoromethyl-phenyl)-ethy]-methyl-amide methansulphonate, at sub therapeutic dose.

In an additional embodiment the pharmaceutical composition of the invention comprises comprises (2*R*,5*R*)-2-(4-{[(2-fluorophenyl)methyl]oxy}phenyl)-7-methyl-1,7-diazaspiro[4.4]nonan-6-one 4-methylbenzenesulfonate and 4-(S)-(4-acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide methanesulphonate.

In an additional embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate at subtherapeutic dose and 4-(S)-(4-acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide methanesulphonate.

In an additional embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate and 4-(S)-(4-acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide methanesulphonate at subtherapeutic dose.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one4-methylbenzenesulfonate and 2-(R)-(4-Fluoro-2-methyl-phenyl)-4-(S)-((8aS)-6-oxo-hexahydro-pyrrolo[1,2-*a*]-pyrazin-2-yl)-piperidine-1-carboxylic acid [1-(R)-(3,5-bis-trifluoromethyl-phenyl)-ethyl]-methylamide hydrochloride.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate at subtherapeutic dose and 2-(R)-(4-Fluoro-2-methyl-phenyl)-4-(S)-((8aS)-6-oxo-hexahydro-pyrrolo[1,2-*a*]-pyrazin-2-yl)-piperidine-1-carboxylic acid [1-(R)-(3,5-bis-trifluoromethyl-pheny)-ethyl]-methylamide hydrochloride.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one4-methylbenzenesulfonate and 2-(R)-(4-Fluoro-2-methyl-phenyl)-4-(S)-((8aS)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide hydrochloride, at subtherapeutic dose.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate and 2-(R)-(4-Fluoro-2-methyl-phenyl)-4-(S)-((8aR)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide, or a pharmaceutically acceptable salt or solvate thereof.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate at subtherapeutic dose and 2-(R)-(4-Fluoro-2-methyl-phenyl)-4-(S)-((8aR)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide, or a pharmaceutically acceptable salt or solvate thereof.

In one embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate and 2-(R)-(4-Fluoro-2-methyl-phenyl)-4-(S)-((8aR)-6-oxo-hexahydro-pyrrolo[1,2-*a*]-pyrazin-2-yl)-piperidine-1-carboxylic acid [1-(R)-(3,5-bis-trifluoromethyl-phenyl)-ethyl]-methylamide, or a pharmaceutically acceptable salt or solvate thereof, at subtherapeutic dose.

In a further embodiment the pharmaceutical composition of the invention comprises (2R,5R)-2-(4-{[(2-fluorophenyl)methyl]oxy}phenyl)-7-methyl-1,7-diazaspiro[4.4]nonan-6-one 4-methylbenzenesulfonate and [2-Methoxy-5-(5-trifuoromethyl-tetrazol-1-yl)-benzyl]-(2S-phenyl-piperidin-3S-yl)-amine dihydrochloride.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate at subtherapeutic dose and [2-Methoxy-5-(5-trifuoromethyl-tetrazol-1-yl)-benzyl]-(2S-phenyl-piperidin-3S-yl)-amine dihydrochloride.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate and [2-Methoxy-5-(5-trifuoromethyl-tetrazol-1-yl)-benzyl]-(2S-phenyl-piperidin-3S-yl)-amine dihydrochloride, at subtherapeutic dose.

In a further embodiment the pharmaceutical composition of the invention comprises (*2R*,5*R*)-2-(4-{[(2-fluorophenyl)methyl]oxy}phenyl)-7-methyl-1,7-diazaspiro[4.4]nonan-6-one 4-methylbenzenesulfonate and 2-[3,5-Bis(trifluoromethyl)phenyl]-*N*-{4-(4-fluoro-2-methylphenyl)-6-[(7*S*,9a*S*)-7-(hydroxymethyl)hexahydropyrazino[2,1-*c*][1,4]oxazin-8(1*H*)-yl]-3-pyridinyl}-*N*,2-dimethylpropanamide, or a pharmaceutically acceptable salt or solvate thereof.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate at subtherapeutic dose and 2-[3,5-Bis(trifluoromethyl)phenyl]-*N*-{4-(4-fluoro-2-methylphenyl)-6-[(7S,9aS)-7-(hydroxymethyl)hexahydropyrazino[2,1-*c*][1,4]oxazin-8(1*H*)-yl]-3-pyridinyl}-*N*,2-dimethylpropanamide, or a pharmaceutically acceptable salt or solvate thereof.

In a further embodiment the pharmaceutical composition of the invention comprises (2*R*,5*R*)-2-(4-{[(2-fluorophenyl)methyl]oxy}phenyl)-7-methyl-1,7-diazaspiro[4.4]nonan-6-one 4-methylbenzenesulfonate and 2-[3,5-Bis(trifluoromethyl)phenyl]-*N*-{4-(4-fluoro-2-methy)phenyl)-6-[(7*S*,9a*S*)-7-(hydroxymethyl)hexahydropyrazino[2,1-*c*][1,4]oxazin-8(1*H*)-yl]-3-pyridinyl}-*N*,2-dimethylpropanamide, or a pharmaceutically acceptable salt or solvate thereof, at subtherapeutic dose.

In another embodiment, any one of the pharmaceutical compositions above described is provided for use in therapy.

In a further embodiment, any one of the pharmaceutical compositions above described is provided for use in the treatment of epilepsy, mood disorders or pain.

In an additional embodiment, the use of any one of the pharmaceutical compositions above described is provided for the treatment of epilepsy, mood disorders or pain.

The pharmaceutically acceptable salts of NK1 antagonist compounds or of a Sodium Channel blocker compound of formula (I) as above defined which contain a basic centre are, for example, non-toxic acid addition salts formed with inorganic acids such as hydrochloric, hydrobromic, hvdroiodic, sulfuric and phosphoric acid, with carboxylic acids or with organo-sulfonic acids. Examples include the HCl, HBr, Hl, sulfate or bisulfate, nitrate, phosphate or hydrogen phosphate, acetate, benzoate, succinate, saccharate, fumarate, maleate, isethionate, lactate, citrate, tartrate, gluconate, camsylate, methanesulfonate, ethanesulfonate, benzenesulfonate, p-toluenesulfonate and pamoate salts. For reviews on suitable pharmaceutical salts see Berge et al, J. Pharm, Sci., 66, 1-19, 1977; P L Gould, International Journal of Pharmaceutics, 33 (1986), 201-217; and Bighley et al, Encyclopedia of Pharmaceutical Technology, Marcel Dekker Inc, New York 1996, Volume 13, page 453-497.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Pharmaceutical compositions comprising pharmaceutically acceptable solvates of NK1 antagonist compounds or of a Sodium Channel blocker compound of formula (I) as above defined [for example (2R,5R)-2-(4-{[(2-fluorophenyl)methyl]oxy}phenyl)-7-methyl-1,7-diazaspiro[4.4]nonan-6-one], or pharmaceutically acceptable salts thereof, are within the scope of the invention.

The adjunctive therapy of the present invention is carried out by administering an NK1 receptor antagonist together with a compound of formula (I) as above defined [ for example (2*R*,5*R*)-2-(4-{[(2-fluorophenyl)methyl]oxy}phenyl)-7-methyl-1,7-diazaspiro[4.4]nonan-6-one], or a pharmaceuticallt acceptable salt or solvate thereof, in any manner which provides effective levels of the compounds in the body at the same time. It will be appreciated that the compounds of the composition may be administered simultaneously, either in the same or separate dosage forms, or sequentially. It will also be understood that the compounds of the composition, whether presented simultaneously or sequentially, may be administered individually, or in multiples, or in any combination thereof.

The amount of a pharmaceutical composition composition according to the invention required to be effective as a treatment for mood disorders, psychotic disorders, epilepsy or pain may, of course, vary and is ultimately at the discretion of the medical practitioner. The factors to be considered include the route of administration and nature of the formulation, the subject mammal's body weight, age and general condition and the nature and severity of the condition to be treated.

Unless otherwise indicated, all weights of active ingredients are calculated in terms of the drug *per se*. The desired dose may preferably be presented as one, two, three, four, five, six or more sub-doses administered at appropriate intervals throughout the day.

A pharmaceutical composition of the invention as hereinabove defined for use in the treatement of mood disorders, psychotic disorders, epilepsy or pain may conveniently be presented as a pharmaceutical composition in a unitary dosage form. Thus pharmaceutical compositions incorporating both compounds are important embodiments of the present invention. Such compositions may take any physical form which is pharmaceutically acceptable, for example orally usable pharmaceutical compositions. Such adjunctive pharmaceutical compositions contain an effective amount of each of the compounds, which effective amount is related to the daily dose of the compounds to be administered. Each adjunctive dosage unit may contain the daily doses of all compounds, or may contain a fraction of the daily doses, such as one- third of the doses. Alternatively, each dosage unit may contain the entire dose of one of the compounds, and a fraction of the dose of the other compounds. In such case, the patient would daily take one of the combination dosage units, and one or more units containing only the other compound. The amounts of each drug to be contained in each dosage unit may depend on the identity of the drugs chosen for the therapy.

Pharmaceutical compositions of the invention as hereinabove defined for use in the treatment of mood disorders, psychotic disorders, epilepsy or pain include those suitable for oral, rectal, nasal, topical (including transdermal, buccal and sublingual), vaginal or parenteral (including subcutaneous, intramuscular, intravenous and intradermal) administration. The compositions may conveniently be presented in unit dosage form and may be prepared by any methods well known in the art of pharmacy. Such methods represent a further feature of the present invention and include the step of bringing into association the active ingredients with the carrier which constitutes one or more accessory ingredients. In general, the compositions are prepared by uniformly and intimately bringing into association the active ingredients with liquid carriers or finely divided solid carriers or both, and then if necessary shaping the product.

Pharmaceutical compositions of the present invention suitable for oral administration may be presented as discrete units such as capsules, caplets, cachets or tablets each comprising a predetermined amount of the active ingredients; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredients in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, sodium croscarmellose cross-linked povidone, cross-linked sodium carboxymethyl cellulose) surface-active or dispersing agent. Moulded tablets may be made by moulding a mixture of the powdered compound moistened with an inert liquid diluent in a suitable machine. The tablets may optionally be coated or scored and may be formulated so as to provide slow or controlled release of the active ingredients therein using, for example, hydroxypropylmethyl cellulose in varying proportions to provide the desired release profile. Tablets may optionally be provided with an enteric coating, to provide release in parts of the gut other than the stomach.

Pharmaceutical compositions suitable for topical administration in the mouth include lozenges comprising the active ingredients in a flavored base, usually sucrose and acacia or tragacanth; pastilles comprising the active ingredients in an inert basis such as gelatin and glycerin, or sucrose and acacia; and mouthwashes comprising the active ingredients in a suitable liquid carrier. Compositions for rectal administration may be presented as a suppository with a suitable base comprising, for example, cocoa butter or polyethylene glycols.

Topical administration may also be by means of a transdermal iontophoretic device.

Pharmaceutical composition suitable for vaginal administration may be presented as tablets, pessaries, tampons, creams, gels, pastes, foams or spray compositions comprising in addition to the active ingredients such carriers as are known in the art to be appropriate.

Pharmaceutical compositions suitable for rectal administration wherein the carrier is a solid are most preferably presented as unit dose suppositories. Suitable carriers include cocoa butter and other materials commonly used in the art. The suppositories may be conveniently formed by admixture of the active combination with the softened or melted carrier(s) followed by chilling and shaping in molds.

Pharmaceutical compositions suitable for parenteral administration include aqueous and nonaqueous isotonic sterile injection solutions which may contain anti-oxidants, buffers, preservatives and solutes which render the formulation isotonic with the blood of the intended recipient; and aqueous and non-aqueous sterile suspensions which may include suspending agents and thickening agents; and liposomes or other microparticulate systems which are designed to target the compound to blood components or one or more organs. The formulations may be presented in unit-dose or multi-dose sealed containers, for example, ampoules and vials, and may be stored in a freeze-dried (lyophilized) condition requiring only the addition of the sterile liquid carrier, for example water for injection, immediately prior to use. Extemporaneous injection solutions and suspensions may be prepared from sterile powders, granules and tablets of the kind previously described.

It should be understood that in addition to the ingredients particularly mentioned above the compositions of this invention may include other agents conventional in the art having regard to the type of formulation in question, for example, those suitable for oral administration may include such further agents as sweeteners, thickeners and flavoring agents.

The pharmaceutical compositions of the invention comprising the two active ingredients may be prepared according to conventional techniques well known in the pharmaceutical industry. Thus, for example (2*R*,5*R*)-2-(4-{[(2-fluorophenyl)methyl]oxy}phenyl)-7-methyl-1,7-diazaspiro[4.4]nonan-6-one 4-methylbenzenesulfonate and [2-Methoxy-5-(5-trifuoromethyl-tetrazol-1-yl)-benzyl]-(2S-phenyl-piperidin-3S-yl)-amine or pharmaceutically acceptable salts or solvates thereof, may be admixed together with suitable excipients such as those described above for the formulation of each of the active ingredients separately. Tablets may be prepared, for example by direct compression of such a mixture or using other conventional methods. Bilayer tablets may be prepared according to conventional procedure. Thus, for example, by separately compressing the two blends in a suitable tabletting machine with two filling stations. Capsules may be prepared by filling the blend along with suitable excipients into gelatin capsules, using a suitable filling machine. Controlled release forms for oral or rectal administration may be formulated in a conventional manner associated with controlled release forms.

Pharmaceutical compositions are often prescribed to the patient in "patient packs" containing the whole course of treatment in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in traditional prescriptions. The inclusion of a package insert has been shown to improve patient compliance with the physician's instructions and, therefore, lead generally to more successful treatment.

It will be understood that the administration of the pharmaceutical compositions of the invention by means of a single patient pack, or patient packs of each formulation,containing within a package insert instructing the patient to the correct use of the invention is a desirable additional feature of this invention.

According to a further aspect of the invention there is provided a multiple, for example, double or triple, pack comprising an NK1 receptor antagonist and a Sodium Channel blocker compound of formula (I) as above defined [for example (2*R*,5*R*)-2-(4-{[(2-fluorophenyl)methyl]oxy}phenyl)-7-methyl-1,7-diazaspiro[4.4]nonan-6-one], or a pharamceutically acceptable salt or solvate thereof, and an information insert containing directions on the use of the compositions of the invention.

### Experimental data

The following table lists the used abbreviations:

| | | |
|---|---|---|
| DMSO | | dimethylsulfoxide |
| DCM | - | dichloromethane |
| DMAP | - | dimethylaminopyridine |
| DMF | - | dimethylformamide |
| TFA | | Trifluoroacetic acid |
| LiHMDS | - | lithium bis(trimethylsilyl)amide |
| MeOH | | Methanol |

| | | |
|---|---|---|
| TEA | - | triethylamine |
| THF | - | tetrahydrofuran |
| MTBE | - | Methyl-t-butyl-ether |
| EtOAc, EA, AcOEt | - | Ethyl Acetate |
| CH | | cyclohexane |
| Et₂O | - | Diethyl ether |
| EtOH | | ethanol |
| DIPEA | - | Diisopropylethyl amine |
| BOC2O | | di-t-butyl-dicarbonate |

### Intermediate 1

### 4-Fluoro-2-methyl-phenyl)-oxo-acetic acid methyl ester

1) To a suspension of magnesium turnings (617 mg) in anh. THF (6 mL), at r.t., under N2, a small crystal of I2 was added, followed by 10% of a solution of commercial 2-bromo-5-fluorotoluene (4.0g) in anh. THF (15 mL). The suspension was heated gently (heat gun) until the brown colour disappeared. The remaining bromide solution was added drop-wise, maintaining the reaction mixture warm (50-60°C) with an oil bath. After the addition was complete (15 min), the suspension was stirred at 70°C until the magnesium turnings had almost completely reacted (2 hr). The new brown solution was used in the next step.
2) A solution of LiBr (4.41 g) in anh. THF (50 mL) was added drop-wise to a suspension of CuBr (3.64g) in anh. THF (50 mL). The reaction mixture was stirred at r.t. for 1 hr (dark green solution with a small amount of white solid in suspension). The Grignard solution previously prepared was then added dropwise (an ice bath was used to maintain the temperature <25°C) followed by methyl oxalyl chloride (1.95 mL). The reaction mixture was stirred at r.t. for 2 hr. The THF was evaporated and the residue was taken up in AcOEt. The organic layer was washed with sat.aq. NH4CI (2x) and dried. The solids were filtered and the solvent evaporated to give a crude oil, which was purified by flash chromatography (CH/AcOEt 95:5) to obtain the title compound as a clear oil (2.44 g).
   NMR (CDCl3): δ (ppm) 7.74 (m, 1H), 6.98-7.04 (m, 2H), 3.96 (s, 3H), 2.61 (s, 3H).

### Intermediate 2

### 3-(4-Fluoro-2-methyl-phenyl)-5,6-dihydro-1H-pyrazin-2-one

To a solution of intermediate 1 (2.01g) and ethylenediamine (684 µL) in toluene (40 mL), at r.t., under N2, anh. Na2SO4 (2 g) was added. The reaction mixture was heated at reflux for 6 hr. It was then cooled down to r.t. and filtered. The solids were rinsed with DCM. The solvent was evaporated and the crude oil was purified by flash chromatography (AcOEt) affording the title compound as a white solid (1.29g).
NMR (CDCl3): δ (ppm) 7.33 (m, 1 H), 6.95-6.90 (m, 2H), 6.56 (m, 1 H), 3.97 (m, 2H), 3.58 (m, 2H), 2.31 (s, 3H).

### Intermediate 2a

### 3-(4-Fluoro-2-methyl-phenyl)-piperazin-2-one

To a solution, at 25°C, of intermediate 2 (168g) in Methanol (2400mL) under nitrogen, Pd/C 10% (44g) was added. The reaction mixture was placed under a H₂ atmosphere and stirred at 25°C for about 16 hours (till no further hydrogen was consumed and the reaction was completed by TLC, EA/MeOH 9/1). The catalyst was filtered in nitrogen atmosphere and the solvent was removed to low volume (360mL) then Methanol (2040mL) and Ethyl Acetate (9600mL) were added and a silica pad (800g) was performed; the eluted solution was concentrated to obtain the title compound (168g).
¹H-NMR (DMSO) δ (ppm) 7.77 (bm, 1H); 7.24 (dd, 1 H); 6.96 (dd, 1 H); 6.92 (td, 1H); 4.43 (s, 1H); 3.30 (m, 1H); 3.14 (m, 1H); 2.92 (m, 1H); 2.82 (m, 2H); 2.33 (s, 3H).

### Intermediate 3

### (+)(S)-3-(4-Fluoro-2-methyl-phenyl)-piperazin- 2-one

### Method A

To a suspension of intermediate 2 (35 g) in AcOEt (900 mL), L(+)-Mandelic Acid (27.3 g) was added. The suspension was stirred at r.t. for 1 hr then at 3-5°C for 2 hr, filtered and dried under vacuum at. r.t to obtain crude L(+)-mandelate 3-(4-fluoro-2-methyl-phenyl)-piperazin-2-one (37 g), which was suspended in AcOEt (370 mL) and heated to reflux till complete solubilisation then cooled to room temperature and stirred for further 2 hours, filtered, washed with of AcOEt (150 mL) and dried under vacuum obtaining (+) L-mandelate 3-(4-Fluoro-2-methyl-phenyl)-5,6 pyrazin-2-one (30.4 g) as white solid. This material (30.4g) was suspended in AcOEt (300 mL) and treated with NaOH (0.73M, 155 mL) saturated with NaCl. The organic phase was then washed with water (90 mL). The aqueous phase was counter-extracted 4 times with AcOEt (90 mL). The combined organic phase (1800 mL) was dried on 10 g of Na2SO4 and concentrated under vacuum obtaining the title compound (25.04 g) as white foam.

### Method B

To a solution, heated at 45°C, of intermediate 2a (168 g) in Ethyl Acetate (2000mL) L(+)-Mandelic Acid (116g) and 3,5-dichloro-salicilaldehyde (10.8g) were added. The solution was stirred for 30min at 45°C then seeded with white crystals of L(+)mandelate-3-(4-Fluoro-2-methyl-phenyl)-piperazin - 2-one (0.4g). The obtained suspension was stirred under nitrogen atmosphere at 45°C for 16 hours then stirred for further 4 hour at 0°C, washed with cooled Ethyl Acetate (2x200ml) then dried under vacuum at room temperature for 2 hours to obtain L(+)mandelate-3-(4-Fluoro-2-methyl-phenyl)-piperazin - 2-one(126.22g) as a white/yellowish solid which was suspended in DCM (2760mL) then NaOH 0.8M in Brine (17.35g of NaOH in 530mL of Brine) was added. The organic phase was then washed with Brine (380mL) and the aqueous phase was counter extracted four times with DCM (4x1500mL). The combined organic phase was dried and concentrated to obtain the title compound (60.35g).
¹H-NMR (DMSO) δ (ppm) 7.77 (bm, 1 H); 7.24 (dd, 1 H); 6.96 (dd, 1 H); 6.92 (td, 1 H); 4.43 (s, 1 H); 3.30 (m, 1 H); 3.14 (m, 1 H); 2.92 (m, 1 H); 2.82 (m, 2H); 2.33 (s, 3H). HPLC:Chiralcel OJ (4.6X250mm) from Daicel; Mobile Phase:n-Hexane/Ethanol 80:20 v/v;Flow:1 mL/min; Detector:UV @ 265nm (or 210 nm for higher signals); Dissolution phase:n-Hexane/Ethanol 80/20 v/v;
Sample Concentration 1 mg/ml; Injection:5 uL;Retention times: 2:8.4 min.
[α]_{D}(solvent CHCl3, Source: Na; Cell volume [ml]: 1; Cell pathlength [dm]:1; Cell temperature [°C]:20; Wavelength [nm]: 589; Conc. sample [% p/v] :1.17)=+17.9.

### Intermediate 4

### (S)-3-(4-Fluoro-2-methyl-phenyl)-piperazine dihydrochloride

To a solution of intermediate 3 (60.35g) in dry THF (180ml), at 0-3°C, under N₂, BH₃·THF 1 M/THF (1220mL) was added dropwise. The solution was refluxed for 4 hours then cooled to 0-3°C and methanol (240mL) was added. The reaction mixture was heated to room temperature then it was concentrated to dryness. The residue was redissolved in methanol (603.5mL), excess HCl 1 N in Et₂O (1207mL) was added and the mixture was refluxed for 2 hours then cooled at 3°C for 4 hours. The suspension was filtered to obtain a white solid that was washed with Et₂O (60.35mL) and dried to yield the title compound (72.02g)
¹H-NMR (DMSO) δ (ppm) 11.0-9.5 (b, 4H); 7.99-7.19 (dd-m, 3H); 4.96 (dd, 1H); 3.65-3.15 (m, 6H); 2.42 (s, 3H).

### Intermediate 5

### (R)-3,5-bis-trifluoromethyl-phenyl)-ethyl]-methyl-amine

To a solution of 3,5-bis-trifluoromethylacetophenone (300g) in MeOH (1120mL), a solution of methylamine 8M in EtOH (372mL) was added dropwise during 15 min. at 25°C under N₂. The mixute was stirred for 24hrs at 25°C under N₂. Then, NaBH₄ was portionwise added over 30 min (27.9g) at 0°C. A second amount of NaBH₄ was added over 30 min (17.1 g) and the mixture stirred for further 1.5hrs.
The mixture was concentrated by evaporating 600mL of solvent under vacuum then it was slowly poured into a mixture of AcOEt (1500mL)/NH₄Cl sat (750mL) and water (750mL). The water phase was back-extracted with AcOEt (1500mL). The combined organic phases were washed with Water/Brine (150mL/150mL) then evaporated to obtain 3,5-bis-trifluoromethyl-phenyl)-ethyl]-methyl-amine (305g) as a yellow oil
To a solution of 3,5-bis-trifluoromethyl-phenyl)-ethyl]-methyl-amine (245.6g) in EtOAc (2380mL), L(+) malic acid was added portionwise (118g). The suspension was stirred for 2hrs at 25°C then 3hrs at 0°C. The suspension was filtered and the cake was washed with EtOAc (240mL). The solid was dried under vacuum obtaining crude L(+)malate3,5-bis-trifluoromethyl-phenyl)-ethyl]-methyl-amine (135.3g) as a white solid which was suspended in Ethyl acetate (1760mL) then heated to reflux till complete dissolution and then cooled at 25°C. The suspension was filtered, washed with Ethyl acetate (135mL) then dried to obtain L(+)malate3,5-bis-trifluoromethyl-phenyl)ethyl]-methyl-amine (128.5g). The solid was stirred in a mixture of NaOH 10%v/v (720mL) and Ethyl acetate (650mL). Organic phase was washed with water (720mL), then concentrated to yield the title compound (82.2g).
¹H-NMR (DMSO) δ (ppm) 7.99 (s, 2H); 7.85 (s, 1 H); 3.78 (q, 1 H); 2.34 (s, 1 H); 2.09 (s, 3H); 1.23 (d, 3H).

### Representative NK1 antagonist Example 1 (ENK1)

### 2-(S)-(4-Fluoro-2-methyl-phenyl)-piperazine-1-carboxylic acid [1-(R)-(3,5-bis-trifluoromethyl-phenyl)-ethyl]-methyl-amide methansulphonate

To a suspension of intermediate 4 (4.9Kg) in AcOEt (137.2L), triethylamine (5.63L) was added. The mixture was cooled to 0°C then a solution of diterbuthyl dicarbonate (3.134Kg) in AcOEt (24.5L) was added in 35 min, maintaining the temperature between 0 and 5°C. The suspension was stirred at 0°C for 15 min, at 20/25°C for 1 hr, then washed with water (3 x 39.2L), concentrated to 24.5L and then added to a solution of triphosgene (1.97Kg) in AcOEt (24.5L) cooled to 0°C. Triethylamine (3.28L) was then added in 40 min, maintaining the temperature between 0 and 8°C. The suspension was stirred for 1 hand 45 min at 20/25°C and 30 min at 70Cand then the solution of intermediate 5 diluted with AcOEt (49L) and triethylamine (2.6L) was added in 30 min. The mixture was refluxed for 15 hrs.
The reaction mixture, cooled at 20/25°C was treated with aqueous solution of NaOH 10%v/v (36.75L). Organic phase was washed with HCl 4%v/v (46.55L) and NaCl 11,5%p/p (4 x 24.5L) then concentrated to 14.7L. and diluted with Ciclohexane (39.2L). The mixture was filtered through a silica pad (4.9Kg) that was washed twice with a mixture of CH/AcOEt 85/15 (2 x 49L). To the Eluted phases (14.7L) cooled at 20/25°C., methyl tertbutyl ether (49L) and methansulphonic acid (4.067L) were added. The mixture was washed with NaOH 10%v/v (31.85L) then with water (4 x 31.85L). Organic phase was concentrated to 9.8L, methyl tertbutyl ether (49L) was added and the solution filtered through a 5micron filter then concentrated to 9.8L. At 20/25°C MTBE (29.4L) and metansulphonic acid (1.098L) were added. The suspension was refluxed for 10 min, stirred at 20/25°C for 10hrs and 2 hrs at O°C.Then the precipitate was filtered, washed with methyl tertbutyl ether (4.9L) dried under vacuum at 20/25°C for 24 hrs to obtain the title compound (5.519Kg.) as white solid.
¹H-NMR (DMSO) δ (ppm) 8.99 (bm, 1 H); 8.66 (bm, 1 H); 8.00 (bs, 1 H); 7.69 (bs, 2H); 7.27 (dd, 1H); 7.00 (dd, 1H); 6.83 (m, 1H); 5.32 (q, 1H); 4.47 (dd, 1H); 3.50-3.20 (m, 4H); 2.96 (m, 2H); 2.72 (s, 3H); 2.37 (s, 3H); 2.28 (s, 3H); 1.46 (d, 3H).
ES⁺: m/z 492 [MH - CH₃SO₃H]⁺
ES⁻: m/z 586 [M - H]⁻; 95 [CH₃SO₃]⁻

### Intermediate 6

### 1-(Benzyloxycarbonyl)-2-(4-fluoro-2-methyl-phenyl)-2,3-dihydro-4-pyridone

A small amount of iodine was added to a suspension of magnesium turnings (13.2 g) in dry THF (300 mL), at r.t., under a nitrogen atmosphere, then the mixture was vigorously refluxed for 20 minutes. To this suspension, a 15% of a solution of 2-bromo-5-fluoro-toluene (52.5 mL) in anhydrous THF (300 mL) was added. The suspension was heated under vigorous reflux until the brown colour disappeared. The remaining part of the bromide solution was added drop-wise over 1 hour to the refluxing suspension which was then stirred for a further 1 hour. This solution of Grignard reagent was then added drop-wise to the pyridinium salt obtained from benzyl chloroformate (48.7 mL) and 4-methoxypyridine (25 mL) in dry THF (900 mL) at -23°C.
The obtained solution was stirred 1 hour at -20°C then it was warmed up to 20°C, a 10% hydrochloric acid solution (560 mL) was added and the aqueous layer was extracted with AcOEt (2 x 750 mL).

The combined organic extracts were washed with 5% sodium hydrogen carbonate solution (600 mL) and brine (600 mL) then partially concentrated *in vacuo.*
CH (400 mL) was added drop-wise over 1 hour at 20°C and the resulting mixture was stirred 30 minutes and then filtered to give the title compound as a white solid (66 g).
IR (nujol, cm⁻¹): 1726 and 1655 (C=O), 1608 (C=C).
NMR (d₆-DMSO): δ (ppm) 8.19 (d, 1H); 7.31-7.18 (m, 5H); 7.08 (m, 2H); 6.94 (dt, 1H); 5.77 (d, 1H); 5.36 (d, 1H); 5.16 (2d, 2H); 3.26 (dd, 1H); 2.32 (d, 1H); 2.26 (s, 3H).
MS (ES/+): m/z=340 [MH]⁺.

### Intermediate 7

### 2-(4-Fluoro-2-methyl-phenyl)-piperidine-4-one

### Method A

2-Methyl-4-fluoro-benzaldehyde (4 g) was added to a solution of 4-aminobutan-2-one ethylene acetal (3.8 g) in dry benzene (50 mL) and the solution was stirred at r.t. under a nitrogen atmosphere. After 1 hour the mixture was heated at reflux for 16 hours and then allowed to cool to r.t. This solution was slowly added to a refluxing solution of p-toluensulphonic acid (10.6 g) in dry benzene (50 mL) previously refluxed for 1 hour with a Dean-Stark apparatus. After 3.5 hours the crude solution was cooled and made basic with a saturated potassium carbonate solution and taken up with AcOEt (50 mL). The aqueous phase was extracted with AcOEt (3 x 50 mL) and Et2O (2 x 50 mL). The organic layer was dried and concentrated *in vacuo* to a yellow thick oil as residue (7.23 g). A portion of the crude mixture (3 g) was dissolved in a 6N hydrochloric acid solution (20 mL) and stirred at 60°C for 16 hours. The solution was basified with solid potassium carbonate and extracted with DCM (5 x 50 mL). The combined organic phases were washed with brine (50 mL), dried and concentrated *in vacuo* to give the title compound (2.5 g) as a thick yellow oil.

### Method B

L-selectride (1M solution in dry THF, 210 mL) was added drop-wise, over 80 minutes, to a solution of intermediate **6** (50 g) in dry THF (1065 mL) previously cooled to -72°C under a nitrogen atmosphere. After 45 minutes, 2% sodium hydrogen carbonate solution (994 mL) was added drop-wise and the solution was extracted with AcOEt (3 x 994 mL). The combined organic phases were washed with water (284 mL) and brine (568 mL). The organic phase was dried and concentrated *in vacuo* to get 1-benzyloxycarbonyl-2-(4-fluoro-2-methyl-phenyl)-piperidine-4-one as a pale yellow thick oil (94 g) which was used as a crude.
This material (94 g) was dissolved in AcOEt (710 mL), then 10% Pd/C (30.5 g) was added under a nitrogen atmosphere. The slurry was hydrogenated at 1 atmosphere for 30 minutes. The mixture was filtered through Celite and the organic phase was concentrated *in vacuo* to give the crude 2-(4-fluoro-2-methyl-phenyl)-piperidine-4-one as a yellow oil. This material was dissolved in AcOEt (518 mL) at r.t. and racemic camphorsulphonic acid (48.3 g) was added. The mixture was stirred at r.t for 18 hours, then the solid was filtered off, washed with AcOEt (2 x 50 mL) and dried *in vacuo* for 18 hours to give 2-(4-fluoro-2-methyl-phenyl)-piperidine-4-one, 10-camphorsulfonic acid salt as a pale yellow solid (68.5 g). (M.p.: 167-169°C - NMR (d₆-DMSO): δ (ppm) 9.43 (bs, 1 H); 9.23 (bs, 1 H); 7.66 (dd, 1 H); 7.19 (m, 2H); 4.97 (bd, 1 H); 3.6 (m, 2H); 2.87 (m, 3H); 2.66 (m, 1 H); 2.53 (m, 2H); 2.37 (s + d, 4H); 2.22 (m, 1 H); 1.93 (t, 1 H); 1.8 (m, 2H); 1.26 (m, 2H); 1.03 (s, 3H); 0.73 (s, 3H).
This material (68.5 g) was suspended in AcOEt (480 mL) and stirred with a saturated sodium hydrogen carbonate (274 mL). The organic layer was separated and washed with further water (274 mL). The organic phase was dried and concentrated *in vacuo* to give the title compound (31 g) as a yellow-orange oil.
NMR (d₆-DMSO): δ (ppm) 7.49 (dd, 1 H); 7.00 (m, 2H); 3.97 (dd, 1H); 3.27 (m, 1H); 2.82 (dt, 1 H); 2.72 (bm, 1 H); 2.47 (m, 1 H); 2.40 (m, 1 H); 2.29 (s, 3H); 2.25 (dt, 1 H); 2.18 (m, 1H).
MS (ES/+): m/z=208 [MH]⁺.

### Intermediate 8

### 2-(R)-(4-Fluoro-2-methyl-phenyl)-piperidin-4-one mandelic acid.

A solution of L-(+)-mandelic acid (22.6 g) in AcOEt (308 mL) was added to a solution of intermediate **7** (31 g) in AcOEt (308 mL). Then isopropanol (616 mL) was added and the solution was concentrated *in vacuo* to 274 mL. The solution was then cooled to 0°C and further cold isopropanol (96 mL) was added. The thick precipitate was stirred under nitrogen for 5 hours at 0°C, then filtered and washed with cold Et2O (250 mL) to give the title compound as a pale yellow solid (20.3 g).
M.p.: 82-85°C.
NMR (d₆-DMSO): δ (ppm) 7.51 (dd, 1H); 7.40 (m, 2H); 7.32 (m, 2H); 7.26 (m, 1H); 7.0 (m, 2H); 4.95 (s, 1 H); 4.04 (dd, 1 H); 3.31 (m, 1 H); 2.88 (m, 1 H); 2.49-2.2 (m, 4H); 2.29 (s, 3H).
Chiral HPLC: HP 1100 HPLC system; column Chiralcel OD-H, 25 cm x 4.6 mm; mobile phase: n-hexane/isopropanol 95:5 + 1% diethylamine; flow: 1.3 ml/min; detection: 240/215nm; retention time 12.07 minutes.

### Intermediate 9

### 2-(R)-(4-Fluoro-2-methyl-phenyl)-4-oxo-piperidine-1-carboxylic acid, [1-(R)-3,5-bis-trifluoromethyl-phenyl)-ethyl]-methylamide (9a) and 2-(S)-(4-Fluoro-2-methyl-phenyl)-4-oxo-piperidine-1-carboxylic acid, [1-(R)-3,5-bis-trifluoromethyl-phenyl)-ethyl]-methylamide (9b)

### Method A

A solution of triphosgene (147 mg) dissolved in dry DCM (5 mL) was added drop-wise to a solution of intermediate **7** (250 mg) and DIPEA (860 µL) in dry DCM (15 mL) previously cooled to 0°C under a nitrogen atmosphere. After 2 hours, [1-(R)-3,5-bis-trifluoromethylphenyl)-ethyl]-methylamine hydrochloride (503 mg) and DIPEA (320 µL) in dry acetonitrile (20 mL) were added and the mixture was heated to 70°C for 16 hours. Further [1-(R)-(3,5-bis-trifluoromethyl-phenyl)-ethyl]-methylamine hydrochloride (170 mg) and DIPEA (100 µL) were added and the mixture was stirred at 70°C for further 4 hours. Next, the mixture was allowed to cool to r.t., taken up with AcOEt (30 mL), washed with a 1N hydrochloric acid cold solution (3 x 15 mL) and brine (2 x 10 mL). The organic layer was dried and concentrated *in vacuo* to a residue, which was purified by flash chromatography (CH/AcOEt 8:2) to give:
1. intermediate **9a** (230 mg) as a white foam,
2. intermediate **9b** (231 mg) as a white foam.

### Intermediate 9a

NMR (d₆-DMSO): δ (ppm) 7.98 (bs, 1 H); 7.77 (bs, 2H); 7.24 (dd, 1H); 6.97 (dd, 1H); 6.89 (m, 1 H); 5.24 (t, 1 H); 5.14 (q, 1 H); 3.61 (m, 1 H); 3.55 (m, 1H); 2.71 (m, 2H); 2.56 (s, 3H); 2.50 (m, 2H); 2.26 (s, 3H); 1.57 (d, 3H).

### Intermediate 9b

NMR (d₆-DMSO): δ (ppm) 7.96 (bs, 1 H); 7.75 (bs, 2H); 7.24 (dd, 1H); 6.98 (dd, 1 H); 6.93 (dt, 1H); 5.29 (q, 1H); 5.24 (t, 1 H); 3.56 (m, 1H); 3.48 (m, 1H); 2.70 (s, 3H); 2.50 (m, 4H); 2.26 (s, 3H); 1.54 (d, 3H).

### Intermediate 9a

### Method B

A saturated sodium hydrogen carbonate solution (324 mL) was added to a solution of intermediate **8** (21.6 g) in AcOEt (324 mL) and the resulting mixture was vigorously stirred for 15 minutes. The aqueous layer was back-extracted with further AcOEt (216 mL) and the combined organic extracts were dried and concentrated *in vacuo* to give 2-(R)-(4-fluoro-2-methyl-phenyl)-piperidin-4-one as a yellow oil, which was treated with TEA (19 mL) and AcOEt (114 mL). The solution obtained was added drop-wise over 40 minutes to a solution of triphosgene (8 g) in AcOEt (64 mL) previously cooled to 0°C under a nitrogen atmosphere, maintaining the temperature between 0°C and 8°C.
After stirring for 1 hours at 0°C and for 3 hours at 20°C, [1-(R)-(3,5-bis-trifluoromethylphenyl)-ethyl]-methylamine hydrochloride (29.7 g), AcOEt (190 mL) and TEA (38 mL) were added to the reaction mixture which was then heated to reflux for 16 hours.
The solution was washed with 10% sodium hydroxide solution (180 mL), 1% hydrochloric acid solution (4 x 150 mL), water (3 x 180 mL) and brine (180 mL). The organic layer was dried and concentrated *in vacuo* to a residue, which was purified through a silica pad (CH/AcOEt 9:1) to give the title compound (21.5 g) as a brown thick oil.
NMR (d₆-DMSO): δ (ppm) 7.97-7.77 (bs + bs, 3H); 7.24 (dd, 1H); 6.97 (dd, 1H); 6.88 (td, 1 H); 5.24 (m, 1H); 5.14 (q, 1 H); 3.58 (m, 2H); 2.7 (m, 2H); 2.56 (s, 3H); 2.49 (m, 2H); 2.26 (s, 3H); 1.57 (d, 3H).

### Representative NK1 antagonist Example 2 (ENK2)

### 4-(S)-(4-Acetyl-piperazin-1-yl)-2-(R)-(4-fluoro-2-methyl-phenyl)-piperidine-1-carboxylic acid, [1-(R)-(3,5-bis-trifluoromethyl-phenyl)-ethyl]-methylamide methanesulphonate

A solution of intermediate **9a** (7.7 g) in acetonitrile (177 mL) was added to a solution of 1-acetyl-piperazine (3.9 g) in acetonitrile (17.7 mL) followed by sodium triacetoxyborohydride (6.4 g) under a nitrogen atmosphere.
The reaction mixture was stirred at room temperature for 24 hours and then quenched with a saturated sodium hydrogen carbonate (23.1 mL) and water (61.6 mL). The resulting solution was concentrated *in vacuo,* then AcOEt (208 mL) was added; the layers were separated and the aqueous layer was back-extracted with further AcOEt (2 x 77 mL). The collected organic phases were washed with brine (2 x 118 mL), dried and concentrated *in vacuo* to give the crude mixture of syn and anti diastereomers (nearly 1:1) as a white foam (9.5 g).
A solution of this intermediate in THF (85.4 mL) was added to a solution of methansulfonic acid (0.890 mL) in THF (6.1 mL) at r.t.. After seeding, the desired syn diastereomer started to precipitate. The resulting suspension was stirred for 3 hours at 0°C and then filtered under a nitrogen atmosphere. The resulting cake was washed with cold THF (15.4 mL) and dried *in vacuo* at + 20°C for 48 hours to give the title compound as a white solid (4.44 g).

NMR (d₆-DMSO): δ (ppm) 9.52 (bs, 1 H); 7.99 (bs, 1 H); 7.68 (bs, 2H); 7.23 (m, 1 H); 6.95 (dd, 1 H); 6.82 (m, 1H); 5.31 (q, 1H); 4.45 (bd, 1 H); 4.20 (dd, 1 H); 3.99 (bd, 1 H); 3.65-3.25 (bm, 5H); 3.17 (m, 1 H); 2.96 (m, 1 H); 2.88-2.79 (m+m, 2H); 2.73 (s, 3H); 2.36 (s, 3H); 2.30 (s, 3H); 2.13-2.09 (bd+bd, 2H); 2.01 (s, 3H); 1.89-1.73 (m+m, 2H); 1.46 (d, 3H). m.p 243.0 °C.
The compound is isolated in a crystalline form.

### Intermediate 10

### 1-(Benzyloxycarbonyl)-2-(4-fluoro-2-methyl-phenyl)-2,3-dihydro-4-pyridone

A small amount of iodine was added to a suspension of magnesium turnings (13.2 g) in dry THF (300 mL), at r.t., under a nitrogen atmosphere, then the mixture was vigorously refluxed for 20 minutes. To this suspension, a 15% of a solution of 2-bromo-5-fluoro-toluene (52.5 mL) in anhydrous THF (300 mL) was added. The suspension was heated under vigorous reflux until the brown colour disappeared. The remaining part of the bromide solution was added drop-wise over 1 hour to the refluxing suspension which was then stirred for a further 1 hour. This solution of Grignard reagent was then added drop-wise to the pyridinium salt obtained from benzyl chloroformate (48.7 mL) and 4-methoxypyridine (25 mL) in dry THF (900 mL) at -23°C.
The obtained solution was stirred 1 hour at -20°C then it was warmed up to 20°C, a 10% hydrochloric acid solution (560 mL) was added and the aqueous layer was extracted with AcOEt (2 x 750 mL).
The combined organic extracts were washed with 5% sodium hydrogen carbonate solution (600 mL) and brine (600 mL) then partially concentrated *in vacuo.*
CH (400 mL) was added drop-wise over 1 hour at 20°C and the resulting mixture was stirred 30 minutes and then filtered to give the title compound as a white solid (66 g).
IR (nujol, cm⁻¹): 1726 and 1655 (C=O), 1608 (C=C).
NMR (d₆-DMSO): δ (ppm) 8.19 (d, 1 H); 7.31-7.18 (m, 5H); 7.08 (m, 2H); 6.94 (dt, 1 H); 5.77 (d, 1 H); 5.36 (d, 1H); 5.16 (2d, 2H); 3.26 (dd, 1H); 2.32 (d, 1H); 2.26 (s, 3H).
MS (ES/+): m/z=340 [MH]⁺.

### Intermediate 11

### 2-(4-Fluoro-2-methyl-phenyl)-piperidine-4-one

### Method A:

4-Fluoro-2-methyl-benzaldehyde (4 g) was added to a solution of 4-aminobutan-2-one ethylene acetal (3.8 g) in dry benzene (50 mL) and the solution was stirred at r.t. under a nitrogen atmosphere. After 1 hour the mixture was heated at reflux for 16 hours and then allowed to cool to r.t. This solution was slowly added to a refluxing solution of p-toluenesulphonic acid (10.6 g) in dry benzene (50 mL) previously refluxed for 1 hour with a Dean-Stark apparatus. After 3.5 hours the crude solution was cooled and made basic with a saturated potassium carbonate solution and taken up with AcOEt (50 mL). The aqueous phase was extracted with AcOEt (3 x 50 mL) and Et2O (2 x 50 mL). The organic layer was dried and concentrated *in vacuo* to a yellow thick oil as residue (7.23 g). A portion of the crude mixture (3 g) was dissolved in a 6N hydrochloric acid solution (20 mL) and stirred at 60°C for 16 hours. The solution was basified with solid potassium carbonate and extracted with DCM (5 x 50 mL). The combined organic phases were washed with brine (50 mL), dried and concentrated *in vacuo* to give the title compound (2.5 g) as a thick yellow oil.

### Method B

L-selectride (1 M solution in dry THF, 210 mL) was added drop-wise, over 80 minutes, to a solution of intermediate **10** (50 g) in dry THF (1065 mL) previously cooled to -72°C under a nitrogen atmosphere. After 45 minutes, 2% sodium hydrogen carbonate solution (994 mL) was added drop-wise and the solution was extracted with AcOEt (3 x 994 mL). The combined organic phases were washed with water (284 mL) and brine (568 mL). The organic phase was dried and concentrated *in vacuo* to get 1-benzyloxycarbonyl-2-(4-fluoro-2-methyl-phenyl)-piperidine-4-one as a pale yellow thick oil (94 g) which was used as a crude.
This material (94 g) was dissolved in AcOEt (710 mL), then 10% Pd/C (30.5 g) was added under a nitrogen atmosphere. The slurry was hydrogenated at 1 atmosphere for 30 minutes. The mixture was filtered through Celite and the organic phase was concentrated *in vacuo* to give the crude 2-(4-fluoro-2-methyl-phenyl)-piperidine-4-one as a yellow oil. This material was dissolved in AcOEt (518 mL) at r.t. and racemic camphorsulphonic acid (48.3 g) was added. The mixture was stirred at r.t for 18 hours, then the solid was filtered off, washed with AcOEt (2 x 50 mL) and dried *in vacuo* for 18 hours to give 2-(4-fluoro-2-methyl-phenyl)-piperidine-4-one, 10-camphorsulfonic acid salt as a pale yellow solid (68.5 g). (M.p.: 167-169°C - NMR (d₆-DMSO): δ (ppm) 9.43 (bs, 1 H); 9.23 (bs, 1 H); 7.66 (dd, 1 H); 7.19 (m, 2H); 4.97 (bd, 1 H); 3.6 (m, 2H); 2.87 (m, 3H); 2.66 (m, 1H); 2.53 (m, 2H); 2.37 (s + d, 4H); 2.22 (m, 1 H); 1.93 (t, 1 H); 1.8 (m, 2H); 1.26 (m, 2H); 1.03 (s, 3H); 0.73 (s, 3H).
This material (68.5 g) was suspended in AcOEt (480 mL) and stirred with a saturated sodium hydrogen carbonate (274 mL). The organic layer was separated and washed with further water (274 mL). The organic phase was dried and concentrated *in vacuo* to give the title compound (31 g) as a yellow-orange oil.
NMR (d₆-DMSO): δ (ppm) 7.49 (dd, 1 H); 7.00 (m, 2H); 3.97 (dd, 1H); 3.27 (m, 1 H); 2.82 (dt, 1 H); 2.72 (bm, 1 H); 2.47 (m, 1 H); 2.40 (m, 1 H); 2.29 (s, 3H); 2.25 (dt, 1 H); 2.18 (m, 1H).
MS (ES/+): m/z=208 [MH]⁺.

### Intermediate 12

### 2-(R)-(4-Fluoro-2-methyl-phenyl)-4-oxo-piperidine-1-carboxylic acid [1-(R)-3,5-bis-trifluoromethyl-phenyl)-ethyl]-methylamide (12a) and 2-(S)-(4-Fluoro-2-methyl-phenyl)-4-oxo-piperidine-1-carboxylic acid [1-(R)-3,5-bis-trifluoromethyl-phenyl)-ethyl]-methylamide (12b)

### Method A:

A solution of triphosgene (147 mg) dissolved in dry DCM (5 mL) was added drop-wise to a solution of intermediate **11** (250 mg) and DIPEA (860 µL) in dry DCM (15 mL) previously cooled to 0°C under a nitrogen atmosphere. After 2 hours, [1-(R)-3,5-bis-trifluoromethylphenyl)-ethyl]-methylamine hydrochloride (503 mg) and DIPEA (320 µL) in dry acetonitrile (20 mL) were added and the mixture was heated to 70°C for 16 hours. Further [1-(R)-(3,5-bis-trifluoromethyl-phenyl)-ethyl]-methylamine hydrochloride (170 mg) and DIPEA (100 µL) were added and the mixture was stirred at 70°C for further 4 hours. Next, the mixture was allowed to cool to r.t., taken up with AcOEt (30 mL), washed with a 1 N hydrochloric acid cold solution (3 x 15 mL) and brine (2 x 10 mL). The organic layer was dried and concentrated *in vacuo* to a residue, which was purified by flash chromatography (CH/AcOEt 8:2) to give:
3. intermediate **12a** (230 mg) as a white foam,
4. intermediate **12b** (231 mg) as a white foam.

### Intermediate 12a

NMR (d₆-DMSO): δ (ppm) 7.98 (bs, 1 H); 7.77 (bs, 2H); 7.24 (dd, 1 H); 6.97 (dd, 1 H); 6.89 (m, 1 H); 5.24 (t, 1 H); 5.14 (q, 1 H); 3.61 (m, 1 H); 3.55 (m, 1 H); 2.71 (m, 2H); 2.56 (s, 3H); 2.50 (m, 2H); 2.26 (s, 3H); 1.57 (d, 3H).

### Intermediate 12b

NMR (d₆-DMSO): δ (ppm) 7.96 (bs, 1 H); 7.75 (bs, 2H); 7.24 (dd, 1H); 6.98 (dd, 1H); 6.93 (dt, 1 H); 5.29 (q, 1H); 5.24 (t, 1 H); 3.56 (m, 1H); 3.48 (m, 1H); 2.70 (s, 3H); 2.50 (m, 4H); 2.26 (s, 3H); 1.54 (d, 3H).

### Intermediate 12a

### Method B

A saturated sodium hydrogen carbonate solution (324 mL) was added to a solution of intermediate **13** (21.6 g) in AcOEt (324 mL) and the resulting mixture was vigorously stirred for 15 minutes. The aqueous layer was back-extracted with further AcOEt (216 mL) and the combined organic extracts were dried and concentrated *in vacuo* to give 2-(R)-(4-fluoro-2-methyl-phenyl)-piperidin-4-one as a yellow oil, which was treated with TEA (19 mL) and AcOEt (114 mL). The solution obtained was added drop-wise over 40 minutes to a solution of triphosgene (8 g) in AcOEt (64 mL) previously cooled to 0°C under a nitrogen atmosphere, maintaining the temperature between 0°C and 8°C. After stirring for 1 hours at 0°C and for 3 hours at 20°C, [1-(R)-(3,5-bis-trifluoromethylphenyl)-ethyl]-methylamine hydrochloride (29.7 g), AcOEt (190 mL) and TEA (38 mL)were added to the reaction mixture which was then heated to reflux for 16 hours.
The solution was washed with 10% sodium hydroxide solution (180 mL), 1% hydrochloric acid solution (4 x 150 mL), water (3 x 180 mL) and brine (180 mL). The organic layer was dried and concentrated *in vacuo* to a residue, which was purified through a silica pad (CH/AcOEt 9:1) to give the title compound (21.5 g) as a brown thick oil.
NMR (d₆-DMSO): δ (ppm) 7.97-7.77 (bs + bs, 3H); 7.24 (dd, 1 H); 6.97 (dd, 1H); 6.88 (td, 1H); 5.24 (m, 1 H); 5.14 (q, 1H); 3.58 (m, 2H); 2.7 (m, 2H); 2.56 (s, 3H); 2.49 (m, 2H); 2.26 (s, 3H); 1.57 (d, 3H).

### Intermediate 13

### 2-(R)-(4-Fluoro-2-methyl-phenyl)-piperidin-4-one L-(+)-mandelate

A solution of L-(+)-mandelic acid (22.6 g) in AcOEt (308 mL) was added to a solution of intermediate **11** (31 g) in AcOEt (308 mL). Then isopropanol (616 mL) was added and the solution was concentrated *in vacuo* to 274 mL. The solution was then cooled to 0°C and further cold isopropanol (96 mL) was added. The thick precipitate was stirred under nitrogen for 5 hours at 0°C, then filtered and washed with cold Et2O (250 mL) to give the title compound as a pale yellow solid (20.3 g).
M.p.: 82-85°C.
NMR (d₆-DMSO): δ (ppm) 7.51 (dd, 1H); 7.40 (m, 2H); 7.32 (m, 2H); 7.26 (m, 1H); 7.0 (m, 2H); 4.95 (s, 1 H); 4.04 (dd, 1 H); 3.31 (m, 1 H); 2.88 (m, 1 H); 2.49-2.2 (m, 4H); 2.29 (s, 3H).
Chiral HPLC: HP 1100 HPLC system; column Chiralcel OD-H, 25 cm x 4.6 mm; mobile phase: n-hexane/isopropanol 95:5 + 1% diethylamine; flow: 1.3 ml/min; detection: 240/215nm; retention time 12.07 minutes.

### Intermediate 14

### 1,4-Dibenzyl-2-piperazinecarboxaldehyde

A solution of ethyl 2,3-dibromopropionate (6 mL) in anhydrous toluene (50 mL) was added to a solution of N,N'-dibenzylethylenediamine (5 g) and DIPEA (12 mL) in anhydrous toluene (50 mL) under a Nitrogen atmosphere. The resulting mixture was heated to 100°C for 21 hours, then allowed to cool to r.t., diluted with AcOEt (100 mL) and washed with brine (3 x 100 mL). The organic extract was dried and concentrated *in vacuo* to a residue which was purified by flash chromatography (CH/AcOEt 9:1) to give ethyl 1,4-dibenzyl-piperazine-2-carboxylate (5.65 g) as a yellow oil, which was used without any purification in the next step.
Diisobutylaluminium hydride (1 M in toluene - 29 mL) was dropped into a solution of ethyl 1,4-dibenzyl-piperazine-2-carboxylate (5.47 g) in anhydrous toluene (110 mL) previously cooled to -78°C under a Nitrogen atmosphere. The solution was stirred at -78°C for 1 hour, then a 20% sodium hydroxide solution (20 mL) was added and the mixture was allowed to warm to r.t.. Further 20% sodium hydroxide solution (50 mL) was added and the solution was extracted with Et2O (2 x 150 mL). The combined organic extracts were dried and concentrated *in vacuo* to give the title compound (5.33 g) as a crude, which was used without any further purification in the next step.
T.I.c.: CH/AcOEt 8:2, Rf=0.36.
NMR (d₆-DMSO): δ (ppm) 9.62 (s, 1 H); 7.4-7.15 (m, 10H); 3.86 (d, 1H); 3.6 (d, 1 H); 3.46 (s, 2H); 3.09 (bt, 1H); 2.82 (t, 1 H); 2.55-2.45 (m, 2H); 2.4-2.3 (m, 3H).

### Intermediate 15

### Hexahydro-pyrrolo[1,2-a]pyrazin-6-one

### Method A:

(Carbethoxymethylene)triphenylphosphorane (11.72 g) was added in two portions to a solution of intermediate **14** (4.95 g) in anhydrous toluene (100 mL) under a Nitrogen atmosphere. The mixture was heated to 80°C for 24 hours, then it was allowed to cool to r.t. and washed with water (100 mL). The organic layer was dried and concentrated *in vacuo* to a residue, which was purified by flash chromatography (CH/AcOEt 85:15) to give ethyl 1,4-dibenzyl-2-piperazine-3-acrylate (4.2 g - T.I.c.: CH/AcOEt 8:2, Rf=0.36).
A solution of ethyl 1,4-dibenzyl-2-piperazine-3-acrylate (2.84 g) in abs. EtOH (40 mL) was hydrogenated over Pd/C 10% (1.42 g) at 3.5 atm. for 2 days. After filtration, the solution was concentrated to nearly 30 mL and heated to 70°C for 16 hours until complete cyclization occurred. The solution was concentrated *in vacuo* and the residue was purified by flash chromatography (DCM/MeOH 7:3) to give the title compound (820 mg) as a pale yellow oil.

### Method B:

Diisobutylaluminium hydride (1.2M in toluene - 262 mL) was dropped into a solution of ethyl 1,4-dibenzyl-piperazine-2-carboxylate (48.4g) synthesised as previously described in anhydrous toluene (450 mL) previously cooled to -78°C under a Nitrogen atmosphere (addition of DIBAL-H took 1.5 hours and the internal temperature was always maintained below -70°C). The solution was stirred at -78°C for 2 hour, then a 10% sodium hydroxide solution (500 mL) was added and the mixture was allowed to warm to r.t.. Further 10% sodium hydroxide solution (400 mL) was added and the solution was extracted with toluene (2 x 250 mL). The combined organic extracts were dried and concentrated *in vacuo* until a volume of -100 mL containing 1,4-dibenzyl-2-piperazinecarboxaldehyde, which was used without any further purification in the next step.
(Carbethoxymethylene)triphenylphosphorane (75 g) was added in two portions to the previous solution of 1,4-dibenzyl-2-piperazine carboxaldehyde in toluene (450 mL) under a Nitrogen atmosphere. The mixture was heated to 80°C overnight, then it was allowed to cool to r.t. and washed with water (2 x 400 mL) and brine (250 mL) The organic layer was dried and concentrated *in vacuo* to a residue, which was purified by flash chromatography (CH/AcOEt 85:15) to give ethyl 1,4-dibenzyl-2-piperazine-3-acrylate (44.8 g - T.I.c.: CH/AcOEt 8:2, Rf=0.36).
To a solution of ethyl 1,4-dibenzyl-2-piperazine-3-acrylate (44.8 g) in MeOH (450 mL) under a Nitrogen atmosphere, ammonium formate (23.2 g) and 5% palladium on charcoal (8.96 g) were added. The resulting mixture was heated to reflux temperature for 6h. After filtration over Celite, the solution was concentrated *in vacuo* and the residue was purified by flash chromatography (DCM/MeOH 8:2) to give the title compound (14.15 g) as a pale yellow oil.

### Method C:

Intermediate 17 (820 g) and toluene (1680 g) were charged in a 5 L stainless steel autoclave and palladium on charcoal (5 %, dry - 50 g) was added. The autoclave was rendered inert with nitrogen, subsequently filled with 100 bar hydrogen, and then heated to 100 °C. When the internal pressure has fallen to 90 bar, the pressure was increased to 100 bar again. After the hydrogen uptake ceased, the autoclave was cooled below 30 °C and the reaction solution was removed. The catalyst was then filtered off with a buchner funnel and washed with toluene (2 x 200 mL). After concentrating the filtrate with a rotatory evaporator, the product was distilled over a 15 cm Vigreux column (bp: 115 to 125 °C @ 0.07 mbar) giving the title compound (574 g) as a slightly yellowish oil.
T.I.c.: DCM/MeOH 7:3, Rf=0.17 (detection with ninhydrin)
NMR (CDCl3): δ (ppm) 4.01 (m, 1 H); 3.54 (m, 1 H); 3.16 (m, 1 H); 3.01 (m, 1 H); 2.81 (m, 1H); 2.6 (dt, 1H); 2.38 (m, 3H); 2.16 (m, 1H); 1.6 (m, 1H).
MS (ES/+): m/z= 141 [M+H]⁺

### Intermediates 16

### (8aS)-Hexahydro-pyrrolo[1,2-a]pyrazin-6-one (16a) and (8aR)-Hexahydro-pyrrolo[1,2-a]pyrazin-6-one (16b) Method A:

Intermediate **15** (746 mg) was separated into the enantiomers via preparative HPLC (Column: Chiralpack AD 25 x 2 cm; mobile phase: n-hexane/EtOH 8:2; flux=1 mL/min;λ=225 nm). Thus, intermediate **16a** (330 mg) and intermediate **16b** (320 mg) were obtained.

### Intermediate 16a (enantiomer 1) :

HPLC: Column Chiralpack AD 25cm x 4.6mm x 5µ; mobile phase n-hexane/EtOH 8:2;
flux=1 mL/min; λ=225 nm; retention time 10.7 minutes. Ratio **16a/16b**=100:0.

### Intermediate 16b (enantiomer 2):

HPLC: Column Chiralpack AD 25cm x 4.6mm x 5µ; mobile phase n-hexane/EtOH 8:2;
flux=1 mL/min; λ=225 nm; retention time 12.8 minutes. Ratio **16a/16b**=0:100.

### Intermediate 16b:

### Method B:

A solution of L-(+)-mandeiic acid (13.03 g) in isopropanol (60 mL) was dropped over 20 minutes into a solution of intermediate **15** (12 g) in isopropanol (60 mL) under a Nitrogen atmosphere. The suspension was stirred at 23°C for 2 hours, then it was filtered off and washed with further isopropanol (120 mL). The solid obtained (ratio enantiomers 20:80) was recrystallized three times from isopropanol (10 volumes) until a complete HPLC enantioselectivity was detected. In this way, (8aR)-hexahydro-pyrrolo[1,2-a]pyrazin-6-one L-(+)-mandelate (5.84 g - enantiomer 2) was obtained.
This material (6.469 g) was dissolved in EtOH (40 mL) and water (4 mL) and stirred with a suspension of resin IRA68 (112 g - previously washed with a 0.05N sodium hydroxide solution (370 mL) and water (4 L) until neutral pH) in EtOH (200 mL). The mixture was stirred at 23°C for 1.5 hours, then filtered off. The organic layer was concentrated *in vacuo* to give the title compound (3.1 g) as a white solid.

### Intermediate 16b:

HPLC: Column Chiralpack AD 25cm x 4.6mm x 5µ; mobile phase n-hexane/EtOH 8:2;
flux=1 mL/min; λ=225 nm; retention time 12.8 minutes. Ratio **16a/16b**=0:100.

### Intermediate 16a:

### Method B:

A part of the mother liquors (3.48 g with ratio **16a:16b**=63:37) was treated with a suspension of resin IRA68 (70 g - previously washed with a 0.05N sodium hydroxide solution (150 mL) and water until neutral pH) in EtOH (150 mL) and water (1 mL). The mixture was stirred at 23°C for 2 hours, then filtered off. The organic layer was concentrated *in vacuo* to give the free hexahydro-pyrrolo[1,2-*a*]pyrazin-6-one (1.6 g) as colourless oil. This material (1.6 g) was dissolved in isopropanol (8 mL) and treated with a solution of D-(-)-mandelic acid (1.74 g) in isopropanol (8 mL).
The suspension was stirred at 23°C for 16 hours, then it was filtered off and washed with further isopropanol (120 mL). The solid obtained (ratio enantiomers 86:14) was recrystallized three times from isopropanol (10 volumes) until a complete HPLC enantioselectivity was detected. In this way, (8aS)-hexahydro-pyrrolo[1,2-a]pyrazin-6-one D-(-)-mandelate (0.88 g - enantiomer 1) was obtained.
This material (0.88 g) was dissolved in EtOH (10 mL) and water (1 mL) and stirred with a suspension of resin IRA68 (15 g - previously washed with a 0.05N sodium hydroxide solution (50 mL) and water until neutral pH in EtOH (30 mL). The mixture was stirred at 23°C for 1 hour, then filtered off. The organic layer was concentrated *in vacuo* to give the title compound (0.434 g) as a white solid.

### Intermediate 16a:

HPLC: Column Chiralpack AD 25cm x 4.6mm x 5µ; mobile phase n-hexane/EtOH 8:2;
flux=1 mL/min; λ=225 nm; retention time 10.7 minutes. Ratio **16a/16b**=100:0.

### Intermediate 17

### 3-Pyrazin-2-yl-propionic acid ethyl ester

Butyl lithium (2.5M in hexane - 2560 mL) was added within 2 hours into a 10 L flask charged with THF (3350 mL) and diisopropylamine (658 g) while the temperature was maintained at 0 - 5°C with an ice bath. The LDA solution was then precooled to -50°C and a mixture of methylpyrazine (606 g) and THF (590 mL) was added within 2 hours under vigorous stirring at -40 to -30°C. The deep red anion solution is then pumped to a cooled (-60°C) mixture of *tert*-butyl bromoacetate (1255 g) and THF (3360 mL) in a 20 L reactor. During the addition of the anion solution, the temperature in the reaction vessel did not exceed -55 °C. After the addition, the mixture is stirred for further 30 min at -55 °C and then transferred to a 30 L reactor (the transesterification and removal of solvents can be done for two runs at once). A solution of sodium ethylate (142 g) dissolved in EtOH (2200 mL) was then added to the orange mixture and about 12 L of solvents were distilled off until a temperature of 80°C was reached in the distillation head and 100°C in the boiling liquid. The mixture was cooled to approximately 30°C and then toluene (840 mL), AcOEt (840 mL), and water (1180 mL) were added. After separation of the phases, the organic layer was extracted three times with AcOEt (420 mL) and toluene (170 mL) each. The combined organic phases were then concentrated *in vacuo* and the residue was distilled over a Vigreux column (bp 115 to 130 °C @ 0.07 mbar) giving the title compound (579 g).
T.I.c.:CH/EtOAc= 1:1, Rf=0.36.
¹H-NMR (d₆-DMSO): δ (ppm) 8.57 (d, 1H); 8.52 (dd, 1H); 8.45 (d, 1H); 4.01 (q, 2H); 3.04 (t, 2H); 2.76 (t, 2H); 1.12 (t, 3H).
MS (ES/+): m/z= 181 [M+H]⁺

### Intermediate 18

### (8aS)-Hexahydro-pyrrolo[1,2-a]pyrazin-6-one S-(+)-O-acetylmandelate (enantiomer 1)

A solution of (S)-(+)-O-acetylmandelic acid (2.77 g) in acetone (12 mL) was added drop-wise to a solution of intermediate **15** (4 g) in acetone (28 mL) at 20°C. The resulting mixture was seeded to initiate the precipitation.
The obtained precipitate was stirred at 20°C over 4 hours then filtered washing with acetone (12 mL). The solid was dried *in vacuo* at 40°C for 18 hours to give the title compound (3.44 g) as a white solid.
HPLC: Column Chiralpack AD 25 x 4.6 x 5µm; mobile phase n-hexane/EtOH=1:1; flow=1ml/min; λ= 210 nm; retention times title compound 5.42min., (8aR) enantiomer 6.06min. E.e.>94%.
¹H-NMR (d₆-DMSO): δ (ppm) 9.5 (broad, 1H); 7.42 (m, 2H); 7.32 (m, 3H); 5.62 (s, 1H); 3.79 (dd, 1H); 3.55 (m, 1 H); 3.14 -3.02 (2dd, 2H); 2.80 (dt, 1H); 2.52 (dt, 1H); 2.40 (t, 1 H); 2.19 (m, 2H); 2.06 (s, 3H); 2.05 (m, 1H); 1.49 (m, 1H).
MS (ES/+): m/z= 141 [M+H-PhCH(OAc)COOH]⁺.

### Representative NK1 antagonist Example 3 (ENK3)

### 2-(R)-(4-Fluoro-2-methyl-phenyl)-4-(R)-((8aS)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide (Example 3a) and 2-(R)-(4-Fluoro-2-methyl-phenyl)-4-(S)-((8aS)-6-oxohexahydro-pyrrolo[1,2-a]-pyrazin-2-yl)-piperidine-1-carboxylic acid [1-(R)-(3,5-bis-trifluoromethyl-phenyl)-ethyl]-methylamide (Example 3b)

### Method A:

Intermediate **12a** (168 mg) and sodium triacetoxyborohydride (127 mg) were added to a solution of intermediate **16a** (80 mg) in anhydrous acetonitrile (4 mL) under a Nitrogen atmosphere. The mixture was stirred at 23°C for 14 hours. The solution was diluted with a 5% sodium hydrogen carbonate solution (5 mL) and extracted with AcOEt (2 x 10 mL). The combined organic extracts were dried and concentrated *in vacuo* to a residue which was purified by flash chromatography (AcOEt/MeOH 9: 1) to give three fractions:
1. example **3a** (18 mg) a a white solid
2. mixture of example **3a** and **3b** (160 mg)
3. example **3b** (8 mg) as a white solid.

### Method B:

A solution of intermediate **16a** (2.4 g) in anhydrous acetonitrile (80 mL) was added to a solution of intermediate **12a** (5.7 g) in anhydrous acetonitrile (30 mL) under a Nitrogen atmosphere. Sodium triacetoxyborohydride (4.36 g) was added in three portions every 15 minutes and the mixture was stirred at 23°C for 22 hours. The solution was diluted with water (75 mL) and a saturated sodium hydrogen carbonate solution (25 mL) and extracted with AcOEt (2 x 200 mL). The combined organic extracts were dried and concentrated *in vacuo* to a residue which was purified by flash chromatography (CH/AcOEt/MeOH 50:50:8) to give four fractions:
1. mixture example **3a** and example **3b** (1.27 g) in ratio 1:1
2. example **3b** (1.66 g) (ratio **3a:3b**=13:87)
3. example **3b** (420 mg) (ratio **3a:3b**=5:95)
4. example **3b** (800 mg) (ratio **3a:3b**=2:98)

### Example 3a:

T.I.c.:AcOEt/MeOH 8:2, Rf=0.55.
MS (ES/+) m/z=629 [M+H]⁺.
HPLC: Column Supelcosil ABZ Plus 25cm x 4.6mm x 5µ; mobile phase NH₄OAc 10 mmol/CH₃CN from 60:40 to 10:90 in 5 min. then NH₄OAc 10 mmol/CH₃CN for 10 min.; flux=0.8 mL/min; λ=220 nm; retention time 9.27 minutes.

### Example 3b:

T.I.c.:AcOEt/MeOH 8:2, Rf=0.48.
MS (ES/+) m/z=629 [M+H]⁺.
HPLC: Column Supelcosil ABZ Plus 25cm x 4.6mm x 5µ; mobile phase NH₄OAc 10 mmol/CH₃CN from 60:40 to 10:90 in 5 min. then NH₄OAc 10 mmol/CH₃CN for 10 min.; flux=0.8 mL/min; λ=220 nm; retention time 8.84 minutes.

### Representative NK1 antagonist Example 4 (ENK4)

### 2-(R)-(4-Fluoro-2-methyl-phenyl)-4-(S)-((8aS)-6-oxo-hexahydro-pyrrolo[1,2,a]-pyrazin-2-yl)-piperidine-1-carboxylic acid [1-(R)-(3,5-bis-trifluoromethyl-phenyl)-ethyl]-methylamide hydrochloride

A solution of example **3b** (8 mg) in dry Et2O (1 mL) was treated with hydrochloric acid (1M in Et2O - 14 µL) at 0°C under a Nitrogen atmosphere. The resulting mixture was stirred at 0°C for 20 minutes, then the mixture was concentrated *in vacuo.* The precipitate was washed with pentane (2 mL) to give the title compound as a white solid (7.6 mg). NMR (d₆-DMSO): δ (ppm) 10.22 (bs, 1H); 7.99 (s, 1H); 7.67 (s, 2H); 7.22 (dd, 1H); 6.94 (dd, 1 H); 6.81 (t, 1 H); 5.31 (q, 1 H); 4.2 (dd, 1 H); 4.0-3.86 (bm, 2H); 3.6-3.4 (m, 2H); 3.1-2.7 (m, 4H); 2.73 (s, 3H); 2.4-2.0 (m, 5H); 2.35 (s, 3H); 1.94 (m, 1H); 1.74 (q, 1H); 1.57 (d, 3H); 1.46 (d, 3H).
MS (ES/+) m/z=629 [M+H-HCl]⁺.
HPLC: Column Supelcosil ABZ Plus 25cm x 4.6mm x 5µ, mobile phase NH₄OAc 10 mmol/CH₃CN from 60:40 to 10:90 in 5 min. then NH₄OAc 10 mmol/CH₃CN from 10:90 for 10 min.; flux=0.8 mUmin; λ=220 nm; retention time 8.86 minutes.
Column X-Terra 4.6 x 100mm, RP18 3.5µm; mobile phase: eluant A: NH₄HCO₃ 5mM (pH=8)/CH₃CN 90/10 - eluant B: NH₄HCO₃ 5mM (pH=8)/ CH₃CN 10/90 - Gradient: from 50% B to 100% B in 7.5min; 100% B for 0.5min then 50% B for 3min.; column temp.: 40°C; flow= 1mL/min; λ= 210 nm; retention time 4.15 minutes.

### Representative NK1 antagonist Example 4a (ENK4a)

### 2-(R)-(4-Fluoro-2-methyl-phenyl)-4-(S)-((8aS)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide hydrochloride as anhydrous crystalline form

A 2% sodium hydroxide solution (100 mL) was added to a suspension of example 5 (10 g) in AcOEt (150 mL). Then the two phases mixture were stirred for 10 minutes and the layers were separated. The organic phase was washed with water (100 mL) and then concentrated *in vacuo* up to 40 mL. AcOEt (100 mL) was added to the organic phase, which was then concentrated *in vacuo* a second time up to 40 mL. The solution was further diluted with AcOEt (60 mL) and 5-6N hydrochloric acid in isopropanol (3 mL) was added. After 5 minutes the clear solution was seeded. Precipitation occurred in a few minutes and after further 20 minutes stirring, n-heptane (100 mL) was added in 10-15 minutes. The obtained mixture was stirred 2 hours at 20°C. The solid was then filtered, washed with AOEt/n-heptane 1/1 (60 mL) and dried *in vacuo* at 40°C for 16 hours to give the title compound (8.08 g) as a white solid.
X ray podwer diffraction data are reported in table 2

**Table 2**

| The X-ray podwer diffraction pattern of the product of the Example **4a** in terms of d spacing is as follows | |
|---|---|
| Angle(°2 Theta) | d value(A) |
| 3,412 | 25,87492 |
| 6,87 | 12,85613 |
| 9,867 | 8,95664 |
| 12,877 | 6,86899 |
| 14,274 | 6,19974 |
| 15,4 | 5,74895 |
| 16,732 | 5,29424 |
| 17,323 | 5,11486 |
| 17,966 | 4,93311 |
| 18,521 | 4,78656 |
| 19,557 | 4,53525 |
| 22,12 | 4,01529 |
| 22,382 | 3,96884 |
| 24,311 | 3,65818 |
| 27,117 | 3,28566 |
| 27,836 | 3,20239 |
| 28,374 | 3,14292 |
| 28,846 | 3,0925 |
| 29,372 | 3,03835 |
| 33,9 | 2,64214 |

### Representative NK1 antagonist Example 4b (ENK4b)

### 2-(R)-(4-Fluoro-2-methyl-phenyl)-4-(S)-((8aS)-6-oxo-hexahydro-pyrrolo[1,2,a]-pyrazin-2-yl)-piperidine-1-carboxylic acid [1-(R)-(3,5-bis-trifluoromethyl-phenyl)-ethyl]-methylamide hydrochloride as dihydrate crystalline form

To a 265 mg of example **4a ,** 3 ml of water was added. The suspension was stirred overnight at 25°C and then centrifuged for 5 min at 10000 rpm. The solid was filtered using a centrifugal filter device (Millipore Ultrafree-MC 0.45µm) to obtain the title compound (250mg)
X ray podwer diffraction data are reported in table 3

**Table 3**

| The X-ray podwer diffraction pattern of the product of the Example **4b** in terms of d spacing is as follows | |
|---|---|
| Angle(°2-Theta) | d value(A) |
| 3,233 | 27,30972 |
| 6,353 | 13,90157 |
| 12,14 | 7,28437 |
| 12,647 | 6,99378 |
| 13,282 | 6,6605 |
| 13,5 | 6,55347 |
| 15,48 | 5,71928 |
| 16,324 | 5,42557 |
| 16,779 | 5,27951 |
| 17,825 | 4,97188 |
| 19,022 | 4,66158 |
| 19,414 | 4,5685 |
| 19,901 | 4,45772 |
| 21,339 | 4,1605 |
| 21,915 | 4,05245 |
| 22,21 | 3,99923 |
| 23,161 | 3,83714 |
| 23,521 | 3,77915 |
| 24,179 | 3,67782 |
| 25,417 | 3,50136 |
| 26 | 3,42415 |
| 26,668 | 3,33994 |
| 28,052 | 3,17821 |
| 28,553 | 3,1236 |
| 29,551 | 3,0203 |
| 31,297 | 2,85568 |
| 32,8 | 2,72816 |
| 34,148 | 2,62353 |

### Representative NK1 antagonist Example 5 (ENK5)

### 2-(R)-(4-Fluoro-2-methyl-phenyl)-4-(S)-((8aS)-6-oxo-hexahydro-pyrrolo[1,2-a]-pyrazin-2-yl)-piperidine-1-carboxylic acid [1-(R)-(3,5-bis-trifluoromethyl-phenyl)-ethyl]-methylamide maleate

### Method A:

Intermediate **18** (25 g) was suspended in acetonitrile (300 mL), then TEA (10.4 mL) was quickly added in order to obtain the free base: the aspect of the slurry did not change as a new precipitate of TEA-acetylmandelate salt was formed. The mixture was kept under stirring for 15-20 minutes. Meanwhile intermediate **12a** (25 g) was dissolved in acetonitrile (125 mL) and the so-obtained solution was quickly added to the slurry. Then Sodium triacetoxyborohydride (15 g) was added all at once and the mixture was kept under stirring conditions for 22 hours. The white precipitate was filtered off and the mother liquors were evaporated to 100 mL. AcOEt (250 mL) was added to the so-obtained mixture and the resulting solution was washed with aqueous 4% sodium hydrogen carbonate solution (2 x 125 mL) and then with 5% sodium chloride solution (125 mL). The organic layer dried and evaporated to 100 mL. Isopropyl alcohol (150 ml) was added and the mixture was evaporated again to 100 mL. This operation was repeated. The final volume of the mixture was adjusted to 200 mL adding further isopropyl alcohol (100 mL). A solution of maleic acid (5.8 g) in isopropyl alcohol (50 mL) was dropped in ca. 10 minutes. The mixture was seeded and precipitation occurred in few minutes. The slurry was stirred 1 hour at 20°C and isoctane (250 mL) was added in 10 minutes. The resulting suspension was stirred at room temperature for 22 hours. The solid was filtered and washed with isopropanol/isoctane 1/1 (150 mL) and dried *in vacuo* at 40°C for 18 hours giving the title compound (13.75g) as a white solid.

### Method B:

Intermediate **18** (1 g) was suspended in acetonitrile (12 mL), then TEA (0.415 mL) was quickly added in order to obtain the free base: the aspect of the slurry did not change as a new precipitate of TEA-acetylmandelate salt was formed. After 30minutes of stirring, the mixture was treated with sodium triacetoxyborohydride (0.6 g) plus formic acid (0.224 mL).
Meanwhile intermediate **12a** (1 g) was dissolved in acetonitrile (6 mL) and the so-obtained solution was quickly added to the slurry and the resulting mixture was kept under stirring conditions for 18 hours. The slurry was evaporated to small volume. AcOEt (10 mL) was added to the so-obtained mixture and the resulting solution was washed with aqueous 4% sodium hydrogen carbonate (2 x 5 mL) and then with 5% sodium chloride solution (5 mL). The organic layer was dried and evaporated to a white foam.
Isopropyl alcohol (10 mL) was added and the mixture was evaporated again to dryness. The resulting foam was, once again, dissolved in isopropyl alcohol (8 mL) and treated drop-wise with a solution of maleic acid (0.232 g) in isopropyl alcohol (2 mL). After 30 minutes the mixture was seeded and precipitation occurred in a few minutes. The slurry was stirred 1 hour at 20°C and then isoctane (10 mL) was added dropwise over 5-10 minutes. The resulting suspension was stirred at room temperature for 19 hours. The solid was filtered and washed with isopropanol/isoctane 1/1 (5 mL) and dried *in vacuo* at 40°C for 18 hours giving the title compound (0.639 g) as a white solid.
HPLC: Column X-Terra 4.6 x 100mm, RP18 3.5µm; mobile phase: eluant A: NH₄HCO₃ 5mM (pH=8)/CH₃CN 90/10 - eluant B: NH₄HCO₃ 5mM (pH=8)/CH₃CN 10/90 - Gradient: from 50% B to 100% B in 7.5 minutes; 100% B for 0.5 minutes then 50% B for 3 minutes; column temp. 40°C; flow= 1mL/min; λ= 210 nm; retention times 4.15 minutes, >99%a/a. ¹H-NMR (d₆-DMSO): δ (ppm) 7.98 (bs, 1H); 7.68 (bs, 2H); 7.21 (dd, 1 H); 6.93 (dd, 1 H); 6.81 (dt, 1 H); 6.09 (s, 2H); 5.31 (q, 1 H); 4.19 (dd, 1 H); 3.93 (m, 1 H); 3.74 (bm, 1 H); 3.46 (m, 1 H); 3.45 (bm, 1H); 3.30 (bm, 2H); 2.93 (bt, 1 H); 2.79 (t, 1 H); 2.73 (s, 3H); 2.73 (bm, 1H); 2.60 (bm, 1H); 2.35 (s, 3H); 2.23 (m, 2H); 2.12 (m, 1H); 2.04 (bd, 1 H); 1.98 (bd, 1 H); 1.84 (m, 1 H); 1.64 (q, 1 H); 1.56 (m, 1 H); 1.46 (d, 3H).
MS (ES/+): m/z= 629 [MH-HOOCCHCHCOOH]⁺

### Intermediate 19

### [2S]-Phenyl-piperidin-[3S]-ylamine

[2S]-Phenyl-piperidin-[3S]-ylamine [2S,3S]-bis(4-methyl-benzoyloxy)-succinic acid salt (1:1) (6.9g) was taken up in concentrated 0.880 aqueous ammonia solution (100ml) and shaken for a few minutes. The basic solution was extracted with chloroform (3x150ml), dried (Na₂SO₄), and concentrated *in vacuo* to give [2S]-phenyl-piperidin-[3S]-ylamine (1.85g) as a colourless oil.
[α]²⁰_{D} (HCl salt) = +65.48° (C=0.006 g/ml)
¹H NMR (HCl salt, D₂O) δ 2.05 (m, 2H), 2.30 (m, 2H), 3.36 (m, 1H), 3.74 (m, 1H), 4.16 (q, 1H, J=4Hz), 4.99 (d, 1H, J=4Hz), 7.45 (m, 2H), 7.59 (m, 3H).

A small sample of the free base (50mg) was derivatized as its trifluoroacetyl analogue for chiral HPLC analysis. The sample was dissolved in acetonitrile (4ml) and treated with 1-(trifluoroacetyl)imidazole (0.4ml). The solution was stirred at 65° for 1h, concentrated *in vacuo* and the residue dissolved in dichloromethane (5ml). The organic layer was washed with dilute sulphuric acid(2ml), then the organic layer concentrated and disssolved in hexane-isoproplyalcohol (98:2) for injection onto the HPLC column.
Chiral HPLC (Chiracel-OD-H column, lot no. 09-02-20709, eluent hexane-isopropylalcohol 98:2, flow rate 1ml/min, detection uv 230nm, temperature 40°) retention time 12.93 mins.

### Intermediate 20

### 2-Hydroxy-5-tetrazol-1-yl-benzaldehyde

A solution of 4-tetrazol-1-yl-phenol (0.01mol) in trifluoroacetic acid (20ml) and hexamethylenetetramine (0.04mol) was heated at 70° for 18h, cooled to room temperature and quenched with 2N solution of sulfuric acid (50ml). The mixture was extracted with ethyl acetate (3x100ml), dried (MgSO₄), filtered and concentrated to give a residue which was purified by FCC (dichloromethane/methanol (9:1)) to afford the title compound in 30% yield.
T.I.c. (dichlormethane/methanol (9:1)) Rf 0.6

Intermediate 21 was prepared through a similar procedure to that described for Intermediate 20:

### Intermediate 21

### 2-Hydroxy-5-(5-trifluoromethyl-tetrazol-1-yl)-benzaldehyde

From 4-(5-trifluoromethyl-tetrazol-1-yl)-phenol (45mmol) to give the title compound (8.8g) as a pale yellow solid.
T.I.c. (Hexane/ether (2:1)) Rf 0.36

### Intermediate 22

### 2-Methoxy-5-tetrazol-1-yl-benzaldehyde

To a solution of 2-hydroxy-5-tetrazol-1-yl-benzaldehyde (2.63mmol) in dimethylformamide (5ml) was added potassium carbonate (3.95mmol) and iodomethane (3.95mmol) and the mixture was stirred under nitrogen atmosphere for 2h. The mixture was poured into water (100ml) and the white solid formed filtered to afford the title compound in a 67% yield.
T.I.c. (ether) Rf 0.45

Intermediate 23 was prepared through a similar procedure to that described for Intermediate 22:

### Intermediate 23

### 2-Methoxy-5-(5-trifuoromethyl-tetrazol-1-yl)-benzaldehyde

From 2-hydroxy-5-(5-trifluoromethyl-tetrazol-1-yl)-benzaldehyde (1.56mmol) to give the title compound as a yellow solid (0.48g).
T.I.c. (ether/hexane (2:1)) Rf 0.38.

### Representative NK1 antagonist Example 6 (ENK6)

### [2-Methoxy-5-(5-trifuoromethyl-tetrazol-1-yl)-benzyl]-([2S,3S]-2-phenyl-piperidin-3-yl)-amine dihydrochloride

A mixture of [2S]-phenyl-piperidin-[3S]-ylamine (1.14mmol), 2-methoxy-5-(5-trifluoromethyl-tetrazol-1-yl-benzaldehyde (1.2mmol), sodium triacetoxyborohydride (2.37mmol) and acetic acid (3 drops) in dichloromethane (25ml) was stirred at 23° under nitrogen for 64h. 2N sodium carbonate solution (50ml) was added and the mixture extracted with dichloromethane (3x25ml). The combined extracts were washed with saturated brine (50ml), dried (MgSO₄) and evaporated. Purification by FCC with dichloromethane/ethanol/ammonia (400:10:1 → 100:10:1) gave a colourless viscous oil. This was dissolved in methanol (10ml) and treated with 2N ethereal hydrogen chloride (~10ml). Evaporation in vacuo and trituration with i-propyl acetate gave the title compound as a white solid (210mg).
T.I.c. (Dichloromethane/ethanol/ammonia (200:10:1)) Rf 0.39
Optical Rotation (c 0.003g/ml. water) +50.35°.

### Intermediate 24

### 1-(1,1-dimethylethyl) 2-methyl (2S)-5-oxo-1,2-pyrrolidinedicarboxylate

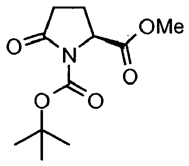

To a solution of commercially available methyl 5-oxo-L-prolinate (20g, 140mmol) in DCM (200ml) were added triethylamine (19.6ml), 140mmol), DMAP(17.2g, 140mmol) and then dropwise a solution of BOC₂O (61g, 280mmol) in DCM (100ml). The resulting red mixture was stirred at room temperature for 2 hours. Then the solvent was removed under reduced pressure and the crude material was purified by chromatography on silica gel eluting with cyclohexane / ethyl acetate (7:3 to 4:6) to afford (after a trituration in hexane / diethylether 1:1) the title compound as a white solid (32.4g, 96%); R_{f} (cyclohexanes:ethyl acetate = 65:35): 0.21; ¹H NMR (300 MHz, CDCl₃) δ(ppm): 4.62 (dd, 1H), 3.78 (s, 3H), 2.68-2.58 (m, 1 H), 2.52-2.45 (m, 1H), 2.37-2.27 (m, 1H), 2.08-1.97 (m, 1H), 1.48 (s, 9H).

### Intermediate 25

### methyl (2S)-2-({[(1.1-dimethylethyl)oxy]carbonyl}amino)-5-oxo-5-{4-[(phennylmethyl)oxy]phenyl}pentanoate

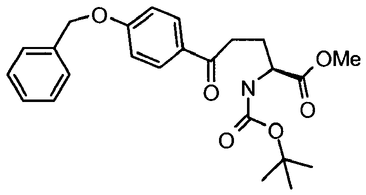

n-Butyl lithium 1.6M solution in hexanes (0.88ml, 1.4mmol) was added dropwise to a solution of commercially available 1-bromo-4-[(phenylmethyl)oxy]benzene (390mg, 1.48mmol) in dry THF (2ml) at -78°C under nitrogen atmosphere. The resulting suspension was stirred at -78°C for 40 minutes and then it was added dropwise to a solution of 1-(1,1-dimethylethyl) 2-methyl (2S)-5-oxo-1,2-pyrrolidinedicarboxylate (300mg, 1.23mmol) in dry THF (2.4ml) previously cooled to -78°C. The mixture was stirred at - 78°C for 40 minutes and at -40°C for 1h, then it was quenched at -40°C with an aqueous saturated ammonium chloride solution. The mixture was diluted with water and extracted with ethyl acetate. The organic phase was then washed with brine, dried over Na₂SO₄, and evaporated under reduced pressure to give the crude material, which was purified by chromatography on silica gel eluting with cyclohexane / ethylacetate (95:5), thus affording the title compound as a white solid (170mg, 32%); R_{f} (cyclohexane:ethyl acetate = 8:2): 0.30; ¹HNMR (300 MHz, CDCl₃) δ(ppm): 7.95 (d, 2H), 7.50-7.33 (m, 5H), 7.03 (d, 2H), 5.20 (bs, 1H), 5.15 (s, 2H), 4.45-4.35 (m, 1H), 3.78 (s, 3H), 3.15-2.95 (m, 2H), 2.36-2.26 (m, 1H), 2.16-2.02 (m, 1 H), 1.45 (s, 9H).

### Intermediate 26

### methyl (2S)-5-{4-[(phenylmethyl)oxy]phenyl}-3,4-dihydro-2H-pyrrole-2-carboxylate

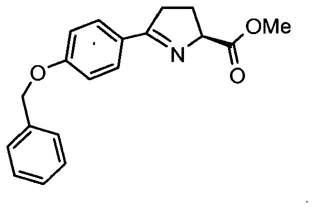

To a solution of methyl (2*S*)-2-({[(1,1-dimethylethyl)oxy]carbonyl}amino)-5-oxo-5-{4-[(phenylmethyl)oxy]phenyl}pentanoate (, 323mg, 0.75mmol) in dry DCM (4ml) at 0°C, under nitrogen atmosphere was added trifluoroacetic acid (1ml) dropwise. The resulting pale pink solution was allowed to warm to room temperature over 1 hour, then it was evaporated under reduced pressure, affording the title compound (D7, 291 mg, 0.68mmol, 91 %) as a greenish oil which may be used in the next step without any further purification; Rt (HPLC): 3.69min; MS: (ES/+) m/z: 310 [MH⁺], C19H19NO3 requires 309.

### Intermediate 27: methyl (5R)-5-{4-[(phenylmethyl)oxy]phenyl}-L-prolinate (I27)

### Intermediate 28: methyl (5S)-5-{4-[(phenylmethyl)oxy]phenyl}-L-prolinate (I28)

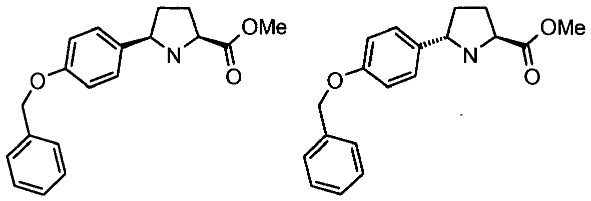

To a solution of methyl (2S)-5-{4-[(phenylmethyl)oxy]phenyl}-3,4-dihydro-2H-pyrrole-2-carboxylate (13.7g, 32.4mmol) in MeOH (200ml) was added PtO₂ (240mg) and the mixture was stirred under a hydrogen atmosphere (2 atmos) for 6 hours. Then the catalyst was filtered off and the solvent removed under reduced pressure to give a red oil which was dissolved in ethyl acetate and washed with NaHCO₃ solution. The resulting crude material was purified by chromatography on silica gel eluting with cyclohexane / ethyl acetate (9:1 to 8:2) to afford the title compounds:
I27, 4.15g, 13.3mmol, Y=41%. MS: (ES/+) m/z: 312 [MH⁺]. C19H21NO3 requires 311. Rt (HPLC): 3.80min. Rf (cyclohexane:ethyl acetate = 7:3): 0.18. ¹HNMR (300 MHz, CDCl₃) δ(ppm): 7.40 (d, 2H); 7.35 (t, 2H); 7.33 (d, 2H); 7.29 (t, 1H); 6.93 (d, 2H); 5.03 (s, 2H); 4.23 (dd, 1 H); 4.00 (dd, 1 H); 3.71-3.79 (m, 3H); 2.18-2.30 (m, 1 H); 2.09-2.18 (m, 2H); 1.67-1.78 (m, 1 H). NOE between the proton at C2 and the proton at C5 could be observed.
127, 0.6g, 1.9mmol, Y=6%. MS: (ES/+) m/z: 312 [MH⁺]. C19H21NO3 requires 311; Rt (HPLC): 3.73min. Rf (cyclohexane:ethyl acetate = 7:3): 0.32. ¹HNMR (300 MHz, CDCl₃) δ(ppm): 7.40 (d, 2H); 7.35 (t, 2H); 7.29 (d, 2H); 7.28 (t, 1 H); 6.91 (d, 2H); 4.97-5.07 (m, 2H); 4.29 (dd, 1 H); 4.09 (dd, 1H); 3.71-3.75 (m, 3H); 2.29-2.42 (m, 1 H); 2.09-2.20 (m, 1H); 1.90-2.02 (m, 1H); 1.69-1.82 (m, 1H). NOE between the proton at C2 and the proton at C5 was not observed.

### Intermediate 29: (5R)-5-{4-[(phenylmethyl)oxy]phenyl}-L-proline (I29)

### Intermediate 30: (5R)-1-{[(1,1-dimethylethyl)oxy]carbonyl}-5-{4-[(phenylmethyl)oxy]phenyl}-L-proline (I30)

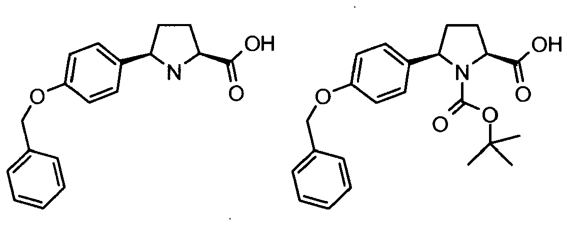

To a solution of methyl (5*R*)-5-{4-[(phenylmethyl)oxy]phenyl}-L-prolinate (120mg, 0.38mmol) in THF (2.3ml) was added LiOH monohydrate (26mg, 0.61mmol) dissolved in water (1.1ml), followed by methanol (1.1ml). The resulting solution was stirred at room temperature for 2.5 hours, then left overnight at -18° C.Then the organic solvent was evaporated under reduced pressure maintaining the temperature at 38°C and the aqueous residue containing the acid intermediate (I29, Rt (HPLC) = 3.63min. MS: (ES/+) m/z: 298 [MH⁺]. C18H19NO3 requires 297) was treated with BOC₂O (168mg, 0.77mmol) dissolved in THF (1.1ml). The reaction mixture was stirred at room temperature for 3.5 hours. The organic solvent was evaporated and the basic aqueous solution was acidified at 0°C with aqueous 1N HCl solution to pH=3, this acidic aqueous solution was extracted with ethyl acetate (2x10ml). The organic phase dried over Na₂SO₄ and evaporated under reduced pressure gave a solid, which was titurated in n-hexanes (3x6ml) affording the title compound as a white powder (130, 137mg, 90% for two steps); Rt (HPLC): 5.81min; Rf (cyclohexane:ethyl acetate = 1:1): 0.34; MS: (ES/+) m/z: 420 [M+Na⁺] C23H27NO5 requires 397; MS: (ES/-) m/z: 396 [M-H] C23H27NO5 requires 397; ¹H NMR (300 MHz, CDCl₃) δ(ppm): 7.5-7.3 (m, 5H), 7.10 (bm, 2H), 6.90 (d, 2H), 5.08 (s, 2H), 4.65 (bm, 1H), 4.50 (bm, 1H), 2.58 (bm, 1H), 2.31 (bm, 1H), 2.11-1.90 (m, 2H), 1.16 (s, 9H).

### Intermediate 31

### 1-[(4-bromophenoxy)methyl]-2-fluorobenzene

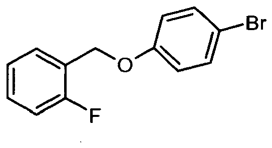

Procedure 1: To a solution of 4-bromophenol (502.08g) dissolved in acetone (7322mL) was added K₂CO₃ (570g) and then benzylbromide (523g). The mixture was heated under reflux for 2hrs. The reaction mixture was then cooled at 25°C, filtered and the filter cake was washed with MTBE (1046mL). The combined filtrate was concentrated to 1000mL and MTBE (4184mL) were added. The mixture was washed with an aqueous 1 M NaOH solution (1464mL), then with brine (1300mL) and the organic phase was concentrated to dryness. THF (1300mL) was added and the solvent was removed under reduced pressure to afford the title compound (902.1g); ¹H NMR (400 MHz, DMSO-d6) δ(ppm): 7.54 (td, 1H); 7.46 (d, 2H); 7.42 (m, 1H); 7.23 (m, 2H); 7.01 (d, 2H); 5.13 (s, 2H).

Procedure 2: A stirred mixture of 4-bromophenol (19.22g, 111mmol), orthofluorobenzyl bromide (20g, 105.8mmol) and potassium carbonate (21.9g, 158.4mmol) in acetone (280ml) was heated at reflux for 6 hours. The reaction mixture was cooled to room temperature and filtered, washing the solid with TBME (40ml). The combined filtrate and washings were concentrated under vacuum to a final volume of about 40ml. The resulting solution was diluted with TBME (160ml) and washed with 1 M sodium hydroxide and brine, then concentrated under vacuum to an oil which slowly solidified to give the title compound (28.9g).
1H NMR (300 MHz, CHCl3-*d*). δ(ppm): 5.10 (s, 2H), 6.86 (m, 2H), 7.10 (m, 1H), 7.17 (m, 1H), 7.29 (m, 1H), 7.35 (m, 2H), 7.38 (m, 1H).

### Intermediate 32

### methyl (2S)-2-({[(1,1-dimethylethyl)oxy]carbonyl}amino)-5-(4-{[(2-fluorophenyl)methyl]oxy}phenyl)-5-oxopentanoate

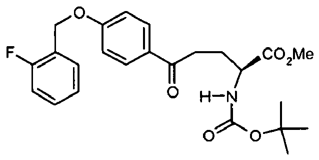

Procedure 1: To a stirred suspension of magnesium metal (90g) in dry THF (600mL) under a nitrogen atmosphere at room temperature was added iodine (0.3g). The mixture was heated to an internal temperature of 64 +/- 2°C. A solution of 1-[(4-bromophenoxy)methyl]-2-fluorobenzene (693g) in THF (1500mL) was added in two batches. Firstly 45 mL was added. Secondly, the remaining solution (1455 mL) was added dropwise. After addition, the reaction was heated at reflux for 1 h. The reaction mixture was cooled to room temperature. This reaction mixture was then added slowly to a solution of commercially available 1-tert-butyl 2-methyl (2S)-5-oxopyrrolidine-1,2-dicarboxylate (300g) in THF (1500mL) cooled to -60°C, maintaining the internal temperature below -60°C. The addition was completed in 2 hours. The reaction mixture was stirred for a further 15 minutes after addition. Isopropyl alcohol (300mL) was then added dropwise whilst maintaining the temperature below -60°C. A mixture of aqueous saturated ammonium chloride solution/aqueous saturated sodium chloride solution (2/1; 900mL) was added whilst maintaining the temperature at -50°C. Water (600 mL) was added to dissolve the yellow precipitate. The organic phase was separated and was washed with aqueous 13% NaCl solution (600mL). The organic phase was concentrated to dryness. EtOAc (1500mL) was then added and the solution was evaporated under reduced pressure to remove water. The residue was purified by chromatography on silica gel eluting with cyclohexane / ethyl acetate (90:10 to 8:2) to afford the title compound (287 g); ¹H NMR (600 MHz, DMSO-d6) δ(ppm): 7.93 (d, 2H); 7.57 (td, 1H); 7.44 (m, 1 H); 7.27 (m, 3H); 7.14 (d, 2H); 5.24 (s, 2H); 4.04 (m, 1H); 3.61 (s, 3H); 3.03 (m, 2H); 1.94 (m, 2H); 1.38 (s, 9H).

Procedure 2: To a mixture of magnesium turnings (12.79g. 533mol), a trace of iodine and 1,2-dibromoethane in THF (86.ml)at 70-75°C, a solution of (4-bromophenyl (2-fluorophenyl)methyl ether) (100g, 355.6mmol) in THF (216.25ml) was added over about 2 hours. The mixture was heated for a further 2 hours at 70-75°C then cooled to room temperature to give a solution of the Grignard reagent. A solution of 1-(1,1-dimethylethyl) 2-methyl (2S)-5-oxo-1,2-pyrrolidinedicarboxylate (43.25g, 177.8mmol) in THF (216.25ml) was cooled to -60°C and the solution of the Grignard reagent was added over 1 hour, then the mixture was stirred for 3 hours at -60°C. Isopropanol (43.25ml) was added dropwise, followed by saturated aqueous ammonium chloride (86.5ml) and brine (43.25ml), then the mixture warmed to room temperature. Water (173ml) and 50% acetic acid (50ml) to pH 6-7, followed by ethyl acetate (129.7ml). The layers were separated and the aqueous extracted with ethyl acetate (2 x 129.7ml). The combined organic layers were washed with brine then concentrated under vacuum. The residue was stirred with hexane (216.2ml), then the solid was filtered and washed with hexane. To the resulting solid, isopropanol (432.5ml) was added and the mixture stirred at 45°C for 15 minutes, then cooled to 5-10°C and stirred for 2 hours. The solid was filtered, washed with isopropanol and dried to give the title compound as a solid.
1 H NMR (300 MHz, CHCl3-*d*): δ(ppm): 1.42 (s, 9H); 2.04 (m, 1 H); 2.28 (m, 1 H); 3.03 (m, 2H); 3.74 (s, 3H); 4.37 (m, 1H); 5.19 (b, 1H); 5.20 (s, 2H); 7.02 (d, 2H); 7.11 (t, 1 H); 7.17 (t, 1 H); 7.33 (m, 1H); 7.48 (t, 1H); 7.94 (d, 2H).

### Intermediate 33

### methyl (2S)-5-{4-[(2-fluorobenzyl)oxy]phenyl}-3,4-dihydro-2H-pyrrole-2-carboxylate

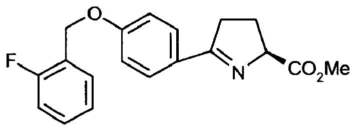

Procedure 1: To a solution of methyl (2*S*)-2-({[(1,1-dimethylethyl)oxy]carbonyl}amino)-5-(4-{[(2-fluorophenyl)methyl]oxy}phenyl)-5-oxopentanoate (243g) in dry DCM (2430mL) at 0°C was added TFA (461 mL) dropwise. The mixture was allowed to warm to room temperature and stirred for 3hrs. Solvent and the excess TFA were removed under vacuum and the resulting dark oil was stripped with EtOAc (2 x 1215mL) and left overnight under a high vacuum. The title compound (392 g) was obtained as a red oil and used in the following step without any further purification; ¹H NMR (400 MHz, DMSO-d6) δ(ppm): 8.16 (m, 2H); 7.60 (td, 1 H); 7.46 (m, 1H); 7.34 (m, 2H); 7.27 (m, 2H); 5.32 (s, 2H); 5.25 (m, 1H); 3.77 (s, 3H); 3.57 (m, 2H); 2.60 (m, 1H); 2.34 (m, 1H).

Procedure 2: A solution of methyl (2*S*)-2-({[(1,1-dimethylethyl)oxy]carbonyl}amino)-5-(4-{[(2-fluorophenyl)methyl]oxy}phenyl)-5-oxopentanoate (46g, 103mmol) in DCM (437ml) was treated dropwise with trifluoroacetic acid (87.4ml) at 0-5°C, then warmed to room temperature and stirred for 3 hours. The solution was cooled to 0-5°C and sodium hydroxide solution added to a final pH of about 7. The aqueous layer was separated and extracted with DCM (13ml), then the combined organic layers were washed with water, dried over sodium sulphate, then concentrated under vacuum to give the title compound as a solid (33.3g). 1 H NMR (300 MHz, CHCl3-*d*): δ(ppm): 2.35 (m, 2H); 2.95 (m, 1 H); 3.12 (m, 1 H); 3.78 (s, 3H); 4.89 (dd, 1H); 5.18 (s, 2H); 7.00 (d, 2H); 7.10 (m, 1H) ; 7.16 (m, 1 H) ; 7.29 (m, 1H);7.5(t, 1H); 7.85 (d, 2H).

### Intermediate 34:

### Methyl (5R)-5-{4-[(2-fluorobenzyl)oxy]phenyl}-L-prolinate

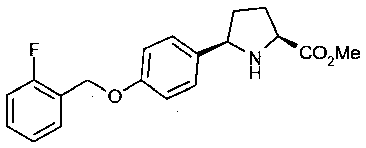

Procedure 1: Methyl (2*S*)-5-{4-[(2-fluorobenzyl)oxy]phenyl}-3,4-dihydro-2*H*-pyrrole-2-carboxylate (392g) was dissolved in EtOAc (3160mL) in a hydrogenation reactor. 5% platinum on carbon (Engelhard code 44379, moisture content ca. 50%, 15.8g) was added, the reactor filled with hydrogen gas to a pressure of 2atm and the reaction mixture was stirred for approximately 1.5 hours. The reactor was depressurised and the spent catalyst filtered through Celite, washing through with EtOAc (2 x 500mL, then further 200mL). Aqueous saturated NaHCO₃ solution (600 mL) was added to the filtrate, followed by aqueous 13% w/w Na₂CO₃ solution (up to pH = 9, 1000mL). The mixture was stirred for 10 minutes and phases were then allowed to separate .The aqueous phase was removed and then the organic layer was washed once with brine (600mL). The resulting solution was concentrated to dryness and the residue was purified by flash chromatography eluting with cyclohexane / ethyl acetate (1:1) to afford the title compound (133 g); ¹H NMR (600 MHz, DMSO-d6) δ(ppm): 7.55 (dt, 1H); 7.41 (m, 1H); 7.34 (m, 2H); 7.23 (m, 2H); 6.97 (m, 2H); 5.12 (s, 2H); 4.09 (dd, 1 H); 3.83 (dd, 1 H); 3.66 (s, 3H); 2.97 (bs, 1H); 2.04 (m, 2H); 1.94 (m, 1 H); 1.52 (m, 1 H).

Procedure 2: A solution of methyl (2*S*)-5-(4-{[(2-fluorophenyl)methyl]oxy}phenyl)-3,4-dihydro-2*H*-pyrrole-2-carboxylate (34g, 103.5mmol) in ethyl acetate (272ml) was placed in an autoclave and treated with trifluoroacetic acid (7.2ml). 5% Platinum on carbon catalyst (1.7g) was transferred as a slurry with ethyl acetate (68ml) and the reaction was stirred at room temperature under 50 psi hydrogen pressure for 5 hours. The mixture was filtered through Hyflo, washing with ethyl acetate (272ml), then the filtrate was washed with aq sodium carbonate solution and brine, dried over sodium sulphate, then concentrated under vacuum, and the residue dried to give the title compound as a crude oil (also comprising some of the anti isomer), 1 H NMR (300 MHz, CHCl3-*d*): δ(ppm): 1.7 (m, 1H); 2.18 (m, 4H) ; 3.75 (s, 3H); 3.91 (m, 1H) ; 4.15 (m, 1H) ; 5.13 (s, 2H); 6.96 (d, 2H) ; 7.07 (m, 1 H); 7.15 (m, 1 H); 7.30 (m, 1 H) ; 7.38 (d, 2H); 7.5 (t, 1H).

### Intermediate 34

### 1-(1,1-Dimethylethyl) 2-methyl (2S,5R)-5-{4-[(phenylmethyl)oxy] phenyl}-1,2-pyrrolidinedicarboxylate

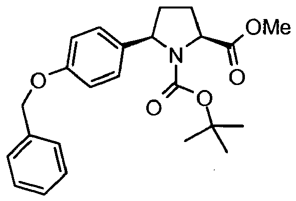

To a solution of methyl (5R)-5-{4-[(phenylmethyl)oxy]phenyl}-L-prolinate (2.6g, 8.35mmol) in DCM (30ml) was added di-tert-butyl dicarbonate (2.0g, 9.18mmol). After stirring for 1 h at room temperature, the mixture was evaporated and the residue was purified by chromatography on silica gel using cyclohexane / ethyl acetate (9:1 to 85:15) to afford the title compound (3.29g, 96%) as a white foam; Rₜ (HPLC): 6.55min; MS: (ES/+) m/z: 434 [M+Na⁺], 312 [M-BOC]; C₂₄H₂₉NO₅ requires 411; ¹H NMR (400 MHz, CDCl₃) δ (ppm): 7.52-7.43 (m, 4H); 7.43-7.37 (m, 2H); 7.37-7.30 (m, 1 H); 6.96 (d, 2H); 5.09 and 5.06 (s,s, 2H); 4.99-4.93 and 4.52-4.44 (m,m, 1H); 4.76-4.68 and 4.39-4.32 (m,m, 1H); 3.82 (s, 3H); 2.36-2.26 (m, 1H); 2.26-2.15 (m, 1H); 2.12-2.01 (m, 1H); 2.01-1.88 (m, 1H); 1.42 and 1.17 (s,s, 9H).

### Reference Description 35

### 1,1-Dimethylethyl (2S,5R)-2-(aminocarbonyl)-2-methyl-5-{4-[(phenylmethyl)oxy]phenyl}-1-pyrrolidinecarboxylate

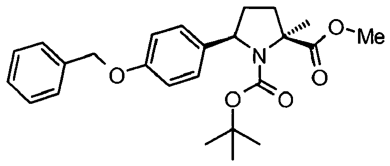

To a solution of 1-(1,1-dimethylethyl) 2-methyl (2*S*,5*R*)-5-{4-[(phenylmethyl)oxy]phenyl}-1,2-pyrrolidinedicarboxylate (1.52g, 3.7mmol) in dry THF (27ml) at -78°C was added LiHMDS (4.0ml, 4.0mmol, 1 M solution in THF). The mixture was allowed to warm to -20°C and was stirred for 40min at that temperature. Then the mixture was again cooled to - 78°C and methyl iodide (3.15g, 22.1mmol) was added. The mixture was left stirring for another 30min at the same temperature. After standard work up, the organic layer was evaporated. The crude material was purified by chromatography on silica gel using cyclohexanes and ethyl acetate (1:0 to 9:1) affording the title compound (1.23g, 78%); Rₜ (HPLC): 6.76min; MS: (ES/+) m/z: 448 [M+Na+]; C25H31NO5 requires 425; ¹H NMR (400 MHz, CDCl₃) δ(ppm): 7.45-7.38 (m, 4H); 7.35 (t, 2H); 7.29 (t, 1H); 6.90 (d, 2H); 5.04 and 5.01 (s,s, 2H); 4.98 and 4.79 (d,d, 1H); 3.79 (s, 3H); 2.50-2.35 (m,1 H); 2.34-2.22 (m, 1H); 1.87-1.73 (m, 2H); 1.58 and 1.55 (s,s, 3H); 1.37 and 1.09 (s,s, 9H). NOE between the methyl group and the proton at C5 could be observed.

### Intermediate 36

### 1-(1,1-Dimethylethyl) 2-methyl (2R,5R)-2-(cyanomethyl)-5-{4-[(phenylmethyl)oxy]phenyl}-1,2-pyrrolidinedicarboxylate

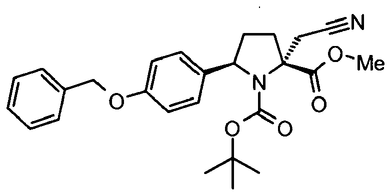

The title compound was prepared (2.71 g, 76%) using a similar procedure as set out earlier in Reference Description 35 using crude 1-(1,1-dimethylethyl) 2-methyl (2*S*,5*R*)-5-{4-[(phenylmethyl)oxy]phenyl}-1,2-pyrrolidinedicarboxylate (3.25 g, 7.89 mmol) and bromoacetonitrile (3.3 ml, 47.38 mmol in 40ml THF); Rₜ (HPLC): 6.4 min; R_{f} (cyclohexane:ethyl acetate = 7:3): 0.40; MS: (ES/+) m/z: 473 [M+Na⁺]; C26H30N2O5 requires 450; ¹H NMR (500 MHz, DMSO-d₆) δ(ppm): 7.46-7.24 (m, 7H); 6.94 (d, 2H); 5.11 (s, 2H); 4.94-4.78 (m, 1 H); 3.78 (s, 3H); 3.33 (d, 1 H); 3.22 (d, 1 H); 2.66-2.52 (m, 1 H); 2.42-2.29 (m, 1H); 2.29-2.16 (m, 1H); 1.92-1.77 (m, 1H); 1.38-0.97 (br. s, 9H); NOE between the aromatic protons and the methyl esther could be observed.

### Intermediate 37

### 1,1-Dimethylethyl (2R,5R)-2-(4-hydroxyphenyl)-6-oxo-1,7-diazaspiro[4.4]nonane-1-carboxylate

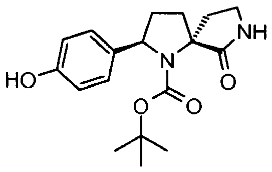

To a solution of 1-(1,1-dimethylethyl) 2-methyl (2*R*,5*R*)-2-(cyanomethyl)-5-{4-[(phenylmethyl)oxy]phenyl}-1,2-pyrrolidinedicarboxylate (2.7g, 5.99mmol): in methanol (50ml) was added Raney Nickel (slurry in water) and the mixture was stirred under a hydrogen atmosphere (7 atmospheres) for 14 hours. The catalyst was filtered off, the solvent removed under reduced pressure, and the solid residue was treated with toluene (3x20ml) and dried under vacuum. The dry white solid obtained was refluxed in methanol (40ml) for five hours until cyclization was complete. The solvent was removed under reduced pressure and the crude material purified by chromatography on silica gel using dichloromethane / methanol (95:5 to 90:10) to afford the title compound as a white solid (1.35g, 68%); Rₜ (HPLC): 3.91 min; R_{f} (dichloromethane:methanol = 9:1): 0.41; MS: (ES/+) m/z: 665 [2M+Na⁺], 355 [M+Na⁺]; C18H24N2O4 requires 332; ¹H NMR (400 MHz, DMSO-d6) δ(ppm): 9.11 (s, 1H); 7.76 and 7.66 (s, s, 1 H); 7.43 (dd, 2H); 6.66 (dd, 2H); 4.81-4.70 (m, 1 H); 3.29-3.20 (m, 1H); 3.19-3.09 (m, 1H); 2.47-2.20 (m, 2H); 2.08-1.85 (m, 2H); 1.84-1.74 (m, 1H); 1.68-1.52 (m, 1H); 1.33 and 1.08 (s, s, 9H).

### Reference Description 38

### 1,1-Dimethylethyl (2S,5R)-2-(aminocarbonyl)-5-(4-{[(2-fluorophenyl)methyl]oxy}phenyl)-2-methyl-1-pyrrolidinecarboxylate

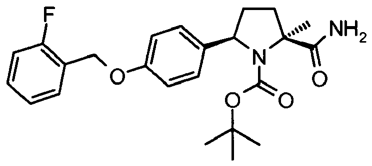

To a solution of 1,1-dimethylethyl (2S,5R)-2-(aminocarbonyl)-5-(4-hydroxyphenyl)-2-methyl-1-pyrrolidinecarboxylate (300mg, 0.936mmol) and potassium carbonate (194mg, 1.4mmol) in acetonitrile (4ml) was added 1-(bromomethyl)-2-fluorobenzene (Sigma Aldrich Ltd.) (170µl, 1.4mmol) and the mixture was stirred overnight at room temperature. After the reaction was finished, as shown by TLC, ethyl acetate and water were added. The organic phase was then washed with brine, dried, filtered and evaporated. The crude material was purified by chromatography on silica gel using cyclohexane / ethyl acetate (8:2 to 7:3) to afford the title compound (306mg, 72%); Rₜ (HPLC): 5.88min; R_{f} (cyclohexane:ethyl acetate = 1:1): 0.51; ¹H NMR (300 MHz, DMSO-d6) δ(ppm): 7.60-7.49 (t, 2H); 7.46-7.32 (m, 2H); 7.29-7.15 (m, 3H); 7.13-7.02 (m, 1H); 6.98-6.88 (m, 2H); 5.11 (s, 2H); 4.87-4.62 (m, 1H); 2.41-2.16 (m, 2H); 1.78-1.64 (m, 1H); 1.64-1.56 (m, 1H); 1.52 (s, 3H); 1.32 and 1.03 (s, s, 9H).

### Intermediate 39:

### 1,1-Dimethylethyl (2R,5R)-2-(4-{[(2-fluorophenyl)methyl]oxy}phenyl)-6-oxo-1,7 diazaspiro[4.4]nonane-1-carboxylate

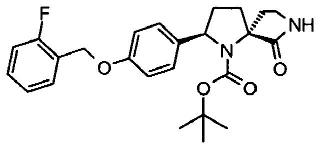

### Method a):

The title compound was prepared using a similar procedure as set out earlier in Reference Description 38 starting from 1,1-dimethylethyl (2*R*,5*R*)-2-(4-hydroxyphenyl)-6-oxo-1,7-diazaspiro[4.4]nonane-1-carboxylate (850 mg, 2.55 mmol) and 2-fluorobenzyl bromide (0.5 ml, 3.83 mmol); R_{f} (HPLC): 5.72min; R_{f} (cyclohexane:ethyl acetate = 3:7): 0.45; MS: (ES/+) m/z: 463 [M+Na⁺]. C25H29FN2O4 requires 440; ¹H NMR (400 MHz, CDCl₃) δ(ppm): 7.61 (d, 2H); 7.56-7.49 (m, 1H); 7.35-7.28 (m, 1H); 7.20-7.13 (m, 1H); 7.12-7.05 (m, 1 H); 7.00-6.94 (dd, 2H); 5.64 and 5.61 (s,s, 1 H); 5.14 and 5.12 (s,s, 2H); 5.08 and 4.88 (d, d, 1 H); 3.58-3.40 (m, 1 H); 3.38-3.24 (m, 1 H); 2.82-2.56 (m, 1 H); 2.43-2.21 (m, 2H); 2.15-2.00 (m, 1 H); 1.95-1.75 (m, 2H), 1.45 and 1.17 (s, s, 9H).

### Method b):

To a solution of 1-(1,1-dimethylethyl) 2-methyl (2*R*,5*R*)-2-(cyanomethyl)-5-(4-{[(2-fluorophenyl)methyl]oxy}phenyl)-1,2-pyrrolidinedicarboxylate (51.3g) in MeOH (- 500 mL) was added CoCl₂·6H₂O (13.04g). To the resulting purple solution were added three batches of NaBH₄ (8.29g, 8.29g and 4.145g respectively; exothermic addition) portionwise every 30 minutes. The reaction mixture was cooled down to ambient temperature, filtered and the resulting solution was heated to reflux overnight. Then the mixture was cooled down and filtered. NH₄Cl saturated solution (513mL) was added and MeOH evaporated in vacuo. The aqueous phase was extracted with EtOAc (2 x 500 mL) and the combined organic phase was evaporated to dryness and the crude material was purified by chromatography on silica gel pad using cyclohexane and ethyl acetate (1:1, 4:6, 3:7) affording the title compound (22.1g) as a white solid.
1 H NMR (400 MHz, CHCl₃-d) δ(ppm): 7.61 (m, 2 H), 7.53 (m, 1 H), 7.31 (m, 1 H), 7.16 (m, 1 H), 7.09 (m, 1 H), 6.97 (m, 2 H), 5.58 (s, 1 H), 5.14 (m, 2 H), 5.04-4.86 (m, 1 H), 3.58 - 3.42 (m, 1 H), 3.32 (m, 1 H), 2.80-2.58 (m, 1 H), 2.32 (m, 2 H), 2.07 (m, 1 H), 1.93-1.78 (m, 2 H), 1.45 (s, 3H), 1.17 (s, 6H).

### Intermediate 40

### 1,1-Dimethylethyl (2R,5R)-2-(4-{[(2-fluorophenyl)methyl]oxy}phenyl)-7-methyl-6-oxo-1,7-diazaspiro[4.4]nonane-1-carboxylate

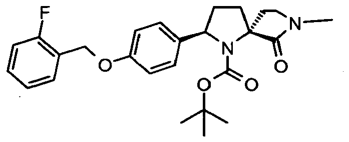

To a solution of 1,1-dimethylethyl (2*R*,5*R*)-2-(4-{[(2-fluorophenyl)methyl]oxy}phenyl-6-oxo-1,7-diazaspiro[4.4]nonane-1-carboxylate (70mg, 0.159 mmol) in dry DMF (1 ml) at 0°C was added NaH 60%wt dispersion in mineral oil (10mg, 0.238 mmol); after 15 minutes of stirring at room temperature, iodomethane (30µl, 0.477 mmol) was added and the resulting mixture was stirred at room temperature for 2.5 hours. The mixture was cooled to 0°C, and water (4 ml) and ethyl acetate (10 ml) were added, the organic layer was washed with ice cold brine (3x10 ml), dried over Na₂SO₄ and evaporated. The crude material was purified by chromatography on silica gel using cyclohexanes / ethyl acetate (7:3) to afford the title compound as a white solid (63 mg, 88%); Rₜ (HPLC): 5.99min; R_{f} (cyclohexane:ethyl acetate = 1:1): 0.28; MS: (ES/+) m/z: 477 [M+Na⁺]; C26H31FN2O4 requires 454. ¹H NMR (400 MHz, CDCl₃) δ(ppm): 7.66 (d, 2H); 7.56-7.49 (m, 1 H); 7.35-7.25 (m, 1H); 7.20-7.13 (m, 1H); 7.12-7.04 (m, 1H); 6.97 (dd, 2H); 5.14 and 5.12 (s, s, 2H); 5.02 and 4.88 (d, d, 1 H); 3.52-3.45 and 3.41-3.22 (m, m, 2H); 2.96 and 2.92 (s, s, 3H); 2.67-2.56 and 2.53-2.42 (m, m, 1 H); 2.40-2.22 (m, 2H); 2.08-1.79 (m, 2H); 1.77-1.68 (m, 1H), 1.40 and 1.16 (s, s, 9H).

### Intermediate 41

### 1-(1,1-Dimethylethyl) 2-methyl (2S,5R)-5-(4-{[(2-fluorophenyl)methyl]oxv}phenyl)-1,2-pyrrolidinedicarboxylate

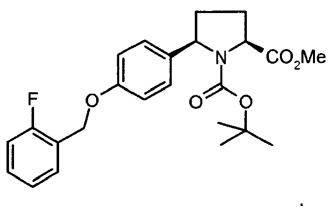

Methyl (5R)-5-(4-{[(2-fluorophenyl)methyl]oxy}phenyl)-L-prolinate (175g) was dissolved in EtOAc (1000mL) and cooled to 0°C. A solution of di-tert-butyl-dicarbonate (127.5g) in EtOAc (750mL) was added dropwise in about 1hour maintaining the temperature at about 0°C. Then the temperature was increased to 25°C and the reaction stirred at 25°C for 2hours. 28%w/w Racemic malic acid (350mL) was added and the mixture stirred for about 10min. The organic phase was washed with saturated NaHCO₃ (700mL). Aqueous pH was - 8. The organic phase was concentrated to a low volume then stripped with cyclohexane (3 x 350mL) to afford the title compound (240.8g). ¹H NMR (400 MHz, CDCl₃) δ(ppm): 7.54 (m, 1 H); 7.50 (d, 2H); 7.33 (m, 1H); 7.18 (dt, 1 H); 7.11 (m, 1H); 6.98 (d, 2H); 5.16 (2s, 2H); 4.97-4.46 (2bm, 1 H); 4.73-4.37 (2t, 1 H); 3.83 (s, 3H); 2.32 (m, 1H); 2.21 (m, 1H); 2.08 (m, 1H); 1.96 (m, 1H); 1.43-1.18 (2bs, 9H).

### Intermediate 42

### 1-(1,1-Dimethylethyl) 2-methyl (2R,5R)-5-(4-{[(2-fluorophenyl)methyl]oxy}phenyl)-2-(2-propen-1-yl)-1,2-pyrrolidinedicarboxylate

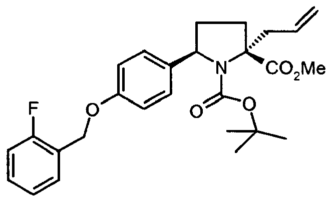

The previous crude of 1-(1,1-dimethylethyl) 2-methyl (2*S*,5*R*)-5-(4-{[(2-fluorophenyl)methyl]oxy}phenyl)-1,2-pyrrolidinedicarboxylate was split in two batches. The first one (100g) was dissolved in dry THF (1000mL) then allyl bromide (42.25g) was added, finally the mixture was cooled to -30°C. 1M LiHMDS in THF (439mL) was added dropwise in about 1.5 hours maintaining the temperature at about -30°C. Water (100mL) was added and temperature allowed to reach 0°C. Saturated NH₄Cl (500mL) was added followed by water (400mL) and EtOAc (500mL). The reaction was warmed to 25°C and the aqueous layer was removed. The organic layer was concentrated to about 700mL and washed with NaHCO₃ saturated solution (200mL). Organic layer was dried over Na₂SO₄ and concentrated to afford the title compound as an oil (119.8g). The second batch (135g) was similarly reacted using dry THF (1350mL), allyl bromide (57.04g) and 1M LiH MDS in THF (471.5mL). After work-up, the title compound was isolated as an oil (179g). ¹H NMR (400 MHz, DMSO-d₆) δ(ppm): 7.53 (m, 1H); 7.39 (m, 3H); 7.22 (m, 2H); 6.95 (m, 2H); 5.82 (m, 1H); 5.12 (m, 4H); 4.80-4.59 (2m, 1H); 3.74-3.71 (2s, 3H); 3.01-1.52 (m, 6H); 1.29-0.98 (2s, 9H).

### Intermediate 43

### 1-(1,1-Dimethylethyl) 2-methyl (2R,5R)-2-(2,3-dihydroxypropyl)-5-(4-{[(2-fluorophenyl)methyl]oxy}phenyl)-1,2-pyrrolidinedicarboxylate

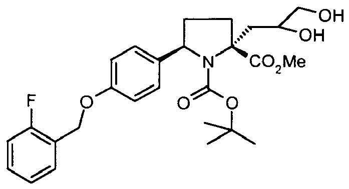

The reaction was performed on the two separated batches of 1-(1,1-dimethylethyl) 2-methyl (2*R*,5*R*)-5-(4-{[(2-fluorophenyl)methyl]oxy}phenyl)-2-(2-propen-1-yl)-1,2-pyrrolidinedicarboxylate obtained from the previous step.The first batch (119.8g) was dissolved in a mixture of 10/1 acetone/water (1200mL). K₂OsO₄2H₂O (4.7g) was added followed by NMO (41.4g) after a few minutes. The mixture was stirred for 7.5 hours. The reaction mixture was treated with EtOAc (1200mL) and washed with saturated NH₄Cl (1200mL) then with saturated NaHCO₃ (1200mL). The organic layer was filtered through a celite/activated charcoal pad and concentrated to low volume. EtOAc (500mL) was added and the solution was washed with brine (300mL) and the organic dried over Na₂SO₄, and evaporated to dryness, EtOAc (300mL) was added and filtered through CUNO filter and washed with EtOAc (50mL). The organic was concentrated to dryness to afford the title compound (135g). The second batch (179g) was dissolved in a mixture of 10/1 acetone/water (1800mL). K₂OsO₄2H₂O (5.5g) was added followed by NMO (56.8g) after a few minutes. The mixture was stirred overnight at room temperature. The reaction mixture was treated with EtOAc (900mL) and washed with saturated NH₄Cl (2 x 900mL) then with saturated NaHCO₃ (900mL). The organic layer was filtered through a celite pad and concentrated to low volume to afford the title compound (250g). ¹H NMR (400 MHz, DMSO-*d*₆) δ(ppm): 7.54 (m, 1 H); 7.40 (m, 3H); 7.22 (m, 2H); 6.95 (m, 2H); 5.11 (m, 2H); 4.95-4.25 (m, 3H); 3.70 (bm, 3H); 3.50-3.10 (m, 3H); 2.50-1.50 (m, 6H); 1.30-0.95 (4bs, 9H).

### Intermediate 44

### 1-(1,1-Dimethylethyl) 2-methyl (2R,5R)-5-(4-{[(2-fluorophenyl)methyl]oxy}phenyl)-2-(2-oxoethyl)-1,2-pyrrolidinedicarboxylate (I43)

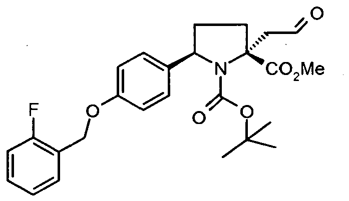

Procedure 1: The reaction was performed on the two separated batches of 1-(1,1-dimethylethyl) 2-methyl (2*R*,5*R*)-2-(2,3-dihydroxypropyl)-5-(4-{[(2-fluorophenyl)methyl]oxy}phenyl)-1,2-pyrrolidinedicarboxylate obtained from the previous step. The first batch (135g) was dissolved in a mixture of 10/1 acetone/water (2000mL) and NalO₄ (74.54g) was added. The solution turned from brown to yellow and a suspension was formed. The mixture was stirred overnight at 25°C. Further NalO₄ (2 x 5.73g) was added and stirred for further 24 hours. EtOAc (1000mL) was added followed by water (1000mL). After mixing the aqueous phase was removed. The organic phase was concentrated to a yellow oil and stripped with EtOAc (250mL) to afford the title compound (109g). The second batch (250g) was dissolved in a mixture of 10/1 acetone/water (1900mL) and NalO₄ (106g) was added. The solution turned from brown to yellow and a suspension was formed. The mixture was stirred for 6 hours then further NalO₄ (8.15g) was added and stirred overnight. EtOAc (1000mL) was added followed by water (2000mL). After mixing the aqueous phase was removed. The organic phase was concentrated to a yellow oil and stripped with EtOAc (300mL) to afford the title compound (180g).
¹H NMR (400 MHz, DMSO-*d*₆) δ(ppm): 9.72 (m, 1 H); 7.56 (m, 1H); 7.42 (m, 3H); 7.24 (m, 2H); 6.99 (m, 2H); 5.14 (m, 2H) 4.90-4.79 (2m, 1 H); 3.80-3.77 (2s, 3H); 3.02 (m, 1H); 2.83 (m, 1 H); 2.55-1.55 (m, 4H); 1.32-1.03 (2s, 9H).

Procedure 2: A solution of 1-(1,1-dimethylethyl) 2-methyl (2*R*,5*R*)-5-(4-{[(2-fluorophenyl)methyl]oxy}phenyl)-2-(2-propen-1-yl)-1,2-pyrrolidinedicarboxylate (15g) in methanol (200ml) was cooled to -10°C. Ozone gas was passed through the solution for 2 hours, then the mixture was warmed to 0-5°C. Dimethylsulphide (7.04ml) was added and the mixture stirred for 1 hours at 0-5°C, then at room temperature for 3 hours. The mixture was then concentrated under vacum at 35-50°C to give a residue. This was treated with water (45ml), then extracted with DCM (2 x 37.5ml). The combined extracts were stirred with a mixture of 60-120 mesh silica gel (45g, DCM (75ml) and 10% oxalic acid (6ml) for 4 hours. The mixture was then filtered and washed with DCM (75ml). The combined flitrates were washed with water (75 ml) and 10% sodium bicarbonate (75ml) and water (75ml). The solution was then dried over sodium sulphate and evaporated, then dried under vacuum to give the title compound as a brownish yellow pasty mass (12.7g).

### Intermediate 45

### 1-(1,1-Dimethylethyl) 2-methyl (2R,5R)-5-(4-{[(2-fluorophenyl)methyl]oxy}phenyl)-2-[2-(methylamino)ethyl]-1,2-pyrrolidinedicarboxylate

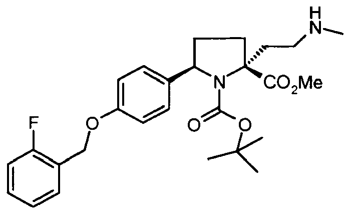

The reaction was performed on the two separated batches of 1-(1,1-dimethylethyl) 2-methyl (2*R*,5*R*)-5-(4-{[(2-fluorophenyl)methyl]oxy}phenyl)-2-(2-oxoethyl)-1,2-pyrrolidinedicarboxylate obtained from the previous step (144, Procedure 1). The first batch (109g) was dissolved in MeOH (440mL) and MeNH₂ in MeOH 2M solution (347mL) was added. AcOH (11mL) was added. NaBH(OAc)₃ (49g) was added portion wise after 10 minutes. More NaBH(OAc)₃ (14.7g) was added portion wise at 25°C. The reaction mixture was quenched with 28% aqueous malic acid (200mL) followed by AcOEt (1000mL). K₂CO₃ was added up to pH-9. Organic phase was concentrated to dryness, re-dissolved in EtOAc (500mL) and extracted with 20% citric acid (4 x 300mL). The combined aqueous phases were treated with EtOAc (500mL) and solid K₂CO₃ was added until pH-9. The phases were separated and the organic phase was dried under Na₂SO₄ and evaporated to dryness to afford the title compound (80g). The second batch (180g) was dissolved in MeOH (628mL) and MeNH₂ in MeOH 2M solution (300mL) was added. AcOH (31mL) was added. NaBH(OAc)₃ (78.8 g) was added portionwise at 0°C after 10 minutes. The reaction mixture was quenched with saturated NH₄Cl (890mL) and EtOAc (890mL). The phases were separated and the aqueous was extracted with EtOAc (4 x 300mL). The organic phase was dried over Na₂SO₄, concentrated to dryness, re-dissolved in EtOAc (500mL) and extracted with 20% citric acid (6 x 150mL). The combined aqueous phases were treated with EtOAc (600mL) and solid K₂CO₃ was added until pH 8/9. The Phases were separated and the organic phase dried over Na₂SO₄ and evaporated to dryness to afford the title compound (78 g). ¹H NMR (400 MHz, DMSO-*d*₆) δ(ppm): 7.55 (m, 1 H); 7.42 (m, 3H); 7.25 (m, 2H); 6.98 (m, 2H); 5.14 (m, 2H); 4.85-4.69 (2m, 1 H); 3.75-3.73 (2s, 3H); 2.36 (bs, 3H) 2.80-2.25 (m, 2H); 2.20-2.00 (m, 3H); 1.70 (m, 1H); 1.32-1.00 (2s, 9H).

### Intermediate 46

### 1-(1,1-Dimethylethyl) 2-methyl (2R,5R)-2-(cyanomethyl)-5-(4-{[(2-fluorophenyl)methyl]oxy}phenyl)-1,2-pyrrolidinedicarboxylate

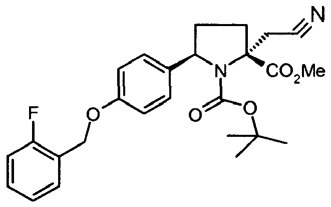

To a solution of 1-(1,1-dimethylethyl) 2-methyl (2*S*,5*R*)-5-(4-{[(2-fluorophenyl)methyl]oxy}phenyl)-1,2-pyrrolidinedicarboxylate (45g) in dry THF (450mL) previously cooled to -65°C, was added 1M LiHMDS in THF (115mL) dropwise. The resulting solution was stirred at -35°C for 30 minutes. Then the mixture was again cooled to -65°C and bromoacetonitrile (22mL) dissolved in dry THF (180mL) was added. The mixture was left stirring for a further 30min at the same temperature. The reaction was quenched with saturated ammonium chloride solution (675mL), THF was evaporated in vacuo and the crude mixture was extracted with EtOAc (2 x 675mL). The organic layer was evaporated and the crude material was purified by chromatography on silica gel using cyclohexanes and ethyl acetate (8:2) affording the title compound (51.3g) as a yellow thick oil.
1 H NMR (400 MHz, CDCl₃-d) δ(ppm): 7.53 (t, 1 H), 7.43 (d, 2H), 7.29-7.37 (m, 1H), 7.17 (t, 1H), 7.07-7.13 (m, 1H), 6.97 (d, 2H), 5.16 (s, 2H), 4.88-4.95 (m, 1 H), 3.90 (s, 3H), 3.52 (d, 1 H), 3.15 (d, 1 H), 2.57-2.70 (m, 1 H), 2.38-2.50 (m, 1H), 2.23-2.34 (m, 1 H), 1.92-2.06 (m, 1 H), 1.44 (s, 9H).

### Intermediate 47

### 1-(1,1-dimethylethyl) 2-methyl (2R,5R)-5-(4-{[(2-fluorophenyl)methyl]oxy}phenyl)-2-[2-(methylamino)ethyll-1,2-pyrrolidinedicarboxylate and 1,1-dimethylethyl (2R,5R)-2-(4-{[(2-fluorophenyl)methy]oxy}phenyl)-7-methyl-6-oxo-1,7-diazaspiro[4.4]nonane-1-carboxylate

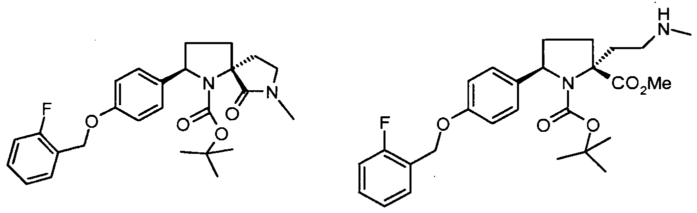

A solution of 1-(1,1-dimethylethyl) 2-methyl (2*R*,5*R*)-5-(4-{[(2-fluorophenyl)methyl]oxy}phenyl)-2-(2-oxoethyl)-1,2-pyrrolidinedicarboxylate (144, 9.5g) in methanol (38ml) was cooled to 5-10°C. Methylamine in methanol (12.7g of a 24.7% solution) was added and the mixture stirred at 5-10°C for 1 hour. Acetic acid (7.6ml) was added to give a pH of approximately 7, and then the temperature was raised to 20-25°C. Sodium triacetoxyborohydride (6.4g) was added in portions over 1 hour, then the mixture stirred at 20-25°C for a further 2 hours. Methanol was distilled off under vacuum, then the mixture treated with ethyl acetate (50ml) and water (50ml). The layers were separated and the aqueous layer re-extracted with ethyl acetate (25ml). The combined ethyl acetate solutions were extracted with 28% citric acid solution (4 x 50ml), then the combined aqueous layers were treated with 30% sodium carbonate solution (150ml) and ethyl acetate to pH 9. The aqueous layer was re-extracted with ethyl acetate (25ml), then the combined ethyl acetate solution was dried over sodium sulphate and evaporated to give the title compound as a crude (6.6g) also comprising the compound 1,1-dimethylethyl (2*R*,5*R*)-2-(4-{[(2-fluorophenyl)methyl]oxy}phenyl)-7-methyl-6-oxo-1,7-diazaspiro[4.4]nonane-1-carboxylate.
MS: (ES/+) m/z: 455 [MH⁺] (cyclised product)

### Representative Na Channel blocker Example 7 (ENa7):

### (2R,5R)-2-(4-{[(2-Fluorophenyl)methyl]oxy}phenyl)-7-methyl-1,7-diazaspiro[4.4]nonan-6-one hydrochloride

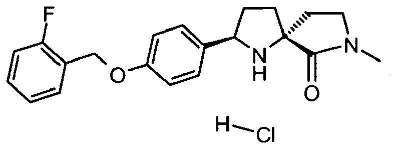

Procedure 1: To a solution of 1,1-dimethylethyl (2*R*,5*R*)-2-(4-{[(2-fluorophenyl)methyl]oxy}phenyl)-7-methyl-6-oxo-1,7-diazaspiro[4.4]-nonane-1-carboxylate (61 mg, 0.134mmol) in a mixture of ethyl acetate (1ml) and methanol (0.1ml) at 0°C was added acetyl chloride (60µl, 0.806mmol). The mixture was stirred for 3 hours at room temperature. The solvent was removed under vacuum and the gummy solid was treated with Et₂O (3 x 2ml) obtaining a white solid (53mg). Rₜ (HPLC): 3.72min; MS: (ES/+) m/z: 355 [MH⁺]; C21H23FN2O2 requires 354; ¹H NMR (500 MHz, DMSO-d₆) δ (ppm): 10.58 (br. s., 1H), 8.87 (br. s., 1H), 7.59-7.52 (m, 3H), 7.47-7.39 (m, 1H), 7.31-7.20 (m, 2H), 7.11 (d, 2H), 5.17 (s, 2H), 4.80 - 4.66 (m, 1 H), 3.43-3.37 (m, 2H), 2.81 (s, 3H), 2.59-2.23 (m, 5H), 2.13-2.03 (m, 1H).

Procedure 2 : The previous crude of (2*R*,5*R*)-2-(4-{[(2-fluorophenyl)methyl]oxy}phenyl)-7-methyl-1,7-diazaspiro[4.4]nonan-6-one (75.85g) was stripped with Et₂O (2 x 150mL), dissolved in DCM (150mL) and 5-6M HCl in IPA (76mL) was added at room temperature. Solvent and excess of HCl were evaporated and then stripped with Et₂O (2 x 76mL) and DCM (2 x 76mL) to get a foam. The foam was suspended in Et₂O (600mL) and DCM (76mL) and stirred overnight at room temperature. Finally the solid was collected, washed with a mixture of Et₂O/DCM 8/1 (3 x 76mL) and dried under high vacuum at 40°C overnight to afford the title compound (79g).
¹H NMR (500 MHz, DMSO-*d₆*) δ(ppm): 10.58 (bs, 1 H); 8.87 (bs, 1 H); 7.55 (m, 3H); 7.43 (m, 1H); 7.25 (m, 2H); 7.11 (d, 2H); 5.17 (s, 2H); 4.73 (m, 1H); 3.40 (m, 2H); 2.81 (s, 3H); 2.60-2.20 (m, 5H); 2.08 (m, 1H).

### Representative Na Channel blocker Example 8 (ENa8):

### (2R,5R)-2-(4-{[(2-Fluorophenyl)methyl]oxy}phenyl)-7-methyl-1,7-diazaspiro[4.4]nonan-6-one

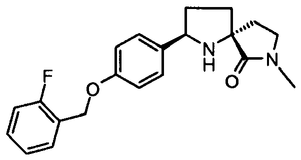

Procedure 1 : The reaction was performed on the two separated batches of 1-(1,1-dimethylethyl) 2-methyl (2*R*,5*R*)-5-(4-{[(2-fluorophenyl)methyl]oxy}phenyl)-2-[2-(methylamino)ethyl]-1,2-pyrrolidinedicarboxylate resulting from procedure decribed for Intermediate 44.
The first batch (80g) was dissolved in MeOH (320mL), 5-6N HCl in IPA (160mL) was added and the mixture stirred overnight at 25°C. 13% aqueous NH₃ (200mL) was added, cooling the reaction to 0°C during addition, until complete conversion. EtOAc (320mL), water (160mL) and NaHCO₃ saturated solution (240mL) were added and the phases separated. Aqueous phase was extracted with EtOAc (6 x 80mL) and the organic phase was concentrated to about 400mL then washed with brine (120mL) which was back extracted with EtOAc (80mL). The combined organic phases were dried over Na₂SO₄ and evaporated to dryness to afford the crude (50g).
The second batch (81g) was dissolved in MeOH (324mL), 5-6N HCl in IPA (162mL) was added and the mixture stirred overnight at 25°C. Further 5-6N HCl in IPA (20mL) was added and stirred for a further 6 hours. NaHCO₃ saturated solution (160mL) and EtOAc (500mL) were added followed by solid NaHCO₃ to pH-8. Phases were separated and the aqueous was back extracted with EtOAc (2 x 200mL). Combined organics were concentrated to about 400mL. DCM (300mL) and brine (200mL) were added, the phases were separated and the organic phase was dried over Na₂SO₄ and evaporated to dryness to afford crude (67g). DCM (135mL) was added and stirred overnight at room temperature then heated to reflux for 3 hours then cooled back to room temperature. 2M NH₃ in methanol (12.5mL) was added due to incomplete cyclisation and then aqueous 13% NH₃ was added and stirred for at least 2 hours to reach complete cyclisation. Brine (100mL) was added, phases were separated and organic was dried over Na₂SO₄ and evaporated to dryness to afford the crude (58g).
The two crudes were combined and purified via chromatographic column (DCM/MeOH 25/1) to afford the title compound (85g).
¹H NMR (600 MHz, DMSO-*d₆*) δ(ppm): 7.56 (dt, 1H); 7.41 (m, 3H); 7.25 (t, 1H); 7.23 (t, 1H); 6.97 (d, 2H); 5.12 (s, 2H); 4.18 (m, 1H); 3.28 (m, 1H); 3.21 (m, 1H); 2.77 (s, 3H); 2.50 (bs, 1H); 2.12 (m, 1H); 2.01 (m, 1H); 1.92 (m, 1H); 1.72 (m, 2H).

Procedure 2: A mixture of 1-(1,1-dimethylethyl) 2-methyl (2*R*,5*R*)-5-(4-{[(2-fluorophenyl)methyl]oxy}phenyl)-2-[2-(methylamino)ethyl]-1,2-pyrrolidinedicarboxylate and 1,1-dimethylethyl (2*R*,5*R*)-2-(4-{[(2-fluorophenyl)methyl]oxy}phenyl)-7-methyl-6-oxo-1,7-diazaspiro[4.4]nonane-1-carboxylate (5.8g) was dissolved in MeOH (23.2mL), HCl in IPA (14.3% solution, 17.7g) was added and the mixture stirred at 25°C for 24 hours. 13% aqueous NH₃ (14.5 ml) and the mixture stirred for 4 hours. EtOAc (60mL) and water (40mL) were added and the phases separated. The aqueous phase was extracted with EtOAc (25ml) then the combined organic phases were dried over Na₂SO₄ and evaporated to dryness to afford the crude (5.52g).The crude was purified via column chromatography (EtOAc to 9:1 EtOAc/MeOH) to afford the title compound (2.1g).

### Representative Na Channel blocker Example 9 (ENa9):

### (2R,5R)-2-(4-{[(2-fluorophenyl)methyl]oxy}phenyl)-7-methyl-1,7-diazaspiro[4.4]nonan-6-one 4-methylbenzenesulfonate

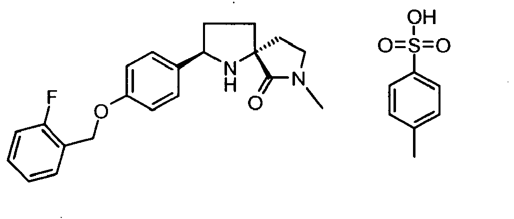

The compound (2*R*,5*R*)-2-(4-{[(2-fluorophenyl)methyl]oxy}phenyl)-7-methyl-1,7-diazaspiro[4.4]nonan-6-one hydrochloride_(57.2g) was suspended in EtOAc (286mL) and NaHCO₃ saturated solution (229mL) was added. The layers were separated and the organic layer was dried over Na₂SO₄.
The salt was filtered off and washed with EtOAc (3 x 57mL).
The solvent was evaporated under reduced pressure and the crude stripped with acetone (2 x 171mL), giving 52.7 g of (2*R*,5*R*)-2-(4-{[(2-fluorophenyl)methyl]oxy}phenyl)-7-methyl-1,7-diazaspiro[4.4]nonan-6-one.
This compound was dissolved in acetone (520mL) and the solution was heated to 40°C. A solution of *p*-toluenesulfonic acid monohydrate (27.84g) in acetone (260mL) was added in 30 min.
A solid precipitated after the addition of approx 50 mL of this solution.
The mixture was stirred at 40°C for 4.5h and then cooled to room temperature.
The solid was collected, washed with acetone (3 x 230 mL) and dried under high vacuum for 16 hours obtaining the Form 1 crystals of the title compound (70.1g). 1 H NMR (400 MHz, DMSO-d6) δ(ppm): 9.90 (bs, 1 H); 8.91 (bs, 1 H); 7.55 (m, 3H); 7.48 (m, 2H); 7.43 (m, 1 H); 7.25 (m, 2H); 7.11 (m, 4H); 5.18 (s, 2H); 4.73 (dd, 1 H); 3.41 (m, 2H); 2.82 (s, 3H); 2.36 (m, 5H); 2.29 (s, 3H); 2.11 (m, 1H).

Unique and discriminating peaks of Form 1 of Example 9 have been identified and are illustrated in the table below:

| Position [°2Th.] | d-spacing [Å] |
|---|---|
| 6.8 | 13.0 |
| 8.7 | 10.2 |
| 10.8 | 8.2 |
| 12.7 | 7.0 |
| 13.6 | 6.5 |
| 14.6 | 6.1 |
| 17.3 | 5.1 |
| 17.8 | 5.0 |
| 18.3 | 4.8 |
| 20.4 | 4.4 |
| 21.0 | 4.2 |
| 22.0 | 4.0 |
| 22.6 | 3.9 |
| 23.1 | 3.8 |
| 24.1 | 3.7 |
| 24.6 | 3.6 |
| 25.0 | 3.6 |
| 27.2 | 3.3 |
| 27.8 | 3.2 |
| 28.1 | 3.2 |
| 28.7 | 3.1 |
| 29.3 | 3.0 |
| 29.6 | 3.0 |
| 30.2 | 3.0 |
| 34.6 | 2.6 |
| 35.4 | 2.5 |
| 36.1 | 2.5 |
| 44.5 | 2.0 |

Melting point: 233 °C.

### Biological Assays

The ability of a compound to act as an NK1 receptor antagonist may be determined using the gerbill foot tapping model as described by Rupniak & Williams, Eur. Jour. of Pharmacol., 1994.
The compound is orally administered and four hours later an NK1 agonist (e.g. delta-Aminovaleryl⁶[Pro⁹,Me-Leu¹⁰]-substance P (7-11)) (3pmol in 5µL icv) is infused directly in the cerebral ventricules of the animals. The duration of hind foot tapping induced by the NK1 agonist (e.g. delta-Aminovaleryl⁶[Pro⁹,Me-Leu¹⁰]-substance P (7-11)) is recorded continuously for 3 min using a stopclock. The dose of the test compound required to inhibit by 50% the tapping induced by the NK1 agonist (e.g. delta-Aminovaleryl⁶[Pro⁹,Me-Leu¹⁰]-substance P (7-11)) expressed as mg/kg is referred to as the ED50 value. Alternatively the compounds may be administered subcutaneously or intraperitoneally.

The ability of the compounds of the invention to modulate the voltage-gated sodium channel subtype NaV 1.3 may be determined by the following assay.

### Cell biology

Stable cell lines expressing hNaV1.3 channels were created by transfecting CHO cells with the pCINS-hNav1.3 vector using the lipofectamine (Invitrogen) transfection method. pCIN5 is a bicistronic vector for the creation of mammalian cell lines that predisposes all neomycin resistant cells to express recombinant protein (see Rees S., Coote J., Stable J., Goodson S., Harris S. & Lee M.G. (1996) Biotechniques, **20**, 102-112) by virtue of the recombinant cDNA being linked to the neomycin-selectable marker cDNA downstream of the CMV promoter (for full details see Chen YH, Dale TJ, Romanos MA, Whitaker WR, Xie XM, Clare JJ. Cloning, distribution and functional analysis of the type III sodium channel from human brain Eur J Neurosci, 2000 Dec;**12**, 4281-9). Cells were cultured in Iscove's modified Dulbecco's medium (Invitrogen) comprising, 10% fetal bovine serum, 1% L-glutamine, 1% Penicillin-Streptomycin (Invitrogen), 1% non-essential amino acids, 2% H-T supplement and 1% G418 (Invitrogen) and maintained at 37°C in a humidified environment comprising 5% CO2 in air. Cells were liberated from the T175 culture flask for passage and harvesting using Versene (Invitrogen).

### Cell preparation

Cells were grown to 60-95% confluence in a T75 flask. Cells were lifted by removing the growth media and incubating with 1.5 ml of warmed (37°C) Versene (Invitrogen, 15040-066) for 6 min. Lifted cells were suspended in 10 ml of PBS (Invitrogen, 14040-133). Cell suspension was then placed into a 10-ml centrifuge tube and centrifuged for 2 min at 700 rpm. After centrifugation, the supernatant was removed and the cell pellet was resuspended in 3 ml of PBS.

### Electrophysiology

Currents were recorded at room temperature (21-23°C) using the IonWorksHT planar array electrophysiology technology (Molecular Devices Corp.). Stimulation protocols and data acquisition were carried out using a microcomputer (Dell Pentium 4). In order to determine planar electrode hole resistances (Rp), a 10 mV, 160 ms potential difference was applied across each hole. These measurements were performed before cell addition. After cell addition a seal test was performed prior to antibiotic (amphotericin) circulation to achieve intracellular access. Leak subtraction was conducted in all experiments by applying a 160 ms hyperpolarizing (10 mV) prepulse 200 ms before the test pulses to measure leak conductance. Test pulses stepping from the holding potential of -90 mV to 0 mV were applied for 20 ms and repeated 10 times at a frequency of 10 Hz. In all experiments, the test pulse protocol was performed in the absence (pre-read) and presence (post-read) of a compound. Pre- and post-reads were separated by a compound addition followed by a 3-3.5 min incubation.

### Solutions and drugs

The intracellular solution contained the following (in m*M*): K-gluconate 100, KCI 40mM, MgCl2 3.2, EGTA 3, HEPES 5, adjusted to pH 7.25. Amphotericin was prepared as 30 mg/ml stock solution and diluted to a final working concentration of 0.1 mg/ml in internal buffer solution. The external solution was Dulbecco's PBS (Invitrogen) and contained the following (in mM): CaCl2 0.90, KCI 2.67, K3PO4 1.47, MgCl2 0.50, NaCl 138, Na3PO4 8.10, with a pH of 7.4. Compounds were prepared in DMSO as 10mM stock solutions and subsequent 1:3 serial dilutions performed. Finally the compounds were diluted 1:100 in external solution resulting in a final DMSO concentration of 1%.

### Data analysis

The recordings were analysed and filtered using both seal resistance (>40 MΩ) and peak current amplitude (>200pA) in the absence of compound to eliminate unsuitable cells from further analysis. Paired comparisons between pre-drug and post-drug additions were used to determine the inhibitory effect of each compound. The concentrations of compounds required to inhibit current elicited by the 1^{st} depolarising pulse by 50% (tonic pIC50) were determined by fitting of the Hill equation to the concentration response data. In addition the use-dependent inhibitory properties of the compounds were determined by assessing the effect of compounds on the 10^{th} versus 1^{st} depolarising pulse. The ratio of the 10^{th} over 1^{st} pulse was calculated in the absence and presence of drug and the % use-dependent inhibition calculated. The data was fitted using the same equation as for the tonic pIC₅₀ and the concentration producing 15% inhibition (use-dependent pUD₁₅) calculated.

### PreClinical Experiments

### Experimental Preparation

This work was conducted in compliance with the Home Office Guidance on the operation of the Animals (Scientific Procedures) Act 1986 and GlaxoSmithKline ethical standards. Male CD rats (100-145g) were supplied by Charles River, UK. Animals were group housed (6 animal per cage) with free access to food (Standard rodent chow; Harlan, UK) and water under a 12 h light/dark cycle (lights on at 0700h). A period of at least one week between arrival at GSK and the study was allowed in all cases.

### Experimental Protocol

Animals were administered a test compound at the appropriate dose, route and pretreatment time and returned to their home cage. Testing occured in a separate room from that used for housing. Testing involved determining the threshold for tonic hindlimb extensor seizures using a Hugo Sachs Electronik stimulator which delivers a constant current of 0.3 second duration, 50Hz, sinewave form, fully adjustable between 1 and 300 mA. Stimuli were delivered via corneal electrodes (Stean TO, Atkins AR, Heidbreder CA, Quinn LP, Trail BK, Upton N. (2005) Br J Pharmacol.144(5):628-35). Seizure threshold was determined using the 'up and down' method of Kimball et al. (1957)( Kimball AW, Burnett WT Jr, Doherty DG. (1957) Radiat Res. 7(1):1-12). The first animal tested in each group was stimulated with a current that might be expected to be close to the threshold for induction of a seizure. If a tonic seizure was not induced, then then next animal in the group received a stimulus 5 mA higher. If a tonic seizure was induced, then the next animal received a stimulus 5 mA lower. This is repeated for all animals within the control (vehicle) group. In the case of groups treated with a sodium channel blocker steps of 10 to 30 may were used.
Satellite animals (n=3/group) received the same drugs or combinations of drugs as the test groups, and were culled at the appropriate time after dosing. Blood and brain samples were taken for analysis of the drug concentrations in these compartments.

### Drugs and Materials

All doses were calculated as base. **ENa13** was dosed via the subcutaneous (s.c.) route at 1 ml/kg in a 2% v/v DMSO/saline vehicle 30 minutes prior to test. Compound **ENK6** was dosed with via the intraperitoneal (i.p.) route 60 minutes before the test. In both cases **ENK6** was dissolved in saline.

### Data Analysis

Induction of seizure is measured as an all-or-nothing effect scored as either present (+) or absent (0) for each animal. The data for each treatment group were recorded as the number of +'s and 0's at each current level employed and this information was then used to calculate the CC50 value (current required for 50% of animals to show seizure behaviour) + standard error of the mean according to the method of Kimball et al. (1957). Drug effects were calculated as the % change in CC50. Significant differences between drug-treated animals and appropriate vehicle treated groups were assessed according to the methods of Litchfield and Wilcoxon (1949). The statistical significance of differences between (2*R*,5*R*)-2-(4-{[(2-fluorophenyl)methyl]oxy)phenyl)-7-methyl-1,7-diazaspiro[4.4]nonan-6-one alone_or in combination with **ENK6** was assessed using a Student's t test.

### ENa7/ ENK6 combination

A dose of 3mg/kg s.c. of **ENa7** was found to give a robust and significant increase in seizure threshold, whereas a lower dose of 1mg/kg s.c. was inactive. **ENK6** (30mg/kg i.p.) had no effect on seizure threshold when administered alone. In the presence of **ENK6, ENa7** produced a significant increase in seizure threshold at 1mg/kg (118% increase in CC50(mA)) and also a significant increase in seizure threshold at 3mg/kg (129% increase in CC50(mA)) (Figure 1).

### Figure 1

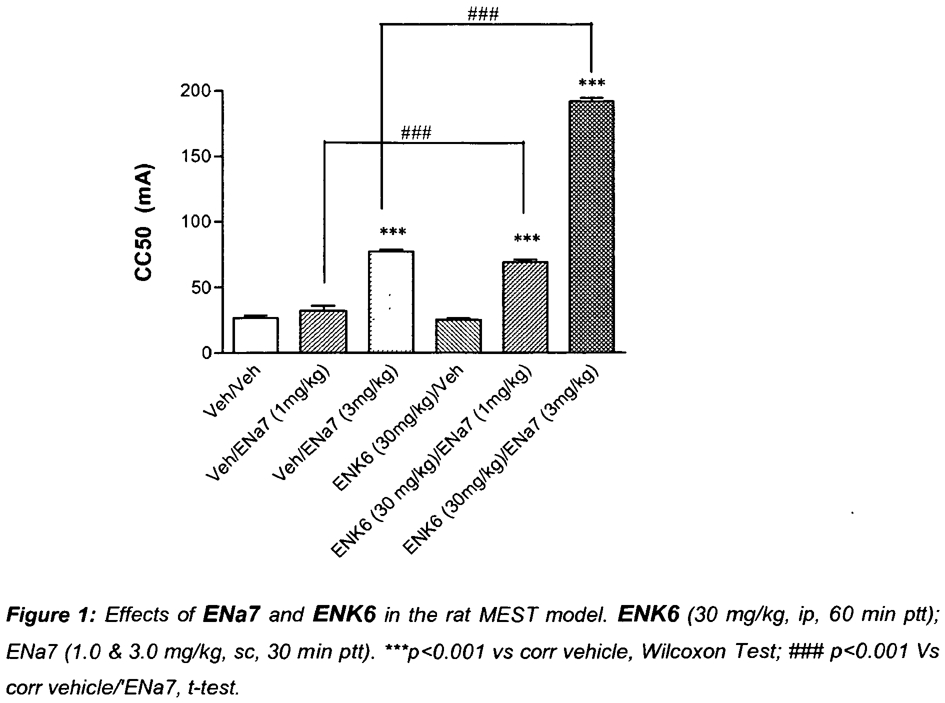

No increase in the concentration of **ENa7** was observed in the presence of the NK1 antagonist (Table 1); suggesting that a PK interaction does not explain the increased PD effect.

**Table 1**

| | | **Blood (ng/mL)** | | **Brain (ng/g)** | |
|---|---|---|---|---|---|
| **ENK6 (mg/kg)** | **ENa7 (mg/kg)** | **ENK6** | **ENa7** | **ENK6** | **ENa7** |
| veh | 1 | | 499 | | 478 |
| veh | 3 | | 1824 | | 1825 |
| 30 | veh | 558* | | 1263* | |
| 30 | 1 | 456 | 416 | 1035 | 508 |
| 30 | 3 | 1209 | 1160 | 4802 | 1235 |

***Table 1:** Blood and brain concentrations of **ENa7** and **ENK6** in satellite animals (n=2 per group, except *n=1). Differences in concentrations of the sodium channel blocker are within inter-animal variability.*

## Claims

1. A pharmaceutical composition comprising an NK1 receptor antagonist and a Sodium Channel blocker compound of formula (I) 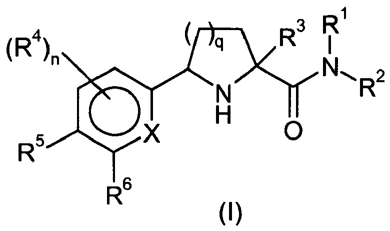 wherein
R¹ and R² are independently hydrogen, C₁₋₆alkyl or C₃₋₆cycloalkylC₁₋₆alkyl; or R¹ and R², together with the nitrogen to which they are attached, may form an unsubstitued 3-, 4-, 5- or 6-membered saturated ring;
R³ is C₁₋₆alkyl, C₁₋₆haloalkyl, C₁₋₃alkoxyC₁₋₃alkyl or C₁₋₃haloalkoxyC₁₋₃alkyl;
or R¹ and R³, together with the interconnecting atoms, form a saturated or unsaturated 4-to 6-membered ring;
X is carbon or nitrogen;
n is 0, 1 or 2, wherein when present each R⁴ is independently selected from the list consisting of C₁₋₃alkyl, halogen, cyano, haloC₁₋₃alkyl, hydroxy, C₁₋₃alkoxy and C₁₋₃haloalkoxy;
q is 1 or 2;
either R⁵ or R⁶ is -O-R⁷ or -OCH₂R⁷, wherein the other R⁵ or R⁶ is hydrogen or R⁴; and wherein R⁷ is either a phenyl ring or a 5- or 6-membered aromatic heterocyclic ring (independently containing one or more nitrogen, sulphur or oxygen atoms) wherein either the phenyl ring or the heterocyclic ring is optionally substituted by one or more groups independently selected from the list consisting of C₁₋₃alkyl, halogen, cyano, haloC₁₋₃alkyl, hydroxy, C₁₋₃alkoxy and C₁₋₃haloalkoxy;
or a pharmaceutically acceptable salt or solvate thereof;
as a combined preparation for simultaneous or sequential administration.

2. A pharmaceutical composition according to claim 1, wherein the compound of formula (I) is (2*R*,5*R*)-2-(4-{[(2-fluorophenyl)methyl]oxy}phenyl)-7-methyl-1,7-diazaspiro[4.4]nonan-6-one, or a pharmaceutically acceptable salt or solvate thereof, as a combined preparation for simultaneous or sequential administration.

3. A pharmaceutical composition according to claim 1 or 2, wherein at least one of the NK1 receptor antagonist or the Sodium channel blocker of formula (I) is at subtherapeutic dose.

4. A pharmaceutical composition according to anyone of claims 1 to 3, for use in therapy.

5. A pharmaceutical composition according to any one of claims 1 to 3, for use in the treatment of epilepsy or mood disorders.

6. Use of a pharmaceutical composition according to any one of claims 1 to 3 in the manufacture of a medicament for the treatment of epilepsy or mood disorders.

## Patentansprüche

1. Ein Arzneimittel, umfassend einen NK1-Rezeptorantagonisten und eine Natriumkanalblockerverbindung der Formel (I) 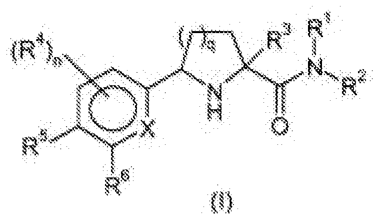 wobei
R¹ und R² unabhängig Wasserstoff, C₁₋₆-Alkyl oder C₃₋₆-Cycloalkyl-C₁₋₆-alkyl sind; oder R¹ und R², zusammen mit dem Stickstoff, an den sie gebunden sind, einen unsubstituierten 3-, 4-, 5- oder 6-gliedrigen gesättigten Ring bilden können;
R³ C₁₋₆-Alkyl, C₁₋₆-Halogenalkyl, C₁₋₃-Alkoxy-C₁-₃-alkyl oder C₁-₃-Halogenalkoxy-C₁-₃-alkyl ist;
oder R¹ und R³, zusammen mit den sie verbindenden Atomen, einen gesättigten oder ungesättigten 4- bis 6-gliedrigen Ring bilden;
X Kohlenstoff oder Stickstoff ist;
n 0, 1 oder 2 ist, wobei jedes R⁴, falls vorhanden, unabhängig aus der Liste bestehend aus C₁₋₃-Alkyl, Halogen, Cyano, Halogen-C₁₋₃-alkyl, Hydroxy, C₁₋₃-Alkoxy und C₁₋₃-Halogenalkoxy ausgewählt ist;
q 1 oder 2 ist;
entweder R⁵ oder R⁶ -O-R⁷ oder -OCH₂R⁷ ist, wobei das andere R⁵ oder R⁶ Wasserstoff oder R⁴ ist; und wobei R⁷ entweder ein Phenylring oder ein 5- oder 6-gliedriger aromatischer heterocyclischer Ring ist (der unabhängig ein oder mehrere Stickstoff-, Schwefel- oder Sauerstoffatome enthält), wobei entweder der Phenylring oder der heterocyclische Ring gegebenenfalls mit einem oder mehreren Resten substituiert ist, die unabhängig aus der Liste bestehend aus C₁₋₃-Alkyl, Halogen, Cyano, Halogen-C₁₋₃-alkyl, Hydroxy, C₁₋₃-Alkoxy und C₁₋₃-Halogenalkoxy ausgewählt sind;
oder ein pharmazeutisch verträgliches Salz oder Solvat davon;
als ein Kombinationspräparat zur gleichzeitigen oder aufeinanderfolgenden Verabreichung.

2. Ein Arzneimittel nach Anspruch 1, wobei die Verbindung der Formel (I) (2R,5R)-2-(4-{[(2-Fluorphenyl)methyl]oxy}phenyl)-7-methyl-1,7-diazaspiro[4.4]nonan-6-on ist, oder ein pharmazeutisch verträgliches Salz oder Solvat davon, als ein Kombinationspräparat zur gleichzeitigen oder aufeinanderfolgenden Verabreichung.

3. Ein Arzneimittel nach Anspruch 1 oder 2, wobei mindestens einer aus dem NK1-Rezeptorantagonisten oder dem Natriumkanalblocker der Formel (I) in subtherapeutischer Dosis vorliegt.

4. Ein Arzneimittel nach einem der Ansprüche 1 bis 3 zur Verwendung bei der Therapie.

5. Ein Arzneimittel nach einem der Ansprüche 1 bis 3 zur Verwendung bei der Behandlung von Epilepsie oder Gemütszustandsstörungen.

6. Verwendung eines Arzneimittels nach einem der Ansprüche 1 bis 3 bei der Herstellung eines Medikaments zur Behandlung von Epilepsie oder Gemütszustandsstörungen.

## Revendications

1. Composition pharmaceutique comprenant un antagoniste du récepteur NK1 et un composé bloquant le canal sodium de formule (I) 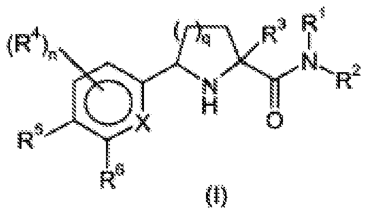 dans laquelle
R¹ et R² représentent indépendamment un atome d'hydrogène, un groupe alkyle en C₁ à C₆ ou (cycloalkyle en C₃ à C₆) - (alkyle en C₁ à C₆) ; ou bien R¹ et R², conjointement avec l'atome d'azote auquel ils sont fixés, peuvent former un noyau saturé tri-, tétra-, penta- ou hexagonal non substitué ;
R³ représente un groupe alkyle en C₁ à C₆, halogénalkyle en C₁ à C₆, (alkoxy en C₁ à C₃) (alkyle en C₁ à C₃) ou halogéno (alkoxy en C₁ à C₃) (alkyle en C₁ à C₃) ;
ou bien R¹ et R³, conjointement avec les atomes d'interconnexion, forment un noyau tétra- à hexagonal saturé ou insaturé ;
X représente un atome de carbone ou d'azote ;
n est égal à 0, 1 ou 2 ; où, lorsqu'il est présent, chaque groupe R⁴ est choisi indépendamment dans la liste consistant en des groupes alkyle en C₁ à C₃, halogéno, cyano, halogénalkyle en C₁ à C₃, hydroxy, alkoxy en C₁ à C₃ et halogénalkoxy en C₁ à C₃ ;
q est égal à 1 ou 2 ;
R⁵ ou bien R⁶ représente un groupe -O-R⁷ ou -OCH₂R⁷, où l'autre groupe R⁵ ou R⁶ représente un atome d'hydrogène ou un groupe R⁴ ; et où R⁷ représente un noyau phényle ou bien un noyau hétérocyclique aromatique penta- ou hexagonal (contenant indépendamment un ou plusieurs atomes d'azote, de soufre ou d'oxygène), le noyau phényle ou le noyau hétérocyclique étant facultativement substitué avec un ou plusieurs groupes choisis indépendamment dans la liste consistant en des groupes alkyle en C₁ à C₃, halogéno, cyano, halogénalkyle en C₁ à C₃, hydroxy, alkoxy en C₁ à C₃ et halogénalkoxy en C₁ à C₃ ;
ou un de ses sels ou produits de solvatation pharmaceutiquement acceptables ;
sous forme d'une préparation combinée pour une administration simultanée ou séquentielle.

2. Composition pharmaceutique suivant la revendication 1, dans laquelle le composé de formule (I) est la (2*R*, 5*R*)-2-(4-{[(2-fluorophényl)méthyl]oxy}phényl-7-méthyl-1,7-diaza-spiro[4.4]nonane-6-one, ou un de ses sels ou produits de solvatation pharmaceutiquement acceptables, sous forme d'une préparation combinée pour une administration simultanée ou séquentielle.

3. Composition pharmaceutique suivant la revendication 1 ou 2, dans laquelle au moins un de l'antagoniste du récepteur NK1 et de l'agent de blocage du canal sodium de formule (I) est présent à une dose sous-thérapeutique.

4. Composition pharmaceutique suivant l'une quelconque des revendications 1 à 3, pour son utilisation en thérapie.

5. Composition pharmaceutique suivant l'une quelconque des revendications 1 à 3, pour son utilisation dans le traitement de l'épilepsie ou de troubles de l'humeur.

6. Utilisation d'une composition pharmaceutique suivant l'une quelconque des revendications 1 à 3, dans la production d'un médicament pour le traitement de l'épilepsie ou de troubles de l'humeur.
